# EUROPEAN PATENT SPECIFICATION

(11) **EP 1 525 328 B1**
(45) Date of publication and mention of the grant of the patent: **20.01.2010**
(21) Application number: 03784164.0
(22) Date of filing: 01.08.2003
(51) Int. Cl.: C12Q 1/68, G06F 19/00

(54) **METHOD FOR AMPLIFICATION OF NUCLEIC ACIDS OF LOW COMPLEXITY**
VERFAHREN ZUR UNSPEZIFISCHEN AMPLIFIZIERUNG NUKLEINSÄUREN MIT NIEDRIGER KOMPLEXITÄT
PROCEDE D'AMPLIFICATION D'ACIDES NUCLEIQUES DE FAIBLE COMPLEXITE

(30) Priority: 02.08.2002 DE 10236406
(43) Date of publication of application: 27.04.2005
(73) Proprietor: Epigenomics AG, 10178 Berlin (DE)
(72) Inventor: RUJAN, Tamas, 10437 Berlin (DE); SCHMITT, Armin, 12203 Berlin (DE); ADORJAN, Peter, 10437 Berlin (DE); PIEPENBROCK, Christian, 10115 Berlin (DE)
(74) Representative: Schubert, Klemens
(86) International application number: PCT/EP2003/008602
(87) International publication number: WO 2004/015139

(56) References cited:
- WO-A-00/49177
- WO-A-00/70090
- WO-A-01/44504
- WO-A-01/62960
- WO-A-98/56952
- WO-A2-03/054224
- WO-A2-03/057909
- US-A- 5 786 146
- US-A- 6 007 231
- US-A1- 2003 068 625
- HERMAN J G ET AL: "METHYLATION-SPECIFIC PCR: A NOVEL PCR ASSAY FOR METHYLATION STATUS OF CPG ISLANDS" PROCEEDINGS OF THE NATIONAL ACADEMY OF SCIENCES OF USA, NATIONAL ACADEMY OF SCIENCE. WASHINGTON, US, vol. 93, 1 September 1996 (1996-09-01), pages 9821-9826, XP002910406 ISSN: 0027-8424 cited in the application
- REIN ET AL: "Identifying 5-methylcytosine and related modifications in DNA genomes" NUCLEIC ACIDS RESEARCH, OXFORD UNIVERSITY PRESS, SURREY, GB, vol. 26, no. 10, 1998, pages 2255-2264, XP002143106 ISSN: 0305-1048 cited in the application
- ZESCHNIGK M ET AL: "A SINGLE-TUBE PCR TEST FOR THE DIAGNOSIS OF ANGELMAN AND PRADER-WILLI SYNDROME BASED ON ALLELIC METHYLATION DIFFERENCES AT THE SNRPN LOCUS" EUROPEAN JOURNAL OF HUMAN GENETICS, KARGER, BASEL, CH, vol. 5, no. 2, 1997, pages 94-98, XP009011533 ISSN: 1018-4813 cited in the application
- GRIFFIN HG AND GRIFFIN AM (EDS.): "PCR TECHNOLOGY: Current Innovations" 1994 , CRC PRESS , BOCA RATON XP008025537 page 5 -page 11
- SEIJEN A M ET AL: "Systematic design of mouse Vh gene family-specific oligonucleotides" JOURNAL OF IMMUNOLOGICAL METHODS, ELSEVIER SCIENCE PUBLISHERS B.V.,AMSTERDAM, NL, vol. 254, no. 1-2, 1 August 2001 (2001-08-01), pages 161-168, XP004245450 ISSN: 0022-1759
- SCHULER G D: "SEQUENCE MAPPING BY ELECTRONIC PCR" GENOME RESEARCH, COLD SPRING HARBOR LABORATORY PRESS, US, vol. 7, 1997, pages 541-550, XP000872176 ISSN: 1088-9051 cited in the application
- ENGELS W R: "Contributing software to the internet: the Amplify program" TRENDS BIOCHEM. SCI., vol. 18, 1993, pages 448-450, XP001161002

## Description

The present invention relates to a method for the amplification of nucleic acids.

### Description

This invention relates to the fields of genetic engineering, molecular biology and computer science, and more specifically to the field of nucleic acid analysis based on specific nucleic acid amplification.

The matter of the present invention is a method for amplifying nucleic acids, such as DNA by means of an enzymatic amplification step, such as a polymerase chain reaction, specified for template nucleic acids of low complexity, e.g. pre-treated DNA, like but not limited to DNA pre-treated with bisulfite. The invention is based on the use of specific oligo-nucleotide primer molecules to solely amplify specific pieces of DNA. It is disclosed how to optimize the primer design for a PCR if the template DNA is of unusually low complexity. Also, for the optimal primer design it was considered that the treated template DNA is single stranded.

The amplification of nucleic acids relies mainly on a method called polymerase chain reaction (PCR). The PCR is based on the activity of the enzyme DNA polymerase, which is elongating primer molecules, which bind to the template DNA by adding dNTPs and hereby copying the template sequence (Saiki RK, Gelfand DH, Stoeffel S, Scharf SJ, Higuchi R, Horn T, Mullis KB and Erlich HA (1988). Primer-directed enzymatic amplification of DNA with a thermostable DNA polymerase. Science 239 : 487-491). The primer molecules are designed to specifically hybridize to those regions of the template DNA that define both ends of the amplificate. The forward primer binds to the 5' end of the sense strand of the amplificate, whereas the reverse primer binds to the 5' end of the reverse strand, hereby defining the starting points of the polymerase reaction and eventually determining the length of the amplificate.

Before the polymerase starts the template DNA gets denatured, this is usually done by a short cycle of heating the reaction mixture up to about 95°C, then cooling it down to the annealing temperature determined by the melting temperature of the primer molecules used and finally allowing the polymerase to elongate the annealed primers at its ideal working temperature for some minutes. This cycle is repeated several times each starting with the denaturation step. The primer molecules hybridize to the single stranded DNA. The forward primer is the starting molecule for a copy of the sense strand and the reverse primer is the starting molecule for a copy of the anti-sense strand.

These first copies will be of unspecific length, limited only by the polymerase's activity. However in the following cycle, the forward primer will also bind to the first copy of the anti-sense strand, the polymerase will take that copy as a template and will elongate the primer only as far as there is template DNA. Hereby the length of the second copy gets limited to the length defined by the first nucleotide of the second primer. In the following cycles more and more pieces of template DNA compete for the primer molecules and eventually the DNA amplificate of defined length will be the main product.

However, in the case of a bisulfite treated DNA the template DNA is single stranded. The bisulfite or similar treatment alters the original sequences on both strands such that these are not complementary to each other after the treatment. As a result no complementary strand to the target sequence exists. A first primer molecule binds to the one end of the single stranded target sequence. The polymerase elongates said primer and copies said target sequence. The second primer molecule cannot bind to the complementary, so called anti-sense strand, as it would in a standard PCR. Therefore the second primer molecule is designed to bind to the first copied sequence instead. More specifically it will bind to that part of the copied nucleic acid which is the complement to the other end of said target sequence.

The results of a PCR are highly depending on the choice of the ideal primer. The choice of a primer molecule must respect constraints permitting a correct amplification by PCR, fulfilling hybridization temperature conditions and auto- or hetero-hybridization prevention.

In other words, as any PCR requires two primer molecules to amplify a specific piece of DNA in one reaction the melting temperatures of both primers need to be very similar in order to allow proper binding of both at the same hybridization temperature. That is why most primer design programs require the user to define a preferred melting temperature or a permitted range of melting temperatures. This requirement becomes the limiting factor when designing primers for a so called multiplex PCR, as all primer pairs in use need to have the same or at least very similar melting temperatures. Additionally primers have to be very specific, in order to only amplify those pieces of DNA that are the target.

By providing the means for designing extremely accurate primer pairs for DNA hybridization procedures this invention relates to the so called PCR primer design. More specifically the body of this invention relates to the specific requirements of primers and therefore of primer design when using template DNA that consists of essentially only three different nucleotides and is single stranded. This is the case when using bisulfite treated DNA as a template, as it contains no cytosine other than the methylated cytosines in a CG dinucleotide and a rest of insufficiently treated and therefore untransformed non-methylated cytosines. The invention relates specifically to the primer design when using bisulfite treated DNA as template.

It would be obvious to an individual skilled in the art that the use of the primers as specified in this invention are not limited to nucleic acid amplification. Said primers can be used for several purposes, such as amplification, but also for nucleic acid sequencing or as blocking oligonucleotides during analysis of bisulfite treated DNA. Therefore the use of said primers is not limited to nucleic acid amplification but extends to all standard molecular biological methods.

Pairs of these primers are used to specifically amplify DNA from a small amount of sample DNA that consists of bisulfite treated DNA originating from a limited source of DNA like a bodily fluid or tissue sample.

DNA can occur methylated or non-methylated at certain positions and this information is relevant for the status of a genes transcription. The methyl group is attached to the cytosine bases in CpG positions. The identification of 5-methylcytosine in a DNA sequence as opposed to un-methylated cytosine is of greatest importance for example when studying the role of DNA methylation in tumorigene-sis. But, because the 5-Methylcytosine behaves just as a cytosine for what concerns its hybridization preference (a property relied upon for sequence analysis) its positions can not be identified by a normal sequencing reaction. Furthermore in a PCR amplification this relevant epigenetic information, methylated cytosine or unmethylated cytosine, will be lost completely.

This problem is usually solved by treating the genomic DNA with a chemical leading to a conversion of the cytosine bases, which consequently allows to differentiate the bases afterwards.

A tool most useful for analyzing DNA methylation is the bisulfite conversion of DNA that converts cytosine bases into bases showing a hybridization behavior as thymin bases. Hereby the DNAs complexity is reduced by a fourth.

Bisulfite conversion is the most frequently used method for analyzing DNA for 5-methylcytosine. It is based upon the specific reaction of bisulfite with cytosine which, upon subsequent alkaline hydrolysis, is converted to uracil, whereas 5-methylcytosine remains unmodified under these conditions (Shapiro et al. (1970) Nature 227: 1047). However, in its base pairing behavior, uracil corresponds to thymine, that is, it hybridizes to adenine; whereas 5-methylcytosine doesn't change its chemical properties under this treatment and therefore still has the base pairing behavior of a cytosine, that is hybridizing with guanine. Consequently, the original DNA is converted in such a manner that methyl-cytosine, which originally could not be distinguished from cytosine by its hybridization behavior, can now be detected as the only remaining cytosine using "normal" molecular biological techniques, for example, by amplification and hybridization or sequencing. All of these techniques are based on base pairing which can now be fully exploited. Comparing the sequences of the DNA prior to and after bisulfite treatment allows an easy identification of those bases that have been methylated.

In the scope of this invention when it says "a nucleotide (...) was converted by the treatment..." this conversion is meant to be able to differentiate between methylated and un-methylated cytosine bases within said sample, as for example the conversion of un-methylated cytosine bases to bases which hybridize to adenine by the treatment with bisulfite.

An alternative method is to use restriction enzymes that are capable of differentiating between methylated and un-methylated DNA, but this is restricted in its uses due to the selectivity of the restriction enzyme towards a specific sequence.

An overview of the further known methods of detecting 5-methylcytosine may be gathered from the following review article: Rein T, DePamphilis ML, Zorbas H, Nucleic Acids Res. 1998, 26, 2255.

In terms of sensitivity, the prior art is defined by a method, which encloses the DNA to be analyzed in an agarose matrix, thus preventing the diffusion and renatura-tion of the DNA (bisulfite reacts with single-stranded DNA only), and which replaces all precipitation and purification steps with fast dialysis (Olek A, Oswald J, Walter J (1996) A modified and improved method for bisulfite based cytosine methylation analysis. Nucleic Acids Res. 24: 5064-6). Using this method, it is possible to analyze individual cells, which illustrates the potential of the method.

To date, barring few exceptions (e.g., Zeschnigk M, Lich C, Buiting K, Doerfler W, Horsthemke B (1997) A single-tube PCR test for the diagnosis of Angelman and Prader-Willi syndrome based on allelic methylation differences at the SNRPN locus. Eur J Hum Genet. 5: 94-8) the bisulfite technique is only used in research. Always, however, short, specific fragments of a known gene are amplified subsequent to a bisulfite treatment and either completely sequenced (Olek A, Walter J (1997) The pre-implantation ontogeny of the H19 methylation imprint. Nat Genet. 3: 275-6) or individual cytosine positions are detected by a primer extension reaction (Gonzalgo ML and Jones PA (1997) Rapid quantitation of methylation differences at specific sites using methylation-sensitive single nucleotide primer extension (Ms-SNuPE). Nucleic Acids Res. 25 :2529-31; WO 95/00669) or by enzymatic digestion (Xiong Z, Laird PW (1997) COBRA: a sensitive and quantitative DNA methylation assay. Nucleic Acids Res. 25: 2532-4).

Another technique to detect hypermethylation is the so called methylation specific PCR (MSP) (Herman JG, Graff JR, Myohanen S, Nelkin BD and Baylin SB (1996), Methylation-specific PCR: a novel PCR assay for methylation status of CpG islands. Proc Natl Acad Sci USA. 93: 9821-6). The technique is based on the use of primers that differentiate between a methylated and a non-methylated sequence if applied after bisulfite treatment of said DNA sequence. The primer either contains a guanine at the position corresponding to the cytosine in which case it will after bisulfite treatment only bind if the position was methylated. Or the primer contains an adenine at the corresponding cytosine position and therefore only binds to said DNA sequence after bisulfite treatment if the cytosine was unmethylated and has hence been altered by the bisulfite treatment so that it hybridizes to adenine.

With the use of these primers amplicons can be produced specifically depending on the methylation status of a certain cytosine and will as such indicate its methylation state. The present invention, however, does preferably not include CpGs in the primer sequence.

Another new technique is the detection of methylation via Taqman PCR, also known as MethylLight (WO 00/70090). With this technique it became feasible to determine the methylation state of single or of several positions directly during PCR, without having to analyze the PCR products in an additional step.

In addition, detection by hybridization has also been described (WO 99/28498).

Further publications dealing with the use of the bisul-fite technique for methylation detection in individual genes are:
Grigg G, Clark S (1994) Sequencing 5-methylcytosine residues in genomic DNA. Bioassays 16: 431-6; Zeschnigk M, Schmitz B, Dittrich B, Buiting K, Horsthemke B, Doerfler W (1997) Imprinted segments in the human genome: different DNA methylation patterns in the Prader-Willi/Angelman syndrome region as determined by the genomic sequencing method. Hum Mol Genet. 6: 387-95; Feil R, Charlton J, Bird AP, Walter J, Reik W (1994) Methylation analysis on individual chromosomes: improved protocol for bisulphite genomic sequencing. Nucleic Acids Res. 22: 695-6; Martin V, Ribieras S, Song-Wang X, Rio MC, Dante R (1995) Genomic sequencing indicates a correlation between DNA hy-pomethylation in the 5' region of the pS2 gene and its expression in human breast cancer cell lines. Gene 157 : 261-4; WO 97/46705; WO 95/15373; WO 97/45560

The US patent 5,786,146 (Herman et al.) describes a method of detection of methylated nucleic acid using agents which modify unmethylated cytosine and distinguishing modified methylated and non-methylated nucleic acids.

Further a method for detecting cytosine methylation in DNA samples is disclosed in the WO 01/62960.

The WO 01/44504 describes a method for detecting methylated CpG-containing nucleic acid.

The WO 98/56952 (Gonzalgo et al.) discloses a cancer diagnostic method based upon DNA methylation differences at specific CpG sites.

For all those methods mentioned above, which are based on PCR amplification of bisulfite treated DNA, the biggest challenge is to design primers that are specific.

### THE PROBLEM AND ITS SOLUTION

There are a number of programs available on the market that offer to design primer pairs in order to amplify a piece of DNA in a PCR. Usually they require as input the template DNA sequence, the preferred melting temperature TM, the desired length of the amplificate and optionally the preferred length of the primer molecules.

However if a primer is required to bind specifically to bisulfite treated DNA, the design of the primer molecule is especially difficult and those tools known in the art are not competent to design primers that lead to specific products. The following problems occur when dealing with bisulfite treated DNA instead of standard DNA:

First, the sequence complexity of the bisulfite treated genome is reduced dramatically. Complexity in this context is meant to be a measure for the similarity of a given sequence to a random or stochastic sequence; the more complex a sequence is the more it is similar to a random sequence. A reduced complexity of the genome means there are less degrees of variation. Where there are essentially only three different nucleotides rather than four, the probability of a sequence to occur twice in a given length of sequence is much higher. For example, a primer molecule of 20 nucleotides in length is likely to be unique in the human genome, if it is not part of a repeat sequence: The human genome is known to consist of about 3 x 109 bases. There are 420 ≈ 1012 different ways to form sequences of a length of 20 nucleotides, assuming equidistribution of the bases, which makes multiple occurrences of a given 20-mer (oligonucleotide of 20 nucleotides) extremely unlikely. However since there are only 320 ≈ 3 x 109 different 20-mers possible over a 3-letter alphabet, this multiple occurrence cannot be excluded. In addition a bisulfite treated sequence, enriched in thymine in the sense strand and enriched in adenine in the reverse complementary strand, will contain more repeats and regions of general low complexity.

Another way to enhance or guarantee uniqueness of primer and/or oligo molecules is to estimate their expected frequency in the genome based upon a Markov model of order n for the human genome or to check their uniqueness explicitly by counting their exact occurrence. The estimation based upon the Markov model relies upon the determination of the probabilities of all 4n n-mers (oligo molecules of n nucleotides) in the human genome or in all amplificates which are used in the hybridization and the conditional probabilities of all four bases given these n-mers. The primer pairs will be constructed from forward and reverse oligos which lie within an appropriate distance to each other and which have minimal individual expected occurrence elsewhere in the genome.

A second challenge in primer design for bisulfite treated DNA is that the melting temperature TM of a bisulfite DNA primer of a certain length is typically lower than the melting temperature TM of a standard primer containing cytosines. This is due to the fact that every cytosine in a bisulfite treated DNA is - after amplification by PCR - replaced by thymine. Cytosine binds its corresponding base guanine via three hydrogen bonds, whereas thymine binds its corresponding base adenine via two hydrogen bonds only, leading to a generally weaker binding, a lower TM.

A third problem arises from the fact that bisulfite treated sequences are not only lacking cytosines but are also thymine-rich. Thymine also hybridizes unspecifically with guanine. This makes mismatching (unspecific binding of a primer to a sequence not identical) of a primer designed for bisulfite treated DNA much more likely than mismatching of a standard primer consisting of four different nucleotides.

It is the aim of this invention to overcome these problems, which are specific for primer based amplification of bisulfite treated DNA.

For a so called "multiplex PCR" it becomes especially difficult to design primer pairs. This expression is used to describe an experiment in which several different pieces of DNA are amplified simultaneously, in one reaction vessel and at the same time. Obviously this saves a lot of effort and time and is as such a basic requirement for high throughput assays based on PCR amplification. An overview on the state of the art concerning multiplex PCR is given by Henegariu et al. (Henegariu O, Heerema NA, Dlouhy SR, Vance GH and Vogt PH (1997) Multiplex PCR: Critical Parameters and Step-by-Step Protocol. BioTechniques 23: 504-511), who offer a step-by-step protocol on how to tackle multiplex PCR problems. However, the possibility of a special primer design is not mentioned in this article.

To ensure that the multiplex PCR works and the multiple products are amplified indeed usually a gel electrophoresis of the reaction mixture is performed. The products get separated due to their different sizes. Unfortunately, the ability of agarose gel electrophoresis to distinguish the products is slightly limited. However, it is possible to test for different product sizes with the means of a fragment analyzer, which is much more accurate and able to distinguish product sizes of one base difference. Hence different product sizes are no longer a requirement to be considered in the primer design for a multiplex PCR.

In patent WO 01/94634 a method for a multiplex PCR using at least two primer pairs is described that consists of basically a two step amplification procedure wherein one step is referred to as pre-amplification. After pre-amplification (by means of PCR) with a number of primer pairs the sample gets divided into as many portions as there are primer pairs. At least one (and preferably only one) of the previously used primer pairs is added. This method doesn't relate in any way to the selection or design of primer molecules described herein.

In an article by Shuber et al. (Shuber AP, Grondin VJ and Klinger KW (1995) A simplified procedure for developing multiplex PCRs. Genome Res 5 (5) : 488-493 ) regarding multiplex PCR, the authors suggest to use primers, which contain a 3' region complementary to sequence specific recognition sites and a 5' region of a defined length of 20 nucleotides each. The authors claim that they could establish identical reaction conditions, cycling times and annealing temperatures for any PCR primer pair following those requirements.

In several recent papers successful multiplex PCRs have been established. For example, Becker et al. have reported the development of a multiplex PCR reaction for the detection of multiple staphylococcal enterotoxin genes, which uses individual primer sets for each toxin gene (Becker K, Roth R and Peters G (1998) Rapid and specific detection of toxigenic Staphylococcus aureus : use of two multiplex PCR enzyme immunoassays for amplification and hybridization of staphylococcal enterotoxin genes, exfoliative toxin genes, and toxic shock syndrome toxin 1 gene. J. Clin. Microbiol. 36: 2548-2553). This has been developed even further by Monday and Bohach, by increasing the number of primer pairs applied in one reaction up to about 10 in order to have one assay to amplify all of the characterized enterotoxin genes. This still required a unique established primer pair for the detection of every individual gene (Monday SR and Bohach GA (1999) Use of multiplex PCR to detect classical and newly described pyrogenic toxin genes in staphylococcal isolates. J. Clin. Microbiol. 37: 3411-3414).

In another paper by Sharma et al. a method for a one-vessel-multiplex PCR is described wherein each of six chosen primer pair consists of one identical universal forward primer, based on a highly conserved region of those genes of interest and one reverse primer, specific for each individual gene. As such the assay leads to a rapid amplification of a family of genes, which all have a conserved region in common. It is designed to detect presence or absence of certain genes in an unknown mixture. No further information is given about the primer design, apart from saying that they were designed by alignment of published DNA sequences. This is certainly not the only requirement though, as one big limitation of the method is the need of getting PCR products of different sizes in order to identify those in the end (Sharma NK, Rees CED and Dodd CER (2000) Development of a single-reaction multiplex PCR toxin typing assay for Staphylococcus aureus strains. Applied and Environmental Microbiology 66 (4) : 1347-1353).

In the patent application WO 01/36669 a method is described which uses a similar approach for the controllable amplification of a higher number of sequences in selecting one randomly chosen reverse primer that hybridizes unspecifically and a number of specific forward primers to amplify a group of sequences. As the reverse primer is labeled all products formed will be labeled as well. By hybridizing said amplicons towards immobilized detection oligos, which are able to differentiate the products, it will be easy to see which products have been amplified and herein the presence or absence of said sequences in the mixture can be determined.

The big disadvantage in all these methods is that every primer pair needs to be established individually first to ensure that a PCR product of the expected size was produced and that no additional or nonspecific products are generated. Once the specificity of the primer pairs had been determined, PCR conditions, buffers, and primer concentrations need to be optimized to establish conditions under which the primer molecules can be combined into one single PCR reaction without affecting the ability of the primer pairs to generate a gene specific amplicon.

A more recently published approach by Nicodeme and Steyaert describes the conditions required for multiplex PCR and suggests an algorithm to automatically select for primer pairs (Nicodeme P and Steyaert JM (1997) Selecting optimal oligonucleotide primers for multiplex PCR. Proc. Int. Conf. Intell Syst Mol Biol; 5 : 210-213). In this approach the conditions for pre-selecting primer pairs for a successful one locus amplification (singleplex PCR conditions) are rather broad. The three basic requirements are the pairing distance between a forward and a reverse primer, the condition of non-palindromicity of a primer, and the condition that the 3' end of a primer must not be reverse complementary to any of the other primers sequence. This selection is done with the help of a typical primer design program called PRIMER. However, PRIMER is a two step program, and in this approach the new method to design primers for a multiplex PCR takes the output from step 1 as input, which is a list of possible forward and a list of possible reverse primers for every amplificate.

The only further selection criteria for the multiplex PCR primers are the absence of the reverse complementarity of their 3' end towards the other primer sequences in the experiment. A second critical factor considered here is the GC versus AT ratio. To some extent it is this ratio that determines the melting temperature of a primer pair. The authors suggest to limit the GC/AT ratio to be inside a given range which would enable the simultaneous hybridization of several primer pairs at one reaction temperature. The final requirement is the electrophoresis distance, determined by the tool that is used to differentiate the PCR products in, for example, a gel electrophoresis. This most common method requires the products to be of different sizes. The whole concept of this method also requires to have a pool of possible primer pairs for each amplicon.

The design of suitable primers for a multiplex PCR on bisulfite treated DNA is an even greater challenge. The low complexity of the DNA, being reduced to essentially three different bases rather than four different bases, requires an extra careful selection of primers to avoid mismatching and unwanted amplification.

In the scope of this invention the word "mismatching" corresponds to the situation when the alignment of two sequences which are essentially complementary reveals positions in one of the sequences where the nucleotide base does not align with its corresponding base but a different one. The corresponding or complementary base pairs are adenine and thymine, cytosine and guanine, are adenine and thymine, cytosine and guanine, uracil and adenine. For example, a cytosine that aligns with a thymine in its otherwise complementary sequence creates a mismatch of one base or nucleotide.

Accordingly "base mismatches" refers to the situation of a base mismatching with another as explained above, respectively "one or more base mismatches" refers to one or more bases (in a given sequence) that cannot be aligned with their corresponding bases.

Also, when the alignment reveals single nucleotide gaps in one of the aligned sequences this is understood under the term "mismatch" in the scope of this invention.

A 'gap' is to be understood as follows: If an alignment reveals that, in order to get the highest number of corresponding base pairs aligned, some bases are lacking a corresponding base in its otherwise complementary sequence, this is called a gap. Such a gap can have a length of one or more nucleotides.

To solve the problems mentioned above we used a method consisting of several steps that is applicable for the amplification of nucleic acids in singleplex as well as in multiplex PCR experiments.

The method is comprised of the following steps:
Firstly, the nucleic acid sample containing the region of interest, which is to be amplified, is isolated. Secondly, this nucleic acid sample is treated in a manner that differentiates between methylated and un-methylated cytosine bases within said sample. Thirdly, a reaction mixture is set up containing a) the treated template nucleic acids, carrying the region of interest (also called: target nucleic acid) that is to be amplified, b) specified oligo-nucleotide primers, c) an enzyme capable of amplifying said nucleic acids in a defined manner, d) the necessary nucleotides required for the nucleic acid synthesis and e) a suitable buffer.

Said specified oligo-nucleotide primers are **characterized in that**
their sequences each reach a predefined measure of complexity (as described in detail below)
every possible combination of two primer molecules in said reaction mixture has a melting temperature below a specified threshold temperature
none of the possible combinations of two primer molecules in said reaction mixture leads to the amplification of an additional unwanted product as determined by virtual testing for amplification.

In the last step of the method said amplified target nucleic acid is detected by means commonly used by one skilled in the art.

We used a method for the amplification of nucleic acids comprising the following steps of isolating a nucleic acid sample, treating said sample in a manner that differentiates between methylated and unmethylated cytosine bases within said sample, amplifying at least one target sequence, within said treated nucleic acid, by means of enzymatic amplification and a set of primer molecules, wherein said primer molecules are **characterized in that**
a) each primer molecule sequence reaches a predefined measure of complexity, b) every combination of any two primer molecules in the set has a melting temperature below a specified threshold temperature and c) every combination of two primer molecules, under conditions allowing for one or more base mismatches per primer, does not lead to the amplification of an unwanted product when virtually tested using the treated and the untreated sample nucleic acids as template and the last step of detecting said amplified target nucleic acid.

### More detailed description of the method:

The method is comprised of the following steps:
In the first step of the method, the nucleic acid sample, which contains the region of interest that is to be amplified, must be isolated from tissue or cellular sources. Such sources may include at least one cell, but usually several cells, cell lines, histological slides, bodily fluids, or tissue embedded in paraffin.

In a preferred embodiment of this use the nucleic acid sample is isolated from a bodily fluid, a cell culture, a tissue sample or a combination thereof.

For example a certain kind of organ sample from a patient or an animal can be used to extract genomic DNA by the usually applied methods. Preferably, in this use DNA is extracted from a tissue sample or a biological fluid like blood, serum, urine or other fluids. 'Bodily fluid' herein refers to a mixture of macromolecules obtained from an organism. This includes, but is not limited to, blood, blood plasma, blood serum, urine, sputum, ejaculate, semen, tears, sweat, saliva, lymph fluid, bronchial lavage, pleural effusion, peritoneal fluid, meningal fluid, amniotic fluid, glandular fluid, fine needle aspirates, nipple aspirate fluid, spinal fluid, conjunctival fluid, vaginal fluid, duodenal juice, pancreatic juice, bile and cerebrospinal fluid. This also includes experimentally separated fractions of all of the preceding. 'Bodily fluid' also includes solutions or mixtures containing homogenized solid material, such as feces.

The nucleic acids may include DNA or RNA. Isolation may be by means that are standard to one skilled in the art, this includes for example extraction of DNA with the use of detergent lysates, sonification and vortexing with glass beads. An example is the extraction of DNA from a piece of a plant, like a leave or fruit. Once the nucleic acids, like genomic double stranded DNA, have been extracted they are used in the analysis.

In a preferred embodiment of this use the nucleic acid sample is comprised of plasmid DNA, BACs (bacterial artificial chromosomes), YACs (yeast artificial chromosomes) or genomic DNA.

In another especially preferred embodiment of this use the nucleic acid sample is comprised of human genomic DNA. It is preferred that the nucleic acids are of human origin.

In the second step, this nucleic acid sample is treated in a manner that differentiates between methylated and un-methylated cytosine bases within said sample. Cytosine bases which are unmethylated at the 5'-position are converted to uracil, thymine, or another base which is dissimilar to cytosine in terms of hybridization behavior. This will be understood as 'treatment' hereinafter. The method most commonly used so far is the so called bisulfite treatment.

This step is of essential meaning to the process as it translates the methylation pattern of said nucleic acids into a pattern that is something like an imprint of the methylation status itself. It contains essentially the same information but the pre-treated nucleic acids are no longer sensitive to amplification via PCR. Amplification via PCR does not differentiate between methylated and unmethylated cytosines and therefore leads to the loss of this level of information. The original methylation status however can be deducted whenever the described pre-treatment had been performed prior to the amplification step. Hence any means suitable to differentiate between a methylated and an un-methylated cytosine base are applicable, as long as the modified bases are still capable of being amplified by enzymatic means after treatment.

It is a preferred embodiment of this use that said sample is treated by means of a solution of a bisulfite, hydrogen sulfite or disulfite. A treatment of genomic DNA as described above is carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

In the third step of this method, a reaction mixture is set up containing a) the treated template nucleic acids, comprising the region of interest (also called target nucleic acid) that is to be amplified, b) specified oligonucleotide primers, c) an enzyme capable of amplifying said nucleic acids in a defined manner, for example a polymerase, d) the necessary nucleotides required for the nucleic acid synthesis and e) a suitable buffer. The template nucleic acid contains at least one target nucleic acid, which is amplified in the reaction. One primer molecule of the at least one primer pair in the reaction mixture is capable of binding to the 3' end of one specified target nucleic acid. The first primer binds to the 3' end of the target sequence, this primer is elongated and a complementary sequence to the target sequence is made. The polymerase stops to elongate unspecifically. The next cycle starts by thermally denaturing the now double stranded template nucleic acid into single stranded template nucleic acids. This is followed by the next phase of annealing when both primer molecules specifically bind to the target nucleic acid and its complementary strand. The second primer is identical to the 5' end of the target molecule. It doesn't bind to the target sequence itself but to said complementary nucleic acid to the target sequence, as soon as this is denatured from the template.

The process is finished by the actual amplification phase at a slightly lower reaction temperature, during which the enzyme, for example the polymerase elongates the primer as a complementary sequence to the target nucleic acid. The polymerase elongates this second primer by using the first copy as template until the end of said copied nucleic acid is reached. That way an identical copy to the original single stranded target nucleic acid is created. Hence, the length of the amplificate is determined by choosing the two primers.

The elongation products, being complementary to each other and hereby building a double stranded version of the target nucleic acid, serve as additional targets for the primer molecules binding in the next cycle of amplification.

Essentially step 3 of the method is comprised of amplifying at least one target sequence, within said treated nucleic acid, by means of enzymatic amplification and a set of primer molecules.

Said primer molecules used in said method are **characterized in that** they, in addition to fulfilling all the usual requirements towards a PCR primer as will be specified in more detail later, also fulfill the following requirements:
Firstly, the sequence of each primer molecule used in step 3 of this method reaches a predefined measure of complexity.

In a preferred embodiment of this method the primer molecules are reaching a certain value of linguistic complexity. A notion and a measure of linguistic complexity has been introduced by Trifonov in 1990 and has been used for analysis of nucleotide sequences before (Trifonov, EN (1990) Making sense of the human genome. In Structure & Methods. Vol 1 pp 69-77 (eds. Sarma, RH and Sarma MH, Adenine Press, Albany, US). The linguistic complexity technique allows a calculation to be made of the structural.complexity of any linear sequence of characters irrespective of whether the text is cognized or presently undeciphered. The sequences are compared exclusively from the point of view of their structural complexity with no reference to the meaning of the texts. In 1997 Trifonov published how the linguistic complexity of nucleosomal sequences is defined (Bolshoy, A; Shapiro, K; Trifonov, E and Ioshikhes I. (1997) Enhancement of the nucleosomal pattern in sequences of lower complexity. NAR 25 (16): 3248-3254). Quote: "The linguistic complexity measure exploits the major distinguishing feature between natural nucleotide sequences and uniformly random ones: the re-petitiveness of the natural sequences, i.e. the frequent repetition, not necessarily a tandem one, of some oligonucleotides ("words"), while others are avoided. (...) Complexity can be directly calculated as the extent to which the maximal possible vocabulary (all word sizes considered) is utilized in a given strength of sequence (...)."

In another preferred embodiment of this method said measure of complexity is set by the so called Shannon entropy (Shannon, C E, (1948) A Mathematical Theory of Communication, University of Illinois Press, Urbana). This is the most common measure to assess the information content (in a technical, non-semantic meaning) of linear information carriers. It attributes the maximal value (which can be chosen to be 1 without restrictions) to sequences where all symbols (characters) occur at equal probability and a value of 0 to sequences consisting of just one repeated symbol (character, letter). A derived and more general measure is the higher order Shannon entropy which attributes maximal value to sequences where all its subsequences occur at equal probability and a value of 0 or close to 0 to sequences consisting of periodic repetitions of short subsequences. The practical determination of the (higher order) Shannon entropy however is limited by the finite lengths of sequences which often does not permit a precise estimation of the probability distribution of their constitutive symbols.

Further possible measures are for example the Lempel-Ziv complexity (Lempel, LB and Ziv, J (1976) On the complexity of finite sequences. IEEE Trans. Inf. Theory IT-22, 75-81) , the grammar complexity (Ebeling, W; Jimenez-Montano, MA (1980) On Grammars, Complexity and Information Measures of Biologoical Macromolecules. Mathematical Bioscience 52, 53-71), the algorithmic complexity (Chaitin, 1990) and the conditial entropy.

Secondly, said primer molecules are also **characterized in that** every possible combination of any two primer molecules, in the set, has a melting temperature below a specified threshold temperature. That way the accumulation of dimers caused by the binding of two primer molecules to each other in said reaction mixture is excluded. The number of primer pairs used in that step can be any between one and n, leading to one or n amplificates respectively (n being a natural number).

As mentioned in the text the word "dimer" refers to a secondary structure formed by the hybridization of two primer molecules to each other.

As referred to in the text 'melting temperature' refers to the temperature at which 50% of the nucleic acid molecules are in duplex and 50% are denatured under standard reaction solution conditions.

Some primer design tools disqualify a primer if, besides the target sequence, a second identical sequence can be found in the template. However, due to the higher probability of a bisulfite primer to mismatch with non-identical bisulfite treated DNA, it is an embodiment of this use that only those primers are allowed to be used in said amplification method, for which no sequence homology can be found, to the extent that even those sequences that are different and/or mismatching in several nucleotides are excluded. However, this would exclude primer molecules unnecessarily. Therefore they are only excluded if two primer molecules match to the template in a distance allowing for the amplification of an unwanted product. This test is performed by means as, for example, the Electronic PCR. Electronic PCR (e-PCR) is an in silico virtual PCR carried out in order to assess the suitability of primer molecules prior to in vitro PCR. In the scope of this use this testing will be called 'virtual testing' and it will be referred to as "virtually tested" or "virtually testing".

Thirdly, the primers used in step 3 of this use are **characterized, in that** every possible combination of two primer molecules, in said reaction mixture, does not lead to the amplification of an additional unwanted product, when virtually testing for amplification using the treated and the untreated nucleic acid sample as template, even under conditions allowing for at least one base but not more than 20% of the total number of bases per sequence mismatching per primer. In the scope of this use it is to be understood that those primer molecules are considered to bind to the template for which a template sequence exists that is in at least 80% of its nucleotide sequence identical to the target sequence the primer originally has been designed for. For example, a primer molecule of 50 nucleotides length is considered to still hybridize to a template sequence that differs in less than 11 nucleotides (= is identical in at least 80% of its nucleotide sequence) from the according target sequence. If a match is considered to be possible it has to be tested whether this match would lead to the amplification of an unwanted product. This can be done with the use of a program similar to e-PCR (see below).

Especially preferred is an embodiment of said method wherein the ability of said primer molecules to amplify an unwanted product is tested by means of in silico PCR, taking as template nucleic acid the coding strand of the treated sample, the non-coding strand of the treated sample and both of the strands of the untreated sample. It is especially preferred to perform the virtual testing with a tool like electronic PCR on the pretreated, preferably bisulfite treated, template sequence consisting of the treated sense and the treated anti-sense strand, and on the unconverted template.

Furthermore it is preferred that this treatment is bisulfite treatment and hence the nucleic acid template is the bisulfite converted coding strand of the human genome, the bisulfite converted non-coding strand of the human genome and both of the strands of the untreated human genome. Preferred is an embodiment of said method wherein the ability of said primer molecules to amplify an unwanted product is tested by means of electronic PCR, hereby taking as template nucleic acid the bisulfite converted coding strand of the human genome, the bisulfite converted non-coding strand of the human genome and both of the strands of the untreated human genome.

It is preferred that the number of mismatches allowed for when virtually testing the amplification of unwanted products according to step 3 c) of the use is less than 20% of the number of nucleotides of the primer.

It is also preferred that the number of mismatches allowed for when virtually testing the amplification of unwanted products according to step 3 c) of the use is less than 10% of the number of nucleotides of the primer.

It is especially preferred that the number of mismatches allowed for when virtually testing the amplification of unwanted products according to step 3 c) of the use is less than 5% of the number of nucleotides of the primer.

It is a preferred embodiment of this use that the number of mismatches allowed for when virtually testing the amplification of unwanted products according to step 3 c) of the use is less than seven.

It is especially preferred that the number of mismatches allowed for when virtually testing the amplification of unwanted products according to step 3 c) of the use is less than five.

It is another preferred embodiment of this use that the number of mismatches allowed for when virtually testing the amplification of unwanted products according to step 3 c) of the use is less than three.

It is especially preferred in the scope of this use that the number of mismatches allowed for when virtually testing the amplification of unwanted products according to step 3 c) of the use is one.

It is also included in the scope of this use to consider such primer molecules as being sufficiently similar to facilitate their binding to the template sequence, for which a template sequence can be found that differs in the number of nucleotides but is otherwise identical to the target sequence. When the alignment of the primer and the template sequence leads to a gap of up to 20% of the nucleotides of one sequence, preferably of the primer sequence, this shall still be considered to be sufficient for binding and hence potentially leading to the amplification of an unwanted product. Therefore these primers also need to be tested with the means of virtual PCR (for example with a program like e-PCR). Only if this test reveals the virtual amplification of an unwanted product caused by the combination of two primers, the according primer pairs are excluded from the set of selected pairs.

It is preferred that the number of nucleotides creating one gap, in one of the sequences, when aligning the primer molecule sequence with the template sequence, allowed for when virtually testing the amplification of unwanted products according to step 3 c) of the use is less than 20% of the number of nucleotides of the primer molecule.

It is also preferred that the number of nucleotides creating one gap, in one of the sequences, when aligning the primer molecule sequence with the template sequence, allowed for when virtually testing the amplification of unwanted products according to step 3 c) of the use is less than 10% of the number of nucleotides of the primer molecule.

It is preferred that the number of nucleotides creating one gap, in one of the sequences, when aligning the primer molecule sequence with the template sequence, allowed for when virtually testing the amplification of un-wanted products according to step 3 c) of the use is less than 5% of the number of nucleotides of the primer molecule.

Both of these situations, mismatching due to an alternative nucleotide or no-matching due to a missing nucleotide, are meant to be covered in the expression describing those primer molecules that will eventually be selected : "said primer molecules are **characterized in that** every combination of two primer molecules, under conditions allowing for one or more base mismatches per primer, does not lead to the amplification of an unwanted product when virtually tested using the treated and the untreated sample nucleic acids as template".

It is also preferred that the primer molecules that exceed a pre-specified melting temperature when binding to the template have to be virtually tested for amplification of unwanted products using the treated and the untreated sample nucleic acids as template according to step 3 c) of the method.

The basic problem of finding a primer specific enough to give only one product on the little complex bisulfite DNA, is finally solved by testing each potential primer pair for hybridization across the whole bisulfite converted human genome. This requires translating the whole human genome sequence information virtually into its bisulfite treated version before performing a similarity search against the primer pairs, which is based on a method like the so called e-PCR (Schuler G.D. (1997) Sequence Mapping by electronic PCR. Genome Research 7(5): 541-550). However, as the bisulfite conversion results in two no longer complementary strands this virtual hybridization test needs to be done against both bisulfite converted strands. In addition in most cases the template DNA is contaminated with unconverted genomic DNA. To also exclude unwanted amplification on the unconverted DNA as template, the same hybridization test has to be performed a third time using the whole human genome sequence as a template.

Therefore it is a preferred embodiment of this use that the ability of said primer molecules to amplify an unwanted product is tested by means such as electronic PCR.

In the last step of the method said amplified target nucleic acid gets detected by any means standard to one skilled in the art.

In a preferred embodiment of this method the set of primer molecules is comprised of at least two primer molecules but not more than 64 primer molecules, given the number is a multiple of 2; in other words, the set is comprised of 1-32 primer pairs.

In another preferred embodiment of this method the set of primer molecules is comprised of between 2 and 32 primer molecules, given the number is a multiple of 2; in other words the set is comprised of 1-16 primer pairs.

In a preferred embodiment of this method, said primer molecule comprises at least one nucleotide within the last three nucleotides from the 3' end of the molecule, wherein said nucleotide is complementary to a nucleotide of the target sequence that, as a result of the treatment performed in step 2) of the use, changed its hybridization behavior.

It is a preferred embodiment of this method, that said primer molecule comprises at least one nucleotide within the last three nucleotides from its 3' end that is complementary to a nucleotide of the target sequence that was converted by the treatment performed in step 2 of the method to another base exhibiting an alternative base pairing behavior.

In an especially preferred embodiment said nucleotide is a cytosine prior to the treatment that converts unmethylated cytosines. In a preferred embodiment said treatment is bisulfite treatment. Said primer molecule comprises at least one nucleotide within the last three nucleotides from the 3' end of the molecule, wherein said nucleotide is complementary to a cytosine, that was converted by bisulfite treatment to another base exhibiting the base pairing behavior of thymine.

This is to exclude binding of said primer molecules to the remaining untreated or un-sufficiently treated nucleic acids, which might still serve as template nucleic acid in the PCR.

Furthermore it is a preferred embodiment of this use that said primer molecules do not form loops or hairpins on their own or with each other.

In another preferred embodiment of the method said primer molecules do not form dimers with each other.

In the text the word 'hairpin' is taken to mean a secondary structure formed by a primer molecule when the 3' terminal region of said nucleic acid hybridizes to the 5' terminal region of said nucleic acid forming a double stranded stem structure and wherein only the central region of the primer is single stranded.

As described in the text the word 'loop' refers to a secondary structure formed by a primer molecule when two or more nucleotides of said molecule hybridize thereby forming a secondary structure comprising a double stranded structure one or more base pairs in length and further comprising a single stranded region between said double stranded region.

The binding of a primer molecules 3' end to any part of a second primer molecule in the set needs to be avoided. Otherwise the polymerase would extend the first primer using the second primer as template, which would lead to a new unwanted product, an extended primer, or rather a primer-hybrid, which would serve as the preferred template for the next round of the polymerase chain reaction and thereby prevent a sufficient amplification of the wanted product.

Therefore it is another preferred embodiment of this method that each of said primer molecules is **characterized in that** the last at least 5 bases at the 3' end of said primer molecule are not complementary to the sequence of any other primer molecule in the set.

It is also preferred that said primer molecules do not bind to nucleic acids which prior to treatment of step 2 contained a 5'-CG-3' site. This would lead to a binding of the primers to bisulfite treated nucleic acids, specifically depending on their cytosines methylation status. A CG corresponding primer would bind to the treated methylated version only, whereas a primer corresponding to TG would bind to the treated unmethylated version of these nucleic acids only. It is therefore preferred that said primer molecules do not contain nucleic acid sequences complementary or identical to nucleic acid sequences which prior to treatment of step 2 contained a 5'-CG-3' site.

In a preferred embodiment of this method said primer molecules are of a specified size range.

It is especially preferred that these primers are comprised of 16-50 nucleotides.

In a preferred embodiment of this method said primer molecules do not comprise sequences that are complementary to regions of the target nucleic acids that contained specified restriction enzyme recognition sites prior to the treatment that altered the unmethylated cytosines base pairing behavior. It is preferred that said primers are complementary to target sequences which prior to the treatment performed in step 2 of the use did not contain specified restriction enzyme recognition sites.

By selecting for the right primer molecules also the amplificates sequence is determined. That is why it has to be taken into account to only use those primer molecules that lead to amplification of nucleic acids containing a reasonable high number of CpG sites to be analyzed. Due to the treatment of step 2 of this use these CpG sites, depending on the methylation status of the cytosine, are converted and will therefore either appear as CG dinucleotides or as TG dinucleotides in the amplificate.

It is preferred that said primer molecules amplify regions of nucleic acids that prior to bisulfite treatment comprise of more than eight 5'-CG-3' sites also referred to as CG dinucleotides.

It is also preferred that said primer molecules amplify regions of nucleic acids that prior to bisulfite treatment comprise of more than six 5'-CG-3' sites also referred to as CG dinucleotides.

It is also preferred that said primer molecules amplify regions of nucleic acids that prior to bisulfite treatment comprise of more than four 5'-CG-3' sites also referred to as CG dinucleotides and finally it is especially preferred that said primer molecules amplify regions of nucleic acids that prior to bisulfite treatment comprise of more than two 5'-CG-3' sites also referred to as CG dinucleotides.

Said primer molecules lead to amplificates within a specified size range.

It is a preferred embodiment of this sequence that said primer molecules lead to amplificates which are comprised of at least 50 bp but not more than 2000 bp.

Especially preferred are primer molecules that lead to amplificates which are comprised of at least 80 bp but not more than 1000 bp.

Furthermore a method is preferred wherein said primer molecules lead to amplificates of treated nucleic acids which prior to the treatment which altered the unmethylated cytosines base pairing behavior did not contain restriction enzyme recognition sites. Said primer molecules lead to amplificates that are amplified regions of the treated nucleic acids which prior to the treatment performed in step 2) of the method did not contain specified restriction enzyme recognition sites.

### SUMMARY OF THE INVENTION

A subject of the invention is a method for designing primer molecules to be used for amplification of nucleic acids which have been treated in a manner that differentiates between methylated and unmethylated cytosine bases, comprising the steps of
a) selecting a set of possible primer pairs per amplificate by means of a PCR primer design program using said treated nucleic acids as template;
b) excluding those amplifying primer pairs which comprise of a primer that in combination with another primer molecule in the same set exceeds a threshold melting temperature, when hybridising with said another primer molecule;
c) excluding those primer pairs which comprise of a primer that does not reach a specified minimum level of complexity;
d) virtually testing in silica all possible combinations of the resulting primers in the set for whether or not an unwanted product is amplified using the treated and the untreated sample nucleic acid as template, under conditions allowing for one or more base mismatches per primer until up to a number of mismatching nucleotides of 20% of the number of nucleotides of the primer molecule;
e) excluding those primer pairs which comprise of a primer that in combination with another primer molecule in the same set, under conditions allowing for one or up to 20% base mismatches per primer, amplifies an unwanted product when virtually tested in silica using the treated and the untreated nucleic acid as template.

A subject of this invention is a method on how to produce said primer molecules. The main step of producing a primer molecule is determining its sequence. In the following the phrase "primer design" will be used instead of primer production, whenever it is referred to the step of determining said specific primer sequences. Designing primer molecules is a process which as such is well known to scientists skilled in the art. The programs usually used for this purpose are such as PRIMER3 or OSP (Rozen S and Skaletsky H (2000) PRIMER3 on the WWW for general users and for biologist programmers. Methods Mol Biol 132: 365-386; Hillier L and Green P (1991) OSP: A computer program for choosing PCR and DNA sequencing primers. PCR Methods and Applications 1: 124-128). Other primer design systems (like described in EP-A 1136932) are often based on those commonly known programs.

An embodiment of this invention takes advantage of using a program like PRIMER3 first, to then add a number of steps that finally result in an advanced method of designing primers that are specifically useful for amplifying sequences of low complexity.

In the first step of this method for designing specific primer molecules for nucleic acids of low complexity, primer pairs that amplify single products are selected by applying standard tools of primer design known in the art, like for example the program PRIMER3 (Rozen, S and Skaletsky, H (2000) Methods Mol Biol 132: 365-386).

In the second step of the method said primer pairs are tested whether or not one of its primer molecules when hybridizing to any other primer molecule in the set exceeds a specified threshold melting temperature TM. If this is the case the primer pair that comprises of said primer is excluded from the set of potentially combined pairs.

In the third step of the method the number of previously selected primer pairs, is reduced to a smaller number by implementing as new criteria a measure for the primer sequence's complexity. Primer pairs that consist of a primer molecule which does not meet said criteria are excluded.

The basic problem of finding a primer specific enough to give only one product on the little complex bisulfite DNA, is finally solved by testing each potential primer pair for hybridization across the whole bisulfite converted human genome. This requires translating the whole human genome sequence information virtually (as in "in silico") into its treated, for example bisulfite treated, version before performing a similarity search against the primer pairs, which is based on a method like the so called e-PCR (Schuler G.D. (1997) Sequence Mapping by electronic PCR. Genome Research 7(5): 541-550). However, as the bisulfite conversion results in two different versions of the double helix whose sense and anti-sense strands are no longer mutually complementary, this in silico amplification needs to be performed on both bisulfite converted versions of the genome. In addition in most cases the template DNA is contaminated with unconverted genomic DNA. It cannot be excluded that single cytosines or longer runs of DNA remain unconverted or are only converted incompletely by the bisulfite treatment. To also exclude unwanted amplification of the unconverted DNA as template, the same hybridization test has to be performed a third time using the whole human genome sequence as a template.

As this is quite some effort and requires time (CPU time) this is the fourth and last step of this design method, that is absolved prior to the final testing in a "wet", lab based, experiment.

In addition to improve the specificity of said primer molecules the stringency of the selection criteria is increased: Some standard primer design tools disqualify a primer if in the template sequence, a second identical sequence, besides the target sequence, can be found. That way mispriming at rather stringent hybridization conditions is avoided. This mispriming would not necessarily lead to an additional unwanted product, but would lead to the dilution of the primer molecules available for amplification. This selection has been performed in step one already (for example by PRIMER3). However, due to the higher probability of a bisulfite primer molecule to mismatch with non-identical bisulfite treated DNA, there is still a chance for said primer molecules to misprime even when up to 20% of the nucleotides of the primer sequence differ. Therefore it is claimed in this invention to only use primer molecules for which not even a weak sequence homology can be found. However, this would exclude primer molecules unnecessarily. Therefore they are only excluded if two primer molecules match to the template and amplify an unwanted product. This test is performed by means as, for example, the Electronic PCR. Electronic PCR (e-PCR) is an in silico virtual PCR carried out in order to asses the suitability of primers prior to in vitro PCR.

In the fourth step of the method on how to design these primers it is therefore tested whether there are any regions of the template nucleic acid, said template being comprised of the sense and the anti-sense strand of the treated and the untreated nucleic acids, that are identical in sequence with the primer molecule to more than 80% and if those primer molecules are able to amplify an unwanted product. If this is the case, the primer pair comprising said primer molecule is excluded from the selection.

The template nucleic acid is comprised of the treated template nucleic acid and the untreated template nucleic acid. The treated nucleic acid in itself is comprised of a two strands which after treatment are not complementary to each other anymore. This virtual testing for example can be performed as described by Gregory Schuler in his article (cited above) about sequence mapping by "Electronic PCR". The primer pairs remaining can be used to specifically amplify regions of nucleic acids of low complexity, which is the aim of this invention. Hence step 4 of the design method is the virtual testing of each possible primer pair combination, under pre-specified conditions at a stringency allowing for one or more base pair mismatches, as to whether no unwanted nucleic acids are amplified. Said virtual testing is carried out upon both untreated and treated nucleic acids. The wording "possible combinations" refers to all combinations that are possible within a set of primer pairs to be used in one amplification reaction vessel.

In a preferred embodiment an additional step is added following the virtual testing, which is testing in a lab based single PCR assay all those pairs that remained, whether the desired amplificate can be obtained or not. If that is the case, the chosen pairs can be used to specifically amplify those regions of nucleic acids of low complexity according to the method as described before.

In a specially preferred embodiment the first step of the design method is characterized as selecting a pool of possible primer pairs per amplificate by means of a standard PCR primer design program using said nucleic acids as template that have been masked for repeats and SNPs considering the following factors:
length of amplificate, length of primer, melting temperature of the primer molecule, dimer formation parameters, loop formation parameters, exclusion of unidentified or ambiguous nucleotides in the primer sequence, exclusion of restriction enzyme recognition sites.

In a preferred embodiment of this invention this measure of complexity is a measure of linguistic complexity as defined by Bolshoy et al. (see above). Those primer pairs are excluded from the previously selected ones, which comprise of one primer that doesn't reach a set level of this linguistic complexity.

In another preferred embodiment of this invention this measure of complexity is a measure of Shannon entropy (as described before).

In an especially preferred embodiment of this design method, prior to performing step d) the additional step of excluding primer pairs from the remaining primer pairs which consist of a primer molecule that comprises of at least one CpG site, is carried out.

In an especially preferred embodiment of this method according to the design of said primers, prior to performing step d) the additional step of excluding primer pairs from the remaining pairs when one of its primer molecules does not contain at least one nucleotide within the last three nucleotides from the 3' end of the molecule wherein said nucleotide is complementary to a nucleotide of the target sequence that was converted to a different nucleotide by bisulfite treatment, is carried out.

In an especially preferred embodiment of this method according to the production of said primers, prior to performing step d) the additional step, of excluding primer pairs from the remaining primer pairs which amplify a nucleic acid that did not prior to treatment with bisulfite contain a minimum of two CpG sites, is carried out.

In an especially preferred embodiment of this method according to the production of said primers, prior to performing step d) the additional step of excluding primer pairs from the remaining primer pairs when one of its primer molecules contains more than 5 bases at its 3' end that are complementary to any other primer molecules sequence in the set, is carried out.

In an especially preferred embodiment of this method according to the production of said primers, prior to performing step d) the additional step of excluding from the remaining primer pairs those pairs, which comprise of one primer molecule that in combination with another primer molecule in the set amplifies an unwanted product, when virtually testing according to step 3 c) of the amplification method under conditions allowing for a number of mismatching nucleotides of 20% of the number of nucleotides of the primer molecule, is carried out.

In an especially preferred embodiment of this method according to the production of said primers, prior to performing step d) the additional step of excluding from the remaining primer pairs those pairs, which comprise of one primer molecule that in combination with another primer molecule in the set amplifies an unwanted product, when virtually testing according to step 3 c) of the amplification method under conditions allowing for a number of nucleotides creating one gap, when aligning the primer molecule sequence with the template sequence, of up to 20% of the number of nucleotides of the primer molecule, is carried out.

In an especially preferred embodiment of this method according to the production of said primers, prior to performing step d) the additional step of excluding from the remaining primer pairs those pairs, which comprise of one primer molecule that in combination with another primer molecule in the set amplifies an unwanted product, when virtually testing according to step 3 c) of the amplification method under conditions allowing for four or less mismatching base pairs, is carried out.

In an especially preferred embodiment of this method according to the production of said primers, prior to performing step d) the additional step of excluding from the remaining primer pairs those pairs, which comprise of one primer molecule that in combination with another primer molecule in the set amplifies an unwanted product, when virtually testing according to step 3 c) of the amplification method under conditions allowing for two or less mismatching base pairs, is carried out.

The following example is intended to illustrate the invention :

### Example

Here we present experimental data that shows that multiplex PCRs designed with a tool according to this invention are more successful compared to multiplex PCRs not designed in this manner.

It is the aim of the experiment to amplify 40 different nucleic acids. The genomic regions of interest are given in the sequence protocol (SEQ ID 41-80). These genomic sequences were translated into their bisulfite converted versions and served as templates for amplification of specific regions with the primer sequences described as follows.

Primer molecule pairs used for single PCRs were originally designed with the use of the standard primer design program PRIMER3 (as mentioned in the description). The criteria used in that step will not be discussed in detail. This selection however provides several possible primer pairs per amplificate. Following the present invention these primer pairs were selected further, according to the following criteria:
- The restriction enzyme recognition site to be excluded from the genomic nucleic acid (which subsequent to bisulfite conversion becomes the template for the PCR amplification step) is : GTTTAAAC.
- The minimum length of the primer molecule is 18 nucleotides. The maximum length is 27 nucleotides. Ideally the primer consists of 22 nucleotides.
- The minimum required measure of linguistic complexity is 0.2.
- The minimum melting temperature of a primer molecule is 54°C and the maximum melting temperature is 57°C. The ideal melting temperature however is 55°C.
- The minimum length of an amplificate is 100 bp and the maximum length is 500 bp.
- The minimum number of CpG sites, that were present in the region of the nucleic acid, prior to bisulfite treatment, that was amplified is 4.
- The number of mismatch bases allowed for when virtually testing the primer pairs according to the invention for amplification of an unwanted product with the help of e-PCR (Electronic PCR) is 2.

The use of this invention, that is the use of either the design method, being the subject of the invention, and/or performing the steps of said method as described above (assuming a set size of 1) leads to the selection of the following 40 optimized primer molecule pairs:

**TABLE 1:**

| primer sequence | amplificate identifier | SEQ ID | number indicating primer direction | starting position of primer in the bisulfite converted sequence of the ROI |
|---|---|---|---|---|
| AATCCTCCAAATTCTAAAAACA | 2025 | 81 | 0 | 1816 |
| AGGAAAGGGAGTGAGAAAAT | 2025 | 82 | 1 | 2138 |
| GGATAGGAGTTGGGATTAAGAT | 2044 | 83 | 0 | 2070 |
| AAATCTTTTTCAACACCAAAAT | 2044 | 84 | 1 | 2483 |
| AACCCTTTCTTCAAATTACAAA | 2045 | 85 | 0 | 1340 |
| TGATTGGGTTTTAGGGAAATA | 2045 | 86 | 1 | 1687 |
| TTGAAAATAAGAAAGGTTGAGG | 2106 | 87 | 0 | 1481 |
| CTTCTACCCCAAATCCCTA | 2106 | 88 | 1 | 1764 |
| TGTTTGGGATTGGGTAGG | 2166 | 89 | 0 | 2226 |
| CATAACCTTTACCTATCTCCTCA | 2166 | 90 | 1 | 2437 |
| TTTTAGATTGAGGTTTTAGGGT | 2188 | 91 | 0 | 101 |
| ATCCATTCTACCTCCTTTTTCT | 2188 | 92 | 1 | 598 |
| GGAGGGGAGAGGGTTATG | 2191 | 93 | 0 | 133 |
| TACTATACACACCCCAAAACAA | 2191 | 94 | 1 | 506 |
| TTGGGAATGGGTTGTAT | 2194 | 95 | 0 | 1628 |
| CTACCCTTAACCTCCATCCTA | 2194 | 96 | 1 | 1996 |
| TTGTTGGGAGTTTTTAAGTTTT | 2212 | 97 | 0 | 1711 |
| CAAATTCTCCTTCCAAATAAAT | 2212 | 98 | 1 | 2063 |
| GTAATTTGAAGAAAGTTGAGGG | 2267 | 99 | 0 | 1709 |
| CCAACAACTAAACAAAACCTCT | 2267 | 100 | 1 | 2004 |
| GGAGTTGTATTGTTGGGAGA | 2317 | 101 | 0 | 1110 |
| TAAAACCCCAATTTTCACTAA | 2317 | 102 | 1 | 1388 |
| TTTGTATTAGGTTGGAAGTGGT | 2383 | 103 | 0 | 1 |
| CCCAAATAAATCAACAACAACA | 2383 | 104 | 1 | 285 |
| GATTTTTGGAGAGGAAGTTAAG | 2387 | 105 | 0 | 789 |
| AAAACTAAAAACCAAACCCATA | 2387 | 106 | 1 | 1169 |
| TGGGGTTAGTTTAGGATAGG | 2391 | 107 | 0 | 1353 |
| CTTAAAAACACTAAAACTTCTCAAA | 2391 | 108 | 1 | 1750 |
| TTTTTGTATTGGGGTAGGTTT | 2395 | 109 | 0 | 547 |
| CCCAACTATCTCTCTCCTCTATAA | 2395 | 110 | 1 | 1094 |
| ATTAGAAGTGAAAGTAATGGAATTT | 2401 | 111 | 0 | 381 |
| TCAATTTCCAAAAACCAAC | 2401 | 112 | 1 | 795 |
| GGGATGGGTTATTAGTTGTAAA | 2453 | 113 | 0 | 1867 |
| CCTTCACACAAAACTACAAAAA | 2453 | 114 | 1 | 2139 |
| TAATTGAAGGGGTTAATAGTGG | 2484 | 115 | 0 | 1861 |
| AAAACCAAAACCAAAACTAAAA | 2484 | 116 | 1 | 2252 |
| AGTGGATTTGGAGTTTAGATGT | 2512 | 117 | 0 | 1016 |
| AACAAAATAAAAACTTCTCCCA | 2512 | 118 | 1 | 1446 |
| TAGGGGAAAAGTTAGAGTTGAG | 2741 | 119 | 0 | 1413 |
| CCCATTAACCCACAAAAA | 2741 | 120 | 1 | 1888 |
| ATTTTAGTTTGTGAAATGGGAT | 2745 | 121 | 0 | 1685 |
| TCTTAACCAATAACCCCTCAC | 2745 | 122 | 1 | 2097 |
| GTGGGTTTTGGGTAGTTATAGA | 2746 | 123 | 0 | 1679 |
| TAACCTCCTCTCCTTACCAA | 2746 | 124 | 1 | 2163 |
| TAGGATGGGGAGAGTAATGTTT | 2747 | 125 | 0 | 972 |
| ACAACTTATCCAACTTCCATTC | 2747 | 126 | 1 | 1448 |
| TCCCACAAAAACTAAACAATTA | 2749 | 127 | 0 | 1370 |
| AGGTTTTAGATGAAGGGGTTT | 2749 | 128 | 1 | 1789 |
| TTTGGAGGGTTTAGTAGAAGTTA | 2751 | 129 | 0 | 88 |
| CCCAATAATCACAAAATAAACA | 2751 | 130 | 1 | 567 |
| ATACAACCTCAAATCCTATCCA | 2752 | 131 | 0 | 228 |
| AGGGAGAAGGAAGTTATTTGTT | 2752 | 132 | 1 | 712 |
| GGAAGATGAGGAAGTTGATTAG | 2755 | 133 | 0 | 1000 |
| CCTACAACCCTATCCTCTAAAA | 2755 | 134 | 1 | 1371 |
| TTAGTAGGGGTGTGAGTGTTTT | 2831 | 135 | 0 | 1313 |
| CAAACAAAACTTCTATCTCAACC | 2831 | 136 | 1 | 1499 |
| TTATAGGGTTGAGTTTGGGAT | 2850 | 137 | 0 | 2100 |
| TAAACAAACAACAAATCTTCCA | 2850 | 138 | 1 | 2400 |
| TGAAAATGAAGGTATGGAGTTT | 2852 | 139 | 0 | 1262 |
| TTAAAACCATATAATCCCTCCA | 2852 | 140 | 1 | 1583 |
| TATGTTTGGTTTTGTTTTGAGA | 2859 | 141 | 0 | 1093 |
| AACCCCATCACTTTTATTTCTT | 2859 | 142 | 1 | 1491 |
| GGGTGTAGAAGTGTTTAGGTTT | 2861 | 143 | 0 | 2385 |
| TTTCTCCCCTTACAACAATAAC | 2861 | 144 | 1 | 2732 |
| TCCCCTTCCAACTATATCTCTC | 2864 | 145 | 0 | 884 |
| TGAGAGTGTTTTAGGGAAGTTT | 2864 | 146 | 1 | 1175 |
| AAAACCAAAACATAAACCAAAA | 2867 | 147 | 0 | 1312 |
| GATTAGGAGGGTTTGTTGAGAT | 2867 | 148 | 1 | 1701 |
| AATGGTTGATGATTTTGGTTT | 2961 | 149 | 0 | 2039 |
| ACTCTCTTCCCTATACCCCTAA | 2961 | 150 | 1 | 2311 |
| AGTTAGAAGAGGAGTTAGGATGG | 3511 | 151 | 0 | 1340 |
| TAATTTTCCAATACCCATTTTC | 3511 | 152 | 1 | 1711 |
| TGTTAGTAGAGTTTTAGGGAGGTT | 3532 | 153 | 0 | 1135 |
| ACACTACCTATCCTTACCCCAC | 3532 | 154 | 1 | 1592 |
| TTTTTGTTTTTATGGGGTGTAT | 3534 | 155 | 0 | 1909 |
| TTAAATATCCCTTCCTTAACCA | 3534 | 156 | 1 | 2385 |
| TGGGTAGTATTTTTGTTGGTTT | 3538 | 157 | 0 | 956 |
| CCTAAAAACTCTCTCATCCTCA | 3538 | 158 | 1 | 1414 |
| AGTGGTTTAGGAGTATTTGGTTA | 3540 | 159 | 0 | 659 |
| AACTCCCTCCATCTACAATATC | 3540 | 160 | 1 | 1064 |

These primer pairs lead to the amplification of specific regions (amplificates Seq IDs 1- 40) of the bisulfite converted sequences of the genomic ROIs (Seq IDs 41- 80) of interest. The ROIs can be identified by the four digit number that specifies the ROI and the corresponding amplificate - as indicated in the following table.

**TABLE 2:**

| **SEQ ID** | **Class** | **Identifier** | **Kind of DNA** | | **SEQ ID** | **Class** | **Identifier** | **Kind of DNA** |
|---|---|---|---|---|---|---|---|---|
| 1 | amplificate | 2025 | bisulfite sequence | | 41 | ROI | 2025 | genomic sequence |
| 2 | amplificate | 2044 | bisulfite sequence | | 42 | ROI | 2044 | genomic sequence |
| 3 | amplificate | 2045 | bisulfite sequence | | 43 | ROI | 2045 | genomic sequence |
| 4 | amplificate | 2106 | bisulfite sequence | | 44 | ROI | 2106 | genomic sequence |
| 5 | amplificate | 2166 | bisulfite sequence | | 45 | ROI | 2166 | genomic sequence |
| 6 | amplificate | 2188 | bisulfite sequence | | 46 | ROI | 2188 | genomic sequence |
| 7 | amplificate | 2191 | bisulfite sequence | | 47 | ROI | 2191 | genomic sequence |
| 8 | amplificate | 2194 | bisulfite sequence | | 48 | ROI | 2194 | genomic sequence |
| 9 | amplificate | 2212 | bisulfite sequence | | 49 | ROI | 2212 | genomic sequence |
| 10 | amplificate | 2267 | bisulfite sequence | | 50 | ROI | 2267 | genomic sequence |
| 11 | amplificate | 2317 | bisulfite sequence | | 51 | ROI | 2317 | genomic sequence |
| 12 | amplificate | 2383 | bisulfite sequence | | 52 | ROI | 2383 | genomic sequence |
| 13 | amplificate | 2387 | bisulfite sequence | | 53 | ROI | 2387 | genomic sequence |
| 14 | amplificate | 2391 | bisulfite sequence | | 54 | ROI | 2391 | genomic sequence |
| 15 | amplificate | 2395 | bisulfite sequence | | 55 | ROI | 2395 | genomic sequence |
| 16 | amplificate | 2401 | bisulfite sequence | | 56 | ROI | 2401 | genomic sequence |
| 17 | amplificate | 2453 | bisulfite sequence | | 57 | ROI | 2453 | genomic sequence |
| 18 | amplificate | 2484 | bisulfite sequence | | 58 | ROI | 2484 | genomic sequence |
| 19 | amplificate | 2512 | bisulfite sequence | | 59 | ROI | 2512 | genomic sequence |
| 20 | amplificate | 2741 | bisulfite sequence | | 60 | ROI | 2741 | genomic sequence |
| 21 | amplificate | 2745 | bisulfite sequence | | 61 | ROI | 2745 | genomic sequence |
| 22 | amplificate | 2746 | bisulfite sequence | | 62 | ROI | 2746 | genomic sequence |
| 23 | amplificate | 2747 | bisulfite sequence | | 63 | ROI | 2747 | genomic sequence |
| 24 | amplificate | 2749 | bisulfite sequence | | 64 | ROI | 2749 | genomic sequence |
| 25 | amplificate | 2751 | bisulfite sequence | | 65 | ROI | 2751 | genomic sequence |
| 26 | amplificate | 2752 | bisulfite sequence | | 66 | ROI | 2752 | genomic sequence |
| 27 | amplificate | 2755 | bisulfite sequence | | 67 | ROI | 2755 | genomic sequence |
| 28 | amplificate | 2831 | bisulfite sequence | | 68 | ROI | 2831 | genomic sequence |
| 29 | amplificate | 2850 | bisulfite sequence | | 69 | ROI | 2850 | genomic sequence |
| 30 | amplificate | 2852 | bisulfite sequence | | 70 | ROI | 2852 | genomic sequence |
| 31 | amplificate | 2859 | bisulfite sequence | | 71 | ROI | 2859 | genomic sequence |
| 32 | amplificate | 2861 | bisulfite sequence | | 72 | ROI | 2861 | genomic sequence |
| 33 | amplificate | 2864 | bisulfite sequence | | 73 | ROI | 2864 | genomic sequence |
| 34 | amplificate | 2867 | bisulfite sequence | | 74 | ROI | 2867 | genomic sequence |
| 35 | amplificate | 2961 | bisulfite sequence | | 75 | ROI | 2961 | genomic sequence |
| 36 | amplificate | 3511 | bisulfite sequence | | 76 | ROI | 3511 | genomic sequence |
| 37 | amplificate | 3532 | bisulfite sequence | | 77 | ROI | 3532 | genomic sequence |
| 38 | amplificate | 3534 | bisulfite sequence | | 78 | ROI | 3534 | genomic sequence |
| 39 | amplificate | 3538 | bisulfite sequence | | 79 | ROI | 3538 | genomic sequence |
| 40 | amplificate | 3540 | bisulfite sequence | | 80 | ROI | 3540 | genomic sequence |

The second task in this example is to select from these 40 primer pairs those pairs which can be combined in five multiplex PCRs to amplify eight targets simultaneously.

The following steps, as disclosed in the invention, are performed for selection of those subsets:
- The melting temperature of any combination of two of those primer molecules hybridizing to each other taking part in one multiplex experiment must be below 20°C.
- The last seven nucleotides from the 3' end of every primer molecule in a subset is used to check if those are complementary and/or binding to any other primer molecules' sequence used in the set.
- The number of mismatch bases allowed for when virtually testing the primer pairs for amplification of an unwanted product is 2. For this step every possible combination of 16 primer molecules in one subset is checked for its ability to amplify an unwanted product. This is done by means of e-PCR (electronic PCR).

Having performed all these steps results in the selection of three different optimized sets of primer molecule pairs that can be used in multiplex PCRs. These sets are in the following described as a set of numbers. Each number refers to a specific amplificate and therefore also to a single primer pair (out of the list given above) which proved to be able to specifically amplify said nucleic acid in a single PCR experiment.

**TABLE 3:**

| **optimized set 1** | | | | | | | | |
|---|---|---|---|---|---|---|---|---|
| 8plexl | 2194 | 2191 | 2391 | 2025 | 2961 | 3540 | 2861 | 2188 |
| 8plex2 | 2484 | 2106 | 2401 | 2850 | 3532 | 2044 | 2512 | 2852 |
| 8plex3 | 2453 | 2741 | 2867 | 2755 | 2267 | 2387 | 2864 | 2317 |
| 8plex4 | 2859 | 2383 | 2752 | 2747 | 2751 | 3511 | 2212 | 2746 |
| 8plex5 | 3534 | 2395 | 2745 | 3538 | 2749 | 2166 | 2831 | 2045 |
| | | | | | | | | |

| **optimized set 2** | | | | | | | | |
|---|---|---|---|---|---|---|---|---|
| 8plexl | 2166 | 2212 | 3511 | 2383 | 2745 | 2859 | 3534 | 2861 |
| 8plex2 | 2749 | 2191 | 2751 | 2395 | 2961 | 2512 | 2831 | 3538 |
| 8plex3 | 2850 | 2025 | 2188 | 2317 | 2391 | 2852 | 3540 | 2194 |
| 8plex4 | 2106 | 2387 | 2867 | 2864 | 2401 | 2747 | 2746 | 2453 |
| 8plex5 | 2044 | 2484 | 2267 | 2755 | 2752 | 2741 | 2045 | 3532 |
| | | | | | | | | |

| **optimized set 3** | | | | | | | | |
|---|---|---|---|---|---|---|---|---|
| 8plex1 | 2194 | 2391 | 2191 | 2749 | 2745 | 3538 | 2861 | 2961 |
| 8plex2 | 2166 | 2188 | 2859 | 2212 | 2864 | 2746 | 2383 | 2752 |
| 8pal-3 | 2484 | 2401 | 2850 | 2852 | 2512 | 2755 | 2106 | 2044 |
| 8plex4 | 2867 | 2453 | 3532 | 2025 | 2741 | 2267 | 2317 | 2387 |
| 8plex5 | 3511 | 3534 | 2751 | 2747 | 2395 | 3540 | 2831 | 2045 |

Without the use of said invention, the selection would have been performed randomly and tested for successful application later. Three randomly chosen subsets are shown here.

**TABLE 4:**

| **random set 1** | | | | | | | | |
|---|---|---|---|---|---|---|---|---|
| 8plex1 | 2191 | 2194 | 2267 | 2741 | 3534 | 3511 | 2749 | 2747 |
| 8plex2 | 2391 | 2484 | 2867 | 2852 | 2453 | 2512 | 2025 | 3538 |
| 8plex3 | 2746 | 2212 | 2755 | 2045 | 2044 | 2188 | 2961 | 2864 |
| 8plex4 | 2831 | 2383 | 3540 | 2859 | 2861 | 2395 | 2401 | 2317 |
| 8plex5 | 2106 | 2751 | 2387 | 2745 | 2752 | 3532 | 2850 | 2166 |
| | | | | | | | | |

| **random set 2** | | | | | | | | |
|---|---|---|---|---|---|---|---|---|
| 8plex1 | 2045 | 2106 | 2212 | 2745 | 2044 | 2749 | 2752 | 2391 |
| 8plex2 | 2025 | 2831 | 2401 | 3540 | 2395 | 2484 | 2453 | 2961 |
| 8plex3 | 2194 | 2859 | 2746 | 2512 | 2267 | 2864 | 2861 | 2751 |
| 8plex4 | 2383 | 2166 | 2747 | 2387 | 3532 | 2741 | 2867 | 2852 |
| 8plex5 | 3534 | 2755 | 2850 | 2317 | 2191 | 3538 | 3511 | 2188 |
| | | | | | | | | |

| **random set 3** | | | | | | | | |
|---|---|---|---|---|---|---|---|---|
| 8plexl | 2484 | 2850 | 2741 | 2747 | 2755 | 2745 | 2025 | 2746 |
| 8plex2 | 2383 | 3534 | 2861 | 2751 | 2749 | 2391 | 2188 | 2191 |
| 8plex3 | 2194 | 3538 | 2512 | 2961 | 2864 | 2867 | 2831 | 3532 |
| 8plex4 | 3511 | 2045 | 2387 | 2212 | 2166 | 2267 | 3540 | 2401 |
| 8plex5 | 2395 | 2317 | 2859 | 2453 | 2852 | 2106 | 2752 | 2044 |

The sequences of all of those amplificates and the according primers are given in the sequence protocol (primers SEQ IDs 81-160; amplificates SEQ IDs 1-40). SEQ IDs refer to the internal numbers used in these tables as is shown in TABLES 1 and 2.

To show if the use of the design method described herein was superior to the common method of selecting primers for simultaneous amplification randomly said multiplex PCRs were performed. This example hereby demonstrates the advantage of the method which is subject of the invention:

A total of 40 amplificates (with lengths ranging from 187 - 499 bp) were partitioned into five 8-plex PCRs using either of two strategies.

First: the grouping was based on the invention using said "optimised sets" ("designed group").

Second: the grouping was done without using the selection criteria established by this invention using the "random sets" ("control group").

Whether such grouping can improve the success rate of mPCRs was subsequently tested experimentally by comparing the number of true and false positives and false negatives for each of the two classes.

Each of the five mPCRs (multiplex PCRs) contained 8 primer pairs specific for 8 amplificates with one primer of each pair being labeled with a Cy-5 fluorescent tag. Only fragments that performed successfully in sPCR (singleplex PCR) using bisulfite-modified human DNA from whole blood were included in this study. Isomolar primer concentrations were used in a 20µl PCR reaction volume and cycling was done for 42 cycles using a 96-well microtiter plate thermocycler.

Group assignments for the "optimized" and "random" groups were done in triplicate and all mPCRs were run at the same time such as to minimize experimental variation in PCR performance.

A mixture of the amplificates that were expected to be generated in a specific mPCR reaction but were generated in eight corresponding sPCR reactions was called sPCR-pool. Electrophoresis of sPCR-pool amplificates and mPCR amplificates was done simultaneously using the ALFexpress system (Amersham Pharmacia). In order to obtain the best comparability for mPCRs with their respective sPCR standard, these products were electrophoresed next to each other on the gels.

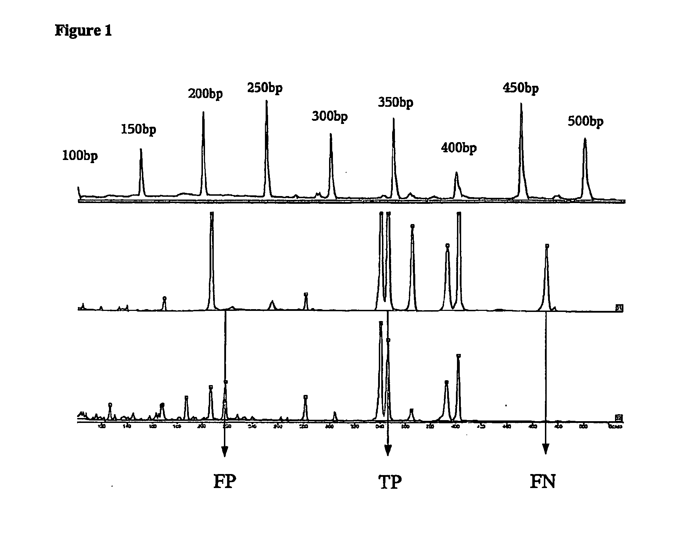

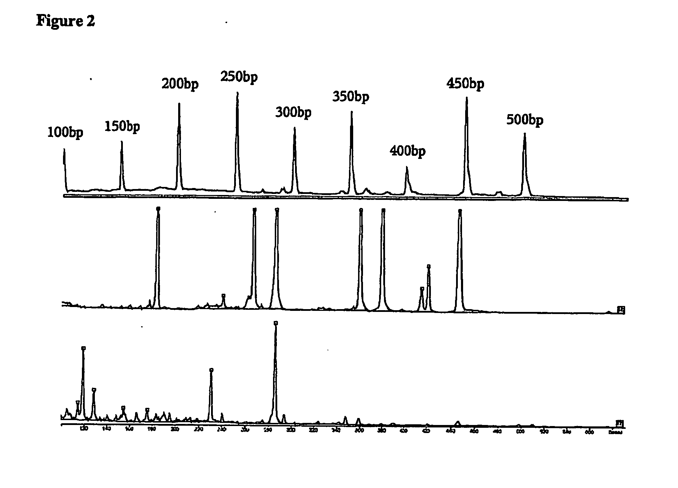

**Figures 1** **and** **2** show examples of these results as electropherograms, given as ALFexpress output files.

Success or failure scoring for each mPCR was based on assessing the number of generated or absent fragments compared to their respective pool of sPCR fragments. Only fragments with peak areas equal or larger than 8% of the largest peak within one electropherogram were included into the analysis.

Figure 1 illustrates a result of an 8-plex PCR based on a primer combination from the "optimized set". The top graph in the figure shows peaks of size standards only. The second graph in the figure shows the electrophoresed mixture of the products from 8 singleplex PCRs. The third graph shows the products resulting from a multiplex PCR employing one of the optimized sets of primer combinations. By comparing these graphs it becomes visible that, in this specific example, there is only one false negative (FN) and three false positives (FP), whereas there are eight true positives (TP).

Figure 2, however, illustrates a result of an 8-plex PCR based on a primer combination from the "control set". The top graph in the figure shows peaks of size standards only. The second graph in the figure shows the electrophoresed mixture of the products from 8 singleplex PCRs. The third graph shows the products resulting from a multiplex PCR employing one of the randomly chosen sets, as is the state of the art. This graph clearly shows that, there are eight false negative and six false positive peaks, whereas there is only one true positive. Hence, for this specific example we have demonstrated the superiority of the design method.

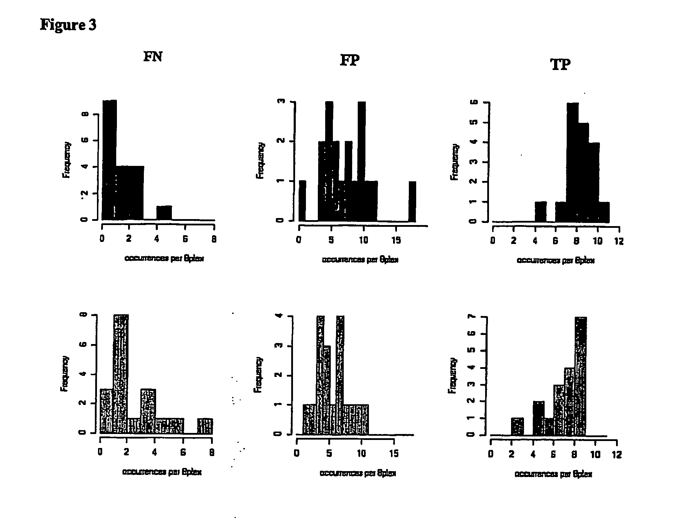

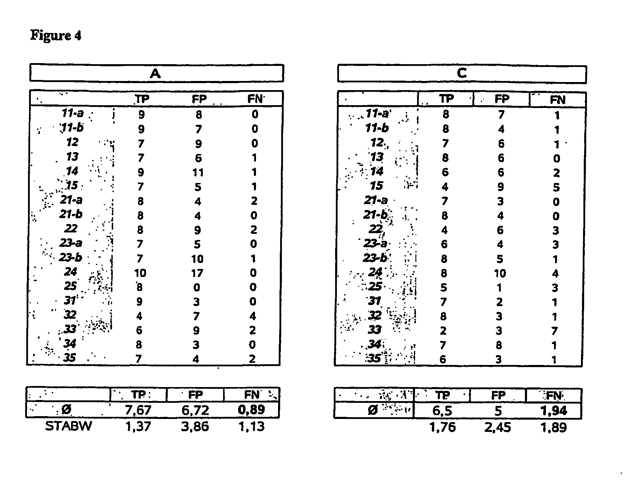

A more comprehensive view on the results is given in **Figures 3 and 4**.

By applying the Wilcoxon rank sum test for the determination of false positives or false negatives as follows, it becomes evident that the optimized set resulted in a more reliable amplification experiment:
data: False negatives (FN)
   p-value = 0.02602 rejection of null hypothesis null hypothesis (H0): true if median of designed set equal or greater than of control set alternative hypothesis (H1): true if median of designed set less than of control set
data: False positives (FP)
   p-value = 0.06711 rejection of null hypothesis null hypothesis (H0): true if median of designed set equal or less than of control set alternative hypothesis (H1): true if median of designed set greater than of control set
data: True positives (TP)
   p-value = 0.02146 rejection null hypothesis null hypothesis (H0): true if median of designed set equal or less than of control set alternative hypothesis (H1): true if median of designed set greater than of control set

Figure 3 illustrates a summary of several such comparisons (as described in detail above). Six diagrams are shown, that illustrate the numbers of false positives (FP), false negatives (FN) and true positives (TP) for a number of 18 experiments. In the top row of figure 3 the results for experiments that employed the design method are shown whereas in the lower row results from experiments are shown, that did use the conventional method of random selection.

At the x-axis the occurrence of an event (like a false positive) per 8plex is given whereas the values of the y-axis indicate the frequency of an event like this occurring within the number of experiments performed.

For example, in the diagram title FN, a y-value of 0 indicates that the event did not occur in a s ingle experiment, a y-value of four indicates that the according number of occurrences given as the x-value was found in four experiments (out of the 18 experiments considered for these analyses). The x-value indicates what kind of occurrence is counted; a x-value of three in this diagram indicates the occurrence of three false negatives. A data point with an x-value of 0 and an y-value of 9 means, that in the set of mPCR results considered, nine experiments showed 0 false negatives.

Figure 4 gives all of the data from the 18 multiplex PCR experiments of this example in one table. The letter A, heading the four columns presented on the left side, is indicating the results from multiplex PCRs of the designed group using the five optimized sets of primer pairs that have been designed and selected according to the invention. The letter C is indicating the results from multiplex PCRs of the control group using the five randomized sets of primer pairs.

The first column lists the identifying numbers of the experiments, the second column gives the numbers of true positives (TP) within this experiment, the third column gives the numbers of false positives (FP) and the last column gives the numbers of false negatives (FN).

The average false negative rate (∅ FN) of the optimized group is significantly lower than in the control group. Complementary the average true positive rate (∅ TP) is significantly higher. The average false positive rates (∅ FP) of the two sets do not differ from each other significantly.

This is due to the high deviation of false positives observed between individual ALFexpress analysis runs. Those 36 sets of amplificates have been analyzed on two separate gel runs These runs were not designed to simply duplicate the results, but could be used to analyze whether the average TP, FP and FN rates are similar, independent of the run, and the sets chosen. Only three of those sets have been duplicated, as indicated by the letters a and b for sets 11, 21 and 23. It turned out that the rate of true positives as well as the rate of false negatives averaged over 18 sets per run were highly reproducible, 6.83 versus 7.33 and 1,44 versus 1.39 respectively. However, the rate of false positives was determined as 4.11 in the first run and 7.61 in the second run.

Taken together, it could be concluded that the overall success rate of amplifying 40 fragments within 5 groups of 8plex PCRs was significantly increased when the primer grouping was based on the method being subject of this invention compared to an arbitrary primer grouping. The improved success rate of only 11% failures versus 24% in the random control group clearly becomes relevant when much larger numbers of mPCRs have to be established as is the case in a high throughput laboratory.

## Claims

1. A method for designing primer molecules to be used for amplification of nucleic acids which have been treated in a manner that differentiates between methylated and unmethylated cytosine bases, comprising the steps of
a) selecting a set of possible primer pairs per amplificate by means of a PCR primer design program using said treated nucleic acids as template;
b) excluding those amplifying primer pairs which comprise of a primer that in combination with another primer molecule in the same set exceeds a threshold melting temperature, when hybridising with said another primer molecule;
c) excluding those primer pairs which comprise of a primer that does not reach a specified minimum level of complexity;
d) virtually testing in silico all possible combinations of the resulting primers in the set for whether or not an unwanted product is amplified using the treated and the untreated sample nucleic acid as template, under conditions allowing for one or more base mismatches per primer until up to a number of mismatching nucleotides of 20% of the number of nucleotides of the primer molecule;
e) excluding those primer pairs which comprise of a primer that in combination with another primer molecule in the same set, under conditions allowing for one or up to 20% base mismatches per primer, amplifies an unwanted product when virtually tested in silica using the treated and the untreated nucleic acid as template.

2. A method for designing primer molecules according to claim 1, adding the step of
f) excluding from the remaining confirmed primer pairs those pairs which in said amplification step do not result in the amplification of the intended product when performing a single PCR experiment.

3. A method according to claims 1 to 2 wherein said measure of complexity is a measure of linguistic complexity.

4. A method according to claims 1 to 2 wherein said measure of complexity is a measure of Shannon entropy.

5. A method according to claims 1 to 4 wherein the following step is carried out prior to performing step d)
excluding from the remaining primer pairs those pairs, which consist of a primer molecule that comprises of at least one CpG site.

6. A method according to claims 1 to 5 wherein the following step is carried out prior to performing step d)
excluding from the remaining primer pairs those pairs, which comprise a primer molecule that does not contain at least one nucleotide within the last three nucleotides from the 3' end of the molecule wherein said nucleotide is complementary to a nucleotide of the target sequence that was converted to a different nucleotide by said treatment.

7. A method according to claims 1 to 6 wherein the following step is carried out prior to performing step d)
excluding from the remaining primer pairs those pairs, which comprise a primer molecule that contains more than 5 bases at its 3' end that are complementary to any other primer molecules' sequence in the set.

8. A method according to claim 1 to 7 wherein the following step is carried out prior to performing step d)
excluding from the remaining primer pairs those pairs, which amplify a nucleic acid that did not, prior to the treatment, contain at least two CpG sites

9. A method according to claims 1 to 8 wherein the treatment is done by means of a solution of a bisulfite, hydrogen sulfite or disulfite.

## Patentansprüche

1. Verfahren zum Designen von Primermolekülen, die zur Amplifizierung von Nukleinsäuren verwendet werden, die auf eine Art behandelt wurden, die zwischen methylierter und unmethylierter Cytosinbase unterscheidet, umfassend die Schritte:
a) Auswahl eines Sets möglicher Primerpaare pro Amplifikat mittels eines PCR Primerdesign-Programms, wobei die behandelten Nukleinsäuren als Vorlage verwendet werden;
b) Ausschließen der amplifizierenden Primerpaare, die einen Primer umfassen, der in Kombination mit einem anderen Primermolekül in demselben Set eine Schemelztemperaturschwelle überschreitet, wenn er mit dem anderen Primermolekül hybridisiert;
c) Ausschließen der Primerpaare, die einen Primer umfassen, der nicht den vorgegebenen Mindestgrad an Komplexität erreicht;
d) virtuelles Untersuchen in silico aller möglichen Kombinationen von resultierenden Primern des Sets darauf, ob ein ungewolltes Produkt amplifiziert wird oder nicht amplifiziert wird, wobei die behandelte und die unbehandelte Nukleinsäurenprobe als Vorlage verwendet wird, unter Bedingungen, die eine oder mehrere Basen-Fehlpaarungen je Primer bis zu einer Anzahl von Nukleotid-Fehlpaarungen von 20% der Anzahl der Nukleotiden des Primermoleküls erlauben;
e) Ausschließen der Primerpaare, die einen Primer umfassen, der in Kombination mit einem anderen Primermolekül in demselben Set, unter Bedingungen, die eine oder bis zu 20% Basen-Fehlpaarungen erlauben, ein ungewolltes Produkt amplifiziert, wenn er virtuell in silico untersucht wird, wobei die behandelte und die unbehandelte Nukleinsäurenprobe als Vorlage verwendet wird.

2. Verfahren zum Designen eines Primermoleküles nach Anspruch 1, mit dem zusätzlichen Schritt:
(f) Ausschließen von den bestätigten Primerpaaren jene Paare, die in dem Amplifizierungsschritt beim Ausführen eines einzelnen PCR-Experiments nicht in der Amplifizierung des angestrebten Produktes resultieren.

3. Verfahren nach Anspruch 1 bis 2, wobei es sich bei dem Maß der Komplexität um ein Maß linguistischer Komplexität handelt.

4. Verfahren nach Anspruch 1 bis 2, wobei es sich bei dem Maß der Komplexität um ein Maß der Shannon-Entropie handelt.

5. Verfahren nach Anspruch 1 bis 4, wobei der folgende Schritt vor dem Schritt d) ausgeführt wird
Ausschließen von den verbleibenden Primerpaaren jene Paare, die aus einem Primermolekül bestehen, der aus mindestens einem CpG-Ort besteht.

6. Verfahren nach Anspruch 1 bis 5, wobei der folgende Schritt vor dem Schritt d) ausgeführt wird
Ausschließen von den verbleibenden Primerpaaren jene Paare, die ein Primermolekül umfassen, das nicht mindestenes ein Nukleotid innerhalb der drei letzten Nukleotide von dem 3'-Ende der Moleküle enthält, wobei das Nukleotid einem Nukleotid der Zielsequenz komplementär ist, das durch die Behandlung in ein anderes Nukleotid umgewandelt wurde.

7. Verfahren nach Anspruch 1 bis 6, wobei der folgende Schritt vor dem Schritt d) ausgeführt wird
Ausschließen von den verbleibenden Primerpaaren jene Paare, die ein Primermolekül umfassen, das mehr als fünf Basen an seinem 3'-Ende enthält, die jeder anderen Sequenz von Primermolekülen in dem Set komplementär sind.

8. Verfahren nach Anspruch 1 bis 7, wobei der folgende Schritt vor dem Schritt d) ausgeführt wird
Ausschließen von den verbleibenden Primerpaaren jene Paare, die eine Nukleinsäure amplifizieren, die vor der Behandlung nicht mindestens zwei CpG-Orte enthielt.

9. Verfahren nach Anspruch 1 bis 8, wobei die Behandlung mittels einer Lösung aus einem Bisulfit, Hydrogensulfit bzw. Disulfit erfolgt.

## Revendications

1. Méthode de conception de molécules d'amorces à utiliser pour l'amplification d'acides nucléiques qui ont été traités d'une manière qui fait la distinction entre les bases de cytosine méthylées et non méthylées, comprenant les étapes suivante :
a) sélection d'un jeu de paires d'amorces possibles par amplificateur au moyen d'un programme de conception d'amorce par PCR en utilisant lesdits acides nucléiques comme modèle ;
b) exclusion des paires d'amorces amplificatrices qui comprennent une amorce qui, en combinaison avec une autre molécule d'amorce du même jeu, dépasse un seuil de température de fusion lors de l'hybridation avec ladite autre molécule d'amorce ;
c) exclusion de paires d'amorces qui comprennent une amorce qui n'atteint pas un niveau minimal de complexité spécifié ;
d) test virtuel in silico de toutes les combinaisons possibles d'amorces résultantes du jeu afin de détecter si un produit indésirable est amplifié ou non en utilisant l'acide nucléique traité et non traité comme modèle, ce dans des conditions permettant un ou plusieurs mésappariements de bases par amorce jusqu'à un nombre de nucléotides mésappariés égal à 20 % du nombre de nucléotides de la molécule d'amorce ;
e) exclusion de paires d'amorces qui comprennent une amorce qui, en combinaison avec une autre molécule d'amorce du même jeu, dans des conditions qui permettent un ou jusqu'à 20 % de mésappariements de bases par amorce, amplifie un produit indésirable lors d'un test virtuel in silico utilisant l'acide nucléique traité et l'acide nucléique non traité comme modèle.

2. Méthode de conception de molécules d'amorces selon la revendication 1, ajoutant l'étape consistant à :
f) exclure des paires d'amorces confirmées restantes les paires qui, à ladite étape d'amplification, n'entraînent pas l'amplification du produit envisagé lors de la réalisation d'une expérience PCR unique.

3. Méthode selon les revendications 1 à 2, dans laquelle ladite mesure de complexité est une mesure de complexité linguistique.

4. Méthode selon les revendications 1 à 2, dans laquelle ladite mesure de complexité est une mesure d'entropie de Shannon.

5. Méthode selon les revendications 1 à 4, dans laquelle l'étape suivante est réalisée avant de réaliser l'étape d)
exclusion des paires d'amorces restantes des paires qui consistent en une molécule d'amorce comprenant au moins un site CpG.

6. Méthode selon les revendications 1 à 5, dans laquelle l'étape suivante est réalisée avant de réaliser l'étape d)
exclusion des paires d'amorces restantes des paires qui comprennent une molécule d'amorce qui ne contient pas au moins un nucléotide parmi les trois derniers nucléotides de l'extrémité 3' de la molécule, dans laquelle ledit nucléotide est complémentaire à un nucléotide de la séquence cible qui avait été convertie en un nucléotide différent par ledit traitement.

7. Méthode selon les revendications 1 à 6, dans laquelle l'étape suivante est réalisée avant de réaliser l'étape d)
exclusion des paires d'amorces restantes des paires qui comprennent une molécule d'amorce qui contient plus de 5 bases à son extrémité 3' qui sont complémentaires à n'importe quelle autre séquence de molécules d'amorces du jeu.

8. Méthode selon les revendications 1 à 7, dans laquelle l'étape suivante est réalisée avant de réaliser l'étape d)
exclusion des paires d'amorces restantes des paires qui amplifient un acide nucléique qui ne contenait pas avant le traitement au moins deux sites CpG.

9. Méthode selon les revendications 1 à 8, dans laquelle le traitement est effectué au moyen d'une solution d'un bisulfite, sulfite d'hydrogène ou disulfite.


### Sequence listing

<110> Epigenomics AG
<120> Method for amplification of nucleic acids of low complexity
<160> 160
<210> 1
   <211> 322
   <212> DNA
   <213> Artificial Sequence
<220>
   <223> 2025
<400> 1
   aatcctccaa attctaaaaa cataaaaata acgcaaccca aaaacaaaaa acccctccgc 60
   ccattaatta ctatacacta acgaaacttt cccgacccac aacgacgaaa ataaaaacaa 120
   tcgctaacgc taaaaaacat caaaaacact acccaaccca aatatcgccg ccgcttccac 180
   aaaactctac taaacgccgc cgccgccgct accaccgcct ctaatccaaa ccacctcccg 240
   ccaaataaac cccgaaatcc taactcaaat atatatctct ccctccctct ccctccattc 300
   gtcattttct cactcccttt cc 322
<210> 2
   <211> 413
   <212> DNA
   <213> Artificial Sequence
<220>
   <223> 2044
<400> 2
   ggataggagt tgggattaag attttcggtt agtttcgtat tttttcgtat tttttagtat 60
   cgtttcgtat ttttcgtatt ttttttcggg ttattacgtt ttttatgtga ttcgtttggg 120
   taacgtcgaa tttagtcgcg tagcgttgta gtgaattttt tttttaaatt gtaataagtc 180
   gttttttaag gtaattacgt tttttttgtt ttttttttaa aaaataaaaa taaaaaattt 240
   atagaaaaaa attcgcgagt ttagaaaaaa gaagtaattg gtagaaggtt ttaattaagg 300
   taaagagttg taaggcgaag ttaagaaaat gtaggtattt aaaaaatgta ggtaattttt 360
   ataagggttt ttggggagag gtatatagag ggattttggt gttgaaaaag att 413
<210> 3
   <211> 347
   <212> DNA
   <213> Artificial Sequence
<220>
   <223> 2045
<400> 3
   aaccctttct tcaaattaca aaccttctta ccttcaaacc tcgactccaa caccaatccg       60
   acaaaaaaac ccaatctaat aaaatacgct cccttcctac cattctctat tccattaacc     120
   tatttcgtaa taaacgtaaa actaatcctc caaaattacc ttattaatta acttacatat     180
   ttattatcta tctatcccac caaaatacaa atttccgaaa aacaaaaatt taaaaaaatc     240
   tattttattc tatataattt tcccatacca aacaccgtac ccgacacaaa ctaaaatccc     300
   aatacacatc tcgaaacgaa aaaaccgtat ttccctaaaa cccaatc                   347
<210> 4
   <211> 283
   <212> DNA
   <213> Artificial Sequence
<220>
   <223> 2106
<400> 4
   ttgaaaataa gaaaggttga ggtagagagg ataatatagt tttagtttat tttttagtat      60
   tttgttaatt ttttttaatt tttagttata aattcgagat ataacgtttt ttttttaaag     120
   aggtcgcgtt ttttttgtgg tggtttttag ggattcgttt tagttttttt ttcgttttta     180
   gttttatata ttgggattat taggtattta agattttatt ttttaggtgg tatttttagc     240
   gtaggttgtt atttagtttt tttttaggga tttggggtag aag                       283
<210> 5
   <211> 211
   <212> DNA
   <213> Artificial Sequence
<220>
   <223> 2166
<400> 5
   tgtttgggat tgggtagggt tatcggggtt gggggggcgg ggtttgtggg taaggcgggc       60
   ggaggcgtgg atttttcgtt cgatgatagg gttggaggag gaaggggcgg gttgaagaag     120
   gggaaggtgg gaagagttta gtcggggtta taaattgggt gaagcgttga ggttttagta     180
   ttttcgtttg aggagatagg taaaggttat g                                    211
<210> 6
   <211> 497
   <212> DNA
   <213> Artificial Sequence
<220>
   <223> 2188
<400> 6
   ttttagattg aggttttagg gttaaaggat tatttttttt tttagcgttg gttcgggaaa      60
   ggtaagtttc gggcgggagc gtacgtcgcg ttttcgaagt ttggtttttt cgttacgttt     120
   attttttgtt tttatttcgc gtttttttag gttttttttc ggtgaatcgg atgttttgtt      180
   agttttttat tttgcgtttt cggtcgcggt tcgggttttt cgtaaagtcg ttgttatttc     240
   ggagggttta gttagcgggt tttcggaggt tggtcgggta ggcgtggtgc gcggtaggag    300
   ttgggcgcgt acggttatcg cgcgtggagg agatattgtt ttgtcgcgat gggggttcgg    360
   ggcgtttttt tacgtcgtag gtaagcgggg cggcggttgc ggtatttgtt tatcgggagt    420
   tttttttttt tttttttgtt gttgttgttt tgtatttagt tcgggggagg atagaagaaa    480
   aaggaggtag aatggat                                                   497
<210> 7
   <211> 373
   <212> DNA
   <213> Artificial Sequence
<220>
   <223> 2191
<400> 7
   ggaggggaga gggttatgcg attttatttt tggttagggt cggggaggtt tttgtttttc     60
   gggagttttg ttcgggtttt ttggtcgtag ggttgttggg ttttaggtag gaacgagagg    120
   gtgaggttta tatgtggttc ggcggtttag ggcggtttgt agcgttttta ttgtttcggt    180
   tgttaggggt tgcggcgacg cggttagtta gtagcgagtt taggtcgcgt agattttatt    240
   gatgagtttt gatttttagt atttttttta agttaagaag agtttagcgt atttttcggt    300
   tgttttattt tagttttttt gttttagttt tttagtttta ttttttttcg ttttgttttg   360
   gggtgtgtat agt                                                       373
<210> 8
   <211> 368
   <212> DNA
   <213> Artificial Sequence
<220>
   <223> 2194
<400> 8
   ttttgggaat gggttgtatc gagaggttcg attagtttta gggttttagt gagggggtag      60
   tggaatttag cgagggattg agagttttat agtatgtacg agtttgatgt tagagaaaaa     120
   gtcgggagat aaaggagtcg cgtgttatta aattgtcgtc gtagtcgtag ttatttaagt     180
   gtcggatttg tgagtatttt gcgtttttag ttttcggata gaagttggag aatttttttg     240
   gagaattttt cgagttagga gacgagattt tttaataatt attatttttt tttgcgtttt     300
   ttatttgtcg ttcgttggga taaacgatag ttatagtttt tttgacgata ggatggaggt     360
   taagggta                                                              368
<210> 9
   <211> 352
   <212> DNA
   <213> Artificial Sequence
<220>
   <223> 2212
<400> 9
   ttgttgggag tttttaagtt ttgtgagaat tttgggagtt ggtgatgtta gattagttgg      60
   gttatttgaa ggttagtagt tcgggtaggg tttatcgaaa gtttattcgt atatattagg     120
   taatttaatt ttttattttg tgtgatagaa gtagtaggaa gtgagttgtt tagaggtagg     180
   agggtttatt ttttgttaaa ggggggatta gaattttttt atgcgagttg tttgaggatt     240
   gggatgtcga gaacgcgagc gattcgagta gggtttgttt gggtatcgtc ggggtaggat     300
   tcggaacgta ttcggaaggt tttttgtaag tatttatttg gaaggagaat tt             352
<210> 10
   <211> 295
   <212> DNA
   <213> Artificial Sequence
<220>
   <223> 2267
<400> 10
   gtaatttgaa gaaagttgag gggaggcggt agatgttttg atttattagg gaaaacgtgg 60
   acgttttttg ttgttatttt gtgaattgtg tgtatttagt tatttttgag taaatatttg 120
   gagcgaggaa tttttgagtg gtgtgggagg gcggtgaggg gtagttgaaa gtcggttaaa 180
   gttttcggag gggttggttt aggaaatatg attggtagtt acgagagagt taggggttgg 240
   acgtcgagga gagggagaag gttttcgggc ggagagaggt tttgtttagt tgttg 295
<210> 11
   <211> 278
   <212> DNA
   <213> Artificial Sequence
<220>
   <223> 2317
<400> 11
   ggagttgtat tgttgggaga tttgggtgta gatgatgggg atgttaggat tattcgaatt 60
   taaagttgaa cgtttaggta gaggagtgga gttttgggga attttgagtc ggtttaaagc 120
   gtattttttt gtatatttat tcggtgttgg gcgtagggaa tttttgaaat aaaagatgta 180
   taaagtattg aggtttgaga tttttggatt tcgaaatatt gagaatttat agttgtatat 240
   tttagagttt atggtatttt agtgaaaatt ggggtttt 278
<210> 12
   <211> 285
   <212> DNA
   <213> Artificial Sequence
<220>
   <223> 2383
<400> 12
   tttgtattag gttggaagtg gtcgttagtt tttcgtgtaa ttttattttt tggaaaagtg 60
   gaattagttg gtattgttta gcgtgatttg tgaggttgag ttttaatagt ttaaagaagt 120
   aaatgggatg ttattttcgc ggggttcgtt tttcgcgagg tgtttatttc gtatttgtta 180
   tgtaaaacga gggagcgtta ggaaggaatt cgttttgtaa agttattggt tttggttatt 240
   agtttttatt taatgttttc gtgatgttgt tgttgattta tttgg 285
<210> 13
   <211> 380
   <212> DNA
   <213> Artificial Sequence
<220>
   <223> 2387
<400> 13
   gattttggga gaggaagtta agtgtttttt tgtttttttt cggtatttta tttaaggcga      60
   ttagtttaga attggttttc ggaagcgttc gggtaaagat tgcgaagaag aaaagatatt    120
   tggcggaaat ttgtgcgttt ggggcggtgg aattcgggga ggagagggag ggattagata    180
   ggagagtggg gattattttt tttgttttta aattggggta gttttttggg ttttcgattt    240
   ttttattttc gtgggtaaaa aattttgttt ttatcgggtt tacgtaattt ttttaagggg    300
   agaggaggga aaaatttgtg gggggtacga aaaggcggaa agaaatagtt atttcgttat    360
   atgggtttgg tttttagttt                                                  380
<210> 14
   <211> 397
   <212> DNA
   <213> Artificial Sequence
<220>
   <223> 2391
<400> 14
   tggggttagt ttaggatagg cgttcggggg acgcgtgttt ttattttacg gggacggtgg      60
   aggagagtta gcgagggttc gaggggtagg tattttaacg aatggttttt ttggtgtttt    120
   ttgcgtttcg tcggtttatt ttttttttta taaaacgggt ttagttttta gtatttattt    180
   ttcgttatta attaggtatt tcgggagatt agttcgttcg aaagtttttg cgttatttcg    240
   cgggtttttt taggtggttt ttttagtttc gttttttttc gggatgtttg ttgattattt    300
   cgagttcgcg tggcgtaaga gtacgagcgt cgagttcgtg cgcgttaagg ttgcgtgggc    360
   gggtatcgat ttttttgaga agttttagtg tttttaa                              397
<210> 15
   <211> 547
   <212> DNA
   <213> Artificial Sequence
<220>
   <223> 2395
<400> 15
   tttttgtatt ggggtaggtt tcggtaggtg tatgggagga agtacggaga atttataagt 60
   ttttcgattt tttagtttag acgttgttgg gtttttttcg ttggagatcg cgtttttttt 120
   aaatttttgt gagcgttgcg gaagtacgcg gggttcgggt cgttgagcgt tgtaagatag 180
   gggagggagt cgggcgggag agggaggggc ggcgtcgggg cgggttttga tatagagtag 240
   gcgtcgcggg tcgtagtata gtcggagatc gtagttcgga gttcgggtta gggtttattt 300
   gttttcgtag cgtcggttcg cgtttttttg tcgtagttat cggtgagtgt cgcggttttg 360
   agattttcgg gtcggatgcg cggcggtttt agttttcgag cgtttgtttg tttcgttttg 420
   ggttgttcgg gttttttggg tttttcggcg gttgtacgga gttaaggcgt ttcgtttcgg 480
   gcgtttttcg cgggtgtcga tttaggttgt tcggagttcg gagtttatag aggagagaga 540
   tagttgg 547
<210> 16
   <211> 414
   <212> DNA
   <213> Artificial Sequence
<220>
   <223> 2401
<400> 16
   attagaagtg aaagtaatgg aatttcgatg taaatataat attatttttt tgtagagtta 60
   ttttgagtat aataaatttg aattgtgtta atgttgggag aaaaaattta aaagaagaac 120
   ggagcgaata gtagtttttt cgttcgttga ttagaaatag taggacgata ttttttcgat 180
   tggaggagag cgtttgcgtt cgtatttagt tggcgttcgt ttttttgttt tttttttagt 240
   cgtttttttt tttttttttc gcgttttagt tattcgggaa ggtattgcgg tagttgggtt 300
   ttgattggtt gttttgaaag tttacgggtt attcgattgg tgaattcggg gttttttagc 360
   gcggtgagtt tgaaattgtt cgtatttggt tttaaagttg gtttttggaa attg 414
<210> 17
   <211> 272
   <212> DNA
   <213> Artificial Sequence
<220>
   <223> 2453
<400> 17
   gggatgggtt attagttgta aatcgtggaa ttttttttga tataatgaaa agatgagggt    60
   gtataagttt tttagtaggg tgatgatata aaaagttatc ggagtatttt ataaggtata   120
   aatttttaga gatagtagag tatataagtt tttaggataa gagttaggaa gaaattatcg   180
   gaaggaatta ttttattgtg tgtaaatatg atttttaagt tggtcgtggt ttttttggta   240
   gtttttttga tttttgtagt tttgtgtgaa gg                                 272
<210> 18
   <211> 391
   <212> DNA
   <213> Artificial Sequence
<220>
   <223> 2484
<400> 18
   taattgaagg ggttaatagt ggaatttggt tgggtgtttg ttaaattttt ttttttggtt    60
   ttgttttggg tttttttttg aagggatttt ttttcgtttt tgtaataaga ttttttataa   120
   agtatagatt ttttatttta tttcgcggta tttgtatcgg gttttattgg ttttaggagt   180
   tgaatatttt tttaggtata tataggtggg atataaataa gggttttgga attattattt   240
   ttttattacg atagtaattt aaaatgtttg ggaagatggt cgtgattttt ggagttttaa   300
   atatattttg gataatgttt gtagtttgta agttattttt ttttatttgt tttaaatgtt   360
   agtatttaat tttagttttg gttttggttt t                                  391
<210> 19
   <211> 430
   <212> DNA
   <213> Artificial Sequence
<220>
   <223> 2512
<400> 19
   agtggatttg gagtttagat gtaatataat gattgatatt ggtatagtat atttattttg    60
   tttttgtaaa taaaatggta tatgtgatgt ttttttttgt ttttttgtat ataaaataat   120
   atttgttttt atttattatg tatttatgtt tttattttgt atgttaggag ttaagtattt   180
   tgtatgtatt aatttatttt gtttttataa taatttttat atgtaggaat tattatagtt   240
   attttatgaa tgagtcgagg aaggtattga gacgttaagt aatttgttta aggttacgta   300
   gttagtaagt ggtagagtaa gaattattat ggttttataa gtttaggaaa aagtttgaaa   360
   gaattaaaat gttaatagcg gggattttaa ggaagtattg aagaggttat gggagaagtt   420
   tttattttgt                                                          430
<210> 20
   <211> 475
   <212> DNA
   <213> Artificial Sequence
<220>
   <223> 2741
<400> 20
   taggggaaaa gttagagttg agaggttggg gcgcgacgag tttggatatc gggcggggat 60
   ttaagttttt ttcgtttagt taataattgt gtttttttta ggaaggcgtg aggaaatgtt 120
   ttaattaatt tttgtatttt ttttttggaa tttgggttgt atttttttat ttattgtaaa 180
   ttttataatt tatttagggg tttttttagt gtttgttttt agcggtttcg gtgtttattt 240
   attagtgttg tttttttttt ttcgtaagat tgcgttttag ttttagtttt ttttttcgcg 300
   ggtgtttttt aaatcgtttt attattttcg ggtttaggga ggcggaatcg tgtttgtttt 360
   tcggtttttt taagaggcgt cggttttatt ttttttagag tcgcggtttg acgcgagatg 420
   atagtaacga gttcggtatg tttatgtaaa taagcgtttt tttgtgggtt aatgg 475
<210> 21
   <211> 412
   <212> DNA
   <213> Artificial Sequence
<220>
   <223> 2745
<400> 21
   attttagttt gtgaaatggg atttaggatt taggtagagg tgcgttttcg gtttggggat 60
   cgagtatttt gtgcgtttcg gtaacgtagg aagatagcgt tattgatatt ttagagatta 120
   gcgggtatcg tttggaggcg tttttattat ttggcggttt cgggttcgcg ttttatcgcg 180
   ttataagatt tacgttcgaa ttacgtgatt agggtcgtgg tttcgtttcg ttttcgcgtc 240
   gcgcgtcgtt ttcggtaggg gcggaaagcg gaagtgtggg agggtttgcg gggcgggttt 300
   aggaggttcg cgggaggatg gagtagtgag cgggtttggg cggttgttgg tagcgttatg 360
   gagacggtat agttgaggaa ttcgtcgcgt cggtgagggg ttattggtta ag 412
<210> 22
   <211> 484
   <212> DNA
   <213> Artificial Sequence
<220>
   <223> 2746
<400> 22
   gtgggttttg ggtagttata gaagttatcg cgttggcggg gaggaggggg atcgatgcgg      60
   tttatgtttc gggtagtttt attttttttg tttgcgaagg gtttttgttc ggcgggagga     120
   gagaggcgcg ttttattcgg gtttttttat atttgtcgtc gtttgggtcg atttcgcggg     180
   tttcgttcgg cgttttagtc gattttcgtt tagtttcggg tttatgggcg cggttagtag     240
   ggcgggttag ggcggcgggg cgcgatattg ggaggaagtg cgggtcgttt gttcgggcgc     300
   gttaaggaag ttgtttaaaa tgaggaagag tcgcgggttc ggcggttgag gttatttcgg     360
   cggcggttgg agagcgagga ggagcgggtg gtttcgcgtt gcgttcgttt tcgttttatt     420
   tggcgtaggt aggtgtggtc gcgtttttta ttcggtcggg attttttggt aaggagagga     480
   ggtt                                                                  484
<210> 23
   <211> 476
   <212> DNA
   <213> Artificial Sequence
<220>
   <223> 2747
<400> 23
   taggatgggg agagtaatgt tttcgagtag aatagggtgg ggtttttaga ttattttttt      60
   ttttttatag ttggttttat tttatcgatt ttattaaagt ttttttggga gtattttaga     120
   gaagagttac gtttaggtcg ggttttggtt gtttggttta cggcggaatt tttagtatta     180
   cgtttcgtac gtcgggttta aagtatgttt agtgaaggag taggtattta ttgttagatg     240
   gagttatttt tttagatttg gggttttttt ataacgatgg ttatgtttgg tatggaagtt     300
   tttttagaag ttaatagtag gaaataaggg ttaatagtat ttaattgtgg agtaaggttt     360
   aaattttagt tttgttattt aatcgtttcg aatttgtttt tttattgtag aggcgaaaag     420
   gttaatatta ttttatttcg gagggttatc gtggagaatg gaagttggat aagttg         476
<210> 24
   <211> 419
   <212> DNA
   <213> Artificial Sequence
<220>
   <223> 2749
<400> 24
   tcccacaaaa actaaacaat tattacaaat tcaaaaaacc ccgaccaatt tttcaaaaat 60
   ttctcctcct cttttccccc taaaactcgt aatactttta ctctactttc aaaatacatt 120
   aaatctccta ctttataact actttaaaac caacaaatac tctaatatat ataattcaaa 180
   ttatacaaat ttcacgaata aatttaatct tattttttaa attaattaaa aaacaaataa 240
   tatttaaaaa aatattaact tataattatt tcaccctttt tactttaaac atttttatta 300
   cttctcgacc ttttaactaa aatcaaatat atactttaaa cattttttaa aataaaaata 360
   tccttttaat ttaataaaaa aacaaaattc tacataaaaa aaccccttca tctaaaacc 419
<210> 25
   <211> 479
   <212> DNA
   <213> Artificial Sequence
<220>
   <223> 2751
<400> 25
   tttggagggt ttagtagaag ttattttagg ggagggttcg ataggaagga aggtaggttt 60
   gtcggagggg tatataggag tttttttttt cgttatagtg tttagggtta attgttttag 120
   tttttaggtt gggttaatag gatgggatag tttaggcgga aggaaatttg tggggaggga 180
   tatttcgtag atagaagtag ggatatgggg tggggagagg taggaagagt tgtcgggttg 240
   ttgagttggc gtttttttag tagatttagg aggggcggtg ataggaggtt attttttttt 300
   tattttcgta gttttgggtt tttttggttt tggttaatag tattattatt attattattg 360
   ttgttgttcg ttagtttggg ttttagatat attagaaaaa aattatcgga agatacgtat 420
   agtattggta gtttttaaaa gaattaattt tttttttgtg tttattttgt gattattgg 479
<210> 26
   <211> 484
   <212> DNA
   <213> Artificial Sequence
<220>
   <223> 2752
<400> 26
   atacaacctc aaatcctatc caaaccccca aaacatcaca ctcgaaactt attctacata 60
   tttttacttt tacctcccac taatactaat tcttccgtaa aacaacctaa atcccttcaa 120
   atacttaata ttttttctca aatactacca taaaaccaaa tctccaccgt cttaaaacat 180
   tcctttttaa aaataaaaaa tatatatcgc tccttttata taatttacat tctatcttaa 240
   ataatttaac catcaccgta attcattcaa atctatttaa atcctaccca tctcaacttc 300
   aatccatttc attcttttaa atctaatcga caattacctc caacaacttc atcacaaatc 360
   actcacaaaa ataaccttaa tcctaaaatt tatttacgaa aaacacactt actaaatata 420
   taacaaatat acaaaaaaca caaaataaaa caacaaatct aaaaacaaat aacttccttc 480
   tccc 484
<210> 27
   <211> 371
   <212> DNA
   <213> Artificial Sequence
<220>
   <223> 2755
<400> 27
   ggaagatgag gaagttgatt agatattaag gatgagcgga tgatttaata ggtttttttg 60
   ttaagatttg gttgggtagg tgaaagataa agtcgaggag tggttatggt gtggtataga 120
   agaagggtta gaggacggtt tttgttattt ttttatgttt gagttttttt ttttgtgaaa 180
   tggggataat aagagtcgtt atatagggaa ttgttgttag gattaaatga gataatgtat 240
   gtgaaacgtt ttggttgtag gttttttagt aaatgggtac gatttgcgga gtggggattt 300
   gaatttacgt ttggcgggat gtttaagttg ttattttgat cgttagggag ttttagagga 360
   tagggttgta g 371
<210> 28
   <211> 186
   <212> DNA
   <213> Artificial Sequence
<220>
   <223> 2831
<400> 28
   ttagtagggg tgtgagtgtt ttgattagaa ttattttttt ttgttagaat ttgatgtaat 60
   tcgaatgttt ttatttttgt ttgaagggtt taaataataa attaggtttt gtcgtgttat 120
   tatgggggtg gttatatttt gtatttagga aataggtacg gtagggttga gatagaagtt 180
   ttgttt 186
<210> 29
   <211> 300
   <212> DNA
   <213> Artificial Sequence
<220>
   <223> 2850
<400> 29
   ttatagggtt gagtttggga tcgaggtgag agtcgtcggg ttgggagtga gggagatggg 60
   aataaggtcg tcggtgggcg aggggagtcg agggaattcg ggggattggg aggtttgggg 120
   cggcgcggtt tggtcgggtt gggatcggtt tttcggttta gacgttcgcg atgttggtat 180
   tttttgttat tttttatttg ggttttaggg gttcgttttt gggtagtttg gagtttttcg 240
   aggtgggagg atcgggcgga ggtggaggaa gttttttttt ggaagatttg ttgtttgttt 300
<210> 30
   <211> 321
   <212> DNA
   <213> Artificial Sequence
<220>
   <223> 2852
<400> 30
   tgaaaatgaa ggtatggagt ttggtgttaa aagaaatttt ttttaaaaat taaataataa 60
   tattagagta aagtttttag ggcgagataa ggagttgtaa taaaataagc ggaaattcga 120
   gaagcgttaa tgttttaaag ggttaatgat tatatataat ttacgtagtt aacgtgttaa 180
   aatatattaa cgtatttttt ttttttaaat aaagtaggaa agcggatttt gtatgagggg 240
   cgggttgtcg atttagtagt ttttttcgga tagttcgttt tgattttttt tggttggtcg 300
   tggagggatt atatggtttt a 321
<210> 31
   <211> 398
   <212> DNA
   <213> Artificial Sequence
<220>
   <223> 2859
<400> 31
   tatgtttggt tttgttttga gatagagttt cgttttgtcg tttaggttgg ttaaaagata 60
   gggttttagt cgggtgcggt ggtttacgtt tgtaatttta gtattttggg aggtcgaggc 120
   gggcggatta tttgaggttc ggagttcgag attagtttgg gttaatatgg cgaaacgttg 180
   tttttattaa aaataataaa aattatttag gcgtggtggc gcgtatttgt aattttagtt 240
   attcgggagg ttgaggtagg agaattattt gaatttagga ggtagacgtt gtagtgagtc 300
   gagatcgcgt tattgtattt tagtttgggc gatagaggga gatttcgttt taaaaaaagg 360
   aaaaaaaaaa aaaagaaaag aaataaaagt gatggggt 398
<210> 32
   <211> 347
   <212> DNA
   <213> Artificial Sequence
<220>
   <223> 2861
<400> 32
   gggtgtagaa gtgtttaggt tttttttcgt tggggttggg agtttgggta ggttagtttt 60
   atttttttta agttcgtttt tggttttcgg gtttagtttc ggttattatg tttcgttaga 120
   ttatttttgt gggttttagt tgtttggatt tgtggaggga aaagaatgat cggttcgttc 180
   gataggttaa ggtaatacgg ttgttggtat tttcggtttg tagttttaag atttttgaaa 240
   gcgggtttgt agtggattta ttttaataga tggggaggga ttgagtttga ttaaagagtt 300
   agaaatgatt ggagaatgta ttttttgtta ttgttgtaag gggagaa 347
<210> 33
   <211> 291
   <212> DNA
   <213> Artificial Sequence
<220>
   <223> 2864
<400> 33
   tccccttcca actatatctc tcacccaaaa ataacttcta actctcgtat tcatctaaaa      60
   ctcctccttc catataccaa caattaacta taacccctcc aaaaacgctc catctccaaa 120
   tatactccca catccaaacc acgaacccct cacccgatca catacttcat acacctataa 180
   ctccgcactc cccaaatata cctctaacgt acaactatta ccccttcccc cgattataac 240
   cctataactc gccacataca actataacta aaacttccct aaaacactct c 291
<210> 34
   <211> 389
   <212> DNA
   <213> Artificial Sequence
<220>
   <223> 2867
<400> 34
   aaaaccaaaa cataaaccaa aaaccaaact cgaaccgaaa acaataaccg caacgcccga 60
   aaactaaacc cacgacgcgc taacaacgcg aaccgaacta cgaaaacgat cacgtcaacg 120
   tccgttccaa accgactaac aatctccgtt ctacattaac gtcaacactc ccgttaaaaa 180
   taatacatct ctcccatacc aaaaaaactt aaatactact aaaaaccaac cctccgaata 240
   ctaccaaacc gacgctcacc cgccaccttc atcttccctt ctcctttacc ccaaaacaac 300
   cgaaaatata taattaaatt ccccctaccc ataaaaaaac caaaaataaa aaactaacga 360
   cctactcgat ctcaacaaac cctcctaat 389
<210> 35
   <211> 272
   <212> DNA
   <213> Artificial Sequence
<220>
   <223> 2961
<400> 35
   aatggttgat gattttggtt ttttttcgtc gtcggagagc ggtgtttcgg aggcggcgga 60
   ggaggattcg gcggtcgttt ttttggttta gtaggagagc gagattgtag gtatagagaa 120
   cgacgagggt ttcggggtat ttgtcggtag ttatgcggtt ttcgcgtagt cgggttttac 180
   gagtgggggt gagttagcgc ggggtttgga gaggggttta gggcgcgtat tcgggggatt 240
   tcggtcgggg tttaggggta tagggaagag ag 272
<210> 36
   <211> 371
   <212> DNA
   <213> Artificial Sequence
<220>
   <223> 3511
<400> 36
   agttagaaga ggagttagga tgggtttcgg gtagtttaat agtatagttg aagttttaat      60
   tattatgtta atagtttttt ggttttatat attttatggg aagaggaaaa taaaaaggta     120
   tttatttgta tattttttta tttttgatat aagaagtaga atttttttta tatgatttat     180
   gtttatttaa tacgttattt tgaaatttat taataaaatt ttttaagcgt tagaaaattg     240
   ttagtggttt tttttatttt tttttatttt tttttgtgtt attaattttg tttttttttt       300
   ttagaaggtt gtcggaatag taaatattta ttgatatgtt ataattattg gaaaatgggt     360
   attggaaaat t                                                          371
<210> 37
   <211> 457
   <212> DNA
   <213> Artificial Sequence
<220>
   <223> 3532
<400> 37
   tgttagtaga gttttaggga ggttttattt tttattttta tttaaagttt tatttgttgg      60
   ggtgggggtt ttgtttggaa ggggaaggtt taaggttgtt tttagcgtgt ttttttattt     120
   tgattgtttt tggcggggcg ggggtgtttt tgttatttag ttgtataacg gttaggaagg     180
   gtttaaatta tttttagggt taatttaagg tcgttttttg ggtttgtata tttttgtgtt    240
   gagtgcggat cgggagaggt tgttgaagat aggaggggat aaatggggga cgaaggggtt     300
   cgagggaggg gattgaagga tttgggttaa gtcgggagtt ttcgagggcg gagttaaaac     360
   gtatttggat tttgttagtt ttaaattttg tttttattgt tgtaagtttt ttagatcgag     420
   gattttcggg ttgagggtgg ggtaaggata ggtagtg                              457
<210> 38
   <211> 476
   <212> DNA
   <213> Artificial Sequence
<220>
   <223> 3534
<400> 38
   tttttgtttt tatggggtgt atatttaagt agttgaaata gatagtgaat aaataaaaaa      60
   ggataataat tttaaataat aatgatgtta tcggttaggt gtggtggttt atgtttataa     120
   ttttagtatt ttgggaagtt aagttaagcg gattatttga ggttaggagt ttaagaatag     180
   tttggttagt atggtgaaat tttattttta ttaaaaatat aaaaattagt tagatatggt     240
   ggtatatatt tgtaatttta gttatttggg aggttgacgt aggagaattg tttgagttcg     300
   ggaggtggag gttgtagtga gttaagattt gataggtttt tagtattatt gtattttaga     360
   ttggttgata gagcgagatt ttgttaaaaa aaaaaaagtt ataaatagat tttaataggg     420
   taatatgata gggagggagg gataggggag tagggtggtt aaggaaggga tattta         476
<210> 39
   <211> 458
   <212> DNA
   <213> Artificial Sequence
<220>
   <223> 3538
<400> 39
   tgggtagtat ttttgttggt ttttttttat attataaggt tacgtagagt tggcggaggg      60
   ttatggtttt atttatgtta ggtgttttta atttggtaag gaaatgtaat ttacgtgaat     120
   tttaataggt agtgaagtat cgtttttttt tgattttagg tagggtgaag aaaatgggat     180
   agtagtacgg ggtgcgggta taaacgtata attttgtttt tttagacgta gagttgtggg     240
   gttgtgagaa tgttaggagg aggtaagaaa gggcggtttt atggggggtt tgtagggtgg      300
   gataagttta agaggttttt atatttaggt ttggtggggg aggtgagttt ttggtttatc     360
   gagggggttt ttttttgttt tcggaaatat tgtagttttt atttttatcg ttttttcgtt     420
   gcggggattt aggggcgtga ggatgagaga gtttttag                             458
<210> 40
   <211> 405
   <212> DNA
   <213> Artificial Sequence
<220>
   <223> 3540
<400> 40
   agtggtttag gagtatttgg ttattttcgg gaaaaatcgg tttggtaaag gttttttcga 60
   gggtacgcgt ttttcggata gtgaggtagg atttaaattt tttcgttaat attatatttt 120
   tcgtattttt gtagtgtttg tatttttagg ttttattatt ttttcgtatt ttttagggag 180
   aagttttcga cgttttattt tttttggaag ggtgttgttt ttagagattt ttaggttaat 240
   ggtttaattt tagtgttttt aggggagagg ggggtgtaga aaaatagttt gggttataaa 300
   agaggtgcga gggttgtgag atttcggagg tatcgacggg aagcgagacg gagaatagga 360
   gggtaggacg ggttggaggt gggggatatt gtagatggag ggagt 405
<210> 41
   <211> 2501
   <212> DNA
   <213> Homo Sapiens
<400> 41
   ccagttccag tcccgggtcc tgtggccgcc ctgccggcga ccctgcggag agcgagtctt 60
   agatacccag tccccagccc cgagttgtta ttccctcgct gtagttaaga aggaggagat 120
   caattaaggg catcttagaa gttaggcgtt cccgctgcct cctttgagca cggaggccac 180
   caacccccta gggggaagag atgtagcgcg aggcaggggt gtcgtgctaa gaaatttcga 240
   cgcttctggg gactgaggac aaaggtgcgg acacgacccc ggggtacctg gagttccgtg 300
   actcgcgcca cggacggcac acctaggggc taatttctgc tctgcctcaa agaacctcaa 360
   gctagagtcc ttgcctccgc ccacagcccc gggatgccgc tgctgcgctc accgcacagg 420
   cagcgcccgg accggctgca gcagatcgcg cgctgcgcgt tccaccggga gatggtggag 480
   acgctgaaaa gcttctttct tgccactctg gacgctgtgg gcggcaagcg ccttagtccc 540
   tacctctgct gagctgaacg ctcaggcaca gtggaactga aacccggttc tgcgggatgt 600
   gagagctgtt gaggtcacgc gtaattgggt gtgatggagg gcgcctgttc gtgatgtgtg 660
   caggtttgat gcaagcaggt catcgtcgtg cgagtgtgtg gatgcgaccg cccgagagac 720
   tcggaggcag gcttgggaca cgtttgagtg aacacctcag gatactcttc tggccagtat 780
   ctgtttttta gtgtctgtga ttcagagtgg gcacatgttg ggagacagta atgggtttgg 840
   gtgtgtgtaa atgagtgtga ccggaagcga gtgtgagctt gatctaggca gggaccacac 900
   agcactgtca cacctgcctg ctctttagta gaggactgaa gtgcgggggt gggggtacgg 960
   ggccggaata gaatgtctct gggacatctt ggcaaacagc agccggaagc aaaggggcag 1020
   ctgtgcaaac ggctcaggca ggtgatggat ggcagggtag gaagggggag gtccagaggt 1080
   ctggatggag gcttccgcat ctgtaccttg caactcaccc ctcaggccca gcaggtcatc 1140
   ggccccctcc tcacacatgt aatggatctg aagagtaccc cgggacagtc cggggagatg 1200
   gagattcgga aagtatccat ggagatctta cagaatcccc tgtgcggacc aggaaactct 1260
   tgtagatccc tgcctatctg aggcccaggc gctgggctgt ttctcacaat attccttcaa 1320
   gatgagattg tggtccccat ttcaaagatg agtacactga gcctctgtga agttacttgc 1380
   ccatgatcac acaaccagga attgggccaa ctgtaattga actcctgtct aacaaagttc 1440
   ttgctcccag ctccgtctct tgtttcccac gagccctggc cctctgtggg taataccagc 1500
   tactggagtc agatttcttg ggcccagaac ccacccttag gggcattaac ctttaaaatc 1560
   tcacttgggc aggggtctgg gatcagagtt ggaagagtcc ctacaatcct ggaccctttc 1620
   cgccaaatcg tgaaaccagg ggtggagtgg ggcgagggtt caaaaccagg ccggactgag 1680
   aggtgaaatt caccatgacg tcaaactgcc ctcaaattcc cgctcacttt aagggcgtta 1740
   cttgttggtg cccccaccat cccccaccat ttccatcaat gacctcaatg caaatacaag 1800
   tgggacggtc ctgctggatc ctccaggttc tggaagcatg agggtgacgc aacccagggg 1860
   caaaggaccc ctccgcccat tggttgctgt gcactggcgg aactttcccg acccacagcg 1920
   gcgggaataa gagcagtcgc tggcgctggg aggcatcaga gacactgccc agcccaagtg 1980
   tcgccgccgc ttccacaggg ctctgctgga cgccgccgcc gccgctgcca ccgcctctga 2040
   tccaagccac ctcccgccag gtgagccccg agatcctggc tcaggtatat gtctctccct 2100
   ccctctccct ccattcgtca ttttctcact ccctttcctc ctctccctct ctctccgtta 2160
   gtctcttcat cagatagtct ctgttagtcc gcgatttata ccaggctcgt gccctaggtt 2220
   ggatcggaca gtctcaatcc cccggctcgc tcttcctgct cggctgcgga ctccagtctt 2280
   actctctcgc actgcacaca ggcttaggcc agtctcggga cactcaggct ccccagggac 2340
   cgcgcacaga gcctgaggca agagaaactt tccgcagacg gtgcgatcag ggacggcgtc 2400
   tggagcccag cagtcccagg gaaattggtt cagaacctgg aacagagcgg atgggtggca 2460
   aataggcacg acgactgagg gacaagcagc cctaaactgc a 2501
<210> 42
   <211> 2501
   <212> DNA
   <213> Homo Sapiens
<400> 42
   agatttactc aaatttaaga atgagaatac aaatccacat cttgaagtgt ttcacagaaa 60
   ggtctatctt aatgtctgga gtatatattt caatgaacat tcattttatt ttatttctct 120
   ccattcctga atcaagcaat cttgaatcta aagttgctat gattagcact gaaaagacca 180
   ctggactatt aattgtgtga ctttgggaca gtaactttct gcaccttagt ttgtttacat 240
   gttatacatg aaggttgaag tctgattctg ctctgtgact atcattctaa acatctgatg 300
   aaatcaaatt tcagtgtttg gaatggtagt acaataaatt tactaagaat aaataattca 360
   ctgcaaaaac acattgattt ccaaatgatg taactgacag ttatattact gcagagggct 420
   gataaataac aaaagaaatg aaagatgcac atggtgagaa ctgaaattat cctgacaagt 480
   cttctacctg tttatcactt aaaatcaatg accatgctga atgcctacaa attacaaaat 540
   ataaaagaaa tcttataaat gcgcatgtac aggagtctaa gttactaaaa gttttaaagc 600
   ataagtttaa accaaactaa tcaaagaagt tgagaggaaa aattggcttt catctttaat 660
   cactactgtt ttgaggtcct atgtttaata taattttcta agtagaggct tcagagagaa 720
   gagttgtgag gatactttca tatttgtgta gaaggaaaag tttgccatcc attctagtat 780
   ccctagtgtt atactgatgt gcaccttgga tttattttgt tcctattgta taaactcata 840
   cttgacttca aagaaaagga aaatccaaag tccctctttt ctaaggggac agaaatcctt 900
   tgtgtcaact gtttgaccct tttctctgta aggtcctatt ggaaatcttt tgtaacacaa 960
   tgcaggggac tcttccatgt gttgatgctg tttacacagt ggggtgggcc tgactgaaga 1020
   aaaaaaatcg catatacgca tgaaagatta tggtcttatt tccggaaagc atgaaaggtg 1080
   attgatactt ccaagaagtc cctgttactc aggaaaatta tcaaatattc tactcagaga 1140
   tacttggaaa gactgaagga aaggaagaac gaagaaagca gaatctagac ttatgtgggg 1200
   agagatttgt ggcagaggaa aagtattctc tttgaatccg acaagggatt tgcctggggg 1260
   aatttcctgt ccagcctttt attaccaggg tcttttgaag ccgggctccc cattgggcag 1320
   ttccctggga gtgcagtggg gaattcttac actttccctc taggtccccg aaggatctcg 1380
   ttttctcagt gtctctttca ggttggcagg agccttgagc ctgacacttc cctttgatgg 1440
   gacaggcaag ctctgtgggc gcgtaaacac gctgtaacca agttctttgc tgattttaca 1500
   gttttgtgtg ctcccgagaa gaagtgatcg tactcaattg tctattgctg gcctgccccc 1560
   taagagcctg ggggctcctt tcccctaacc cagaactagc tgcacggggg gcggggaaat 1620
   gggggtgggg aaggagtggg agggcagtgg tttccgcgag cagagcgatg ttactgagtg 1680
   agtccctgaa tggggagcgc tgctgtcccc aagccgattg gtacttcttg tcaggaagaa 1740
   acgccaagag gtgggagtgc ctggggaggg aggcaggcgg tccctaccgc aggcgcgggg 1800
   agctgccttt ccgcccctcc gcctgctttc caagcctgga ctcttaggag tggctgaagc 1860
   tgcggagcgc ttttggagcc tgtgaatgaa ccctcctcct ctccctcctc cttcttctcg 1920
   ctgagtctcc tcctcggctc tgacggtaca gtgatataat gatgatgggt gtcacaaccc 1980
   gcatttgaac ttgcaggcga gctgccccga gcctttctgg ggaagaactc caggcgtgcg 2040
   gacgcaacag ccgagaacat taggtgttgt ggacaggagc tgggaccaag atcttcggcc 2100
   agccccgcat cctcccgcat cttccagcac cgtcccgcac cctccgcatc cttccccggg 2160
   ccaccacgct tcctatgtga cccgcctggg caacgccgaa cccagtcgcg cagcgctgca 2220
   gtgaattttc cccccaaact gcaataagcc gccttccaag gtaatcacgt ttcttttgtt 2280
   ccccccttaa aaaacaaaaa caaaaaactt atagaaaaaa acccgcgagc ttagaaaaaa 2340
   gaagcaattg gtagaaggct ttaattaagg caaagagctg taaggcgaag ttaagaaaat 2400
   gtaggcactt aaaaaatgca ggtaactttc ataagggctt ttggggagag gcatacagag 2460
   ggaccttggt gttgaaaaag attcagacaa aagaaaccca g 2501
<210> 43
   <211> 2501
   <212> DNA
   <213> Homo Sapiens
<400> 43
   tgtgggtcat taatgcaatg ttatttaaga ctaggatttg gctgggcgca gtggctcacg 60
   cctgtaatcc cagcactgtg ggaggccgag ccgggaggat cacctgaggt caggagttca 120
   agaccagcct gaccaacatg gtgaaaccac gtctctacta aaaatacaaa attagccggg 180
   catagtcaca tgcctgtaat cccagctact gggtagcctg aggcaggaga atcgcttgaa 240
   cccgggaggc ggaggcggag tttgcagtga gccaagattt cacaactgca ctccagtctg 300
   ggccacaaga gcgaaaaccc gtctcaaaaa aaaaaaaaag actaggattt gacataaggc 360
   ctgaggggta ttcttttgtt ttgttttgcc ttgttttcaa gaggccaaaa tcttcacagt 420
   tgaaaatttc tgttgaacca cagagatttg aaccaactca gtttagaaag cctggggatt 480
   tgaacaacgg tatggatcgg aaatctcttc atctgtcagt tttcatcatt ctaggcagta 540
   aaatagattt ccctttagga gcttttcacc gtttggggtt ctccagcagt gggatgtggg 600
   gaatcaaccc ttcttcgtct ccacccaaac attaggtggg agcaaggggt gggaagtaga 660
   gaaagtggat agaggtctcc agtggatatg ggatctttgt gtagaccagc acagtcctca 720
   gaaatctcat gcaagcaaca taggtactgt tatattttct agtggccacc ttttaaaaag 780
   taaacaggtg aggccgggcg cggtcgtcac gcctgtaatc ccagcacttt gggaggccca 840
   ggcgggcgga tcacgaggtc aagagatgga gaccatcctg gtcgacacgg tgaaaccccg 900
   tctctactaa aaatacaaaa attagctggg catggtgacg cgcgactgta gtcctagcta 960
   ctggggaggc cgaggcagga gaatcacttg aaccctggag gtggaggttg ccacgctcca 1020
   ctacactcca gcctggcgac agagtgagac tccgtctcaa aaaaaagaaa gtaaacaggt 1080
   gaaattaatt ttaataatat attttgttta acccaacgta tccaaaatac tatcatttga 1140
   aagtgtaatg aatataaaaa tattcatgag atatttttca ttctcatatc catactgtct 1200
   tggactctaa tgtgtatttt acacttacag cacaattaat ttgggactag ctacatttca 1260
   gctcaacaat agccaatagc atatgggata gcgcaaataa actctgcgtc tctgttgctt 1320
   ctttgggtct cggagacctc aaccctttct tcagattgca aaccttcttg ccttcaagcc 1380
   tcggctccaa caccagtccg gcagaggaac ccagtctaat gaggtacgct cccttcctgc 1440
   cattctctat tccattaacc tgtttcgtgg taaacgtagg actgatcctc caaaattacc 1500
   ttattaatta gcttacatat ttattatcta tctgtcccac cagaatgcag gtttccggaa 1560
   ggcagggatt taaaaaaatc tgttttgttc tatgtgattt tcccatacca agcaccgtgc 1620
   ccggcacaag ctgggatccc agtacacatc tcgggacgga agaaccgtgt ttccctagaa 1680
   cccagtcaga gggcagctta gcaatgtgtc acaggtgggg cgcccgcgtt ccgggcggac 1740
   gcactggctc cccggccggc gtgggtgtgg ggcgagtggg tgtgtgcggg gtgtgcgcgg 1800
   tagagcgcgc cagcgagccc ggagcgcgga gctgggagga gcagcgagcg ccgcgcagaa 1860
   cccgcagcgc cggcctggca gggcagctcg gaggtgggtg ggccgcgccg ccagcccgct 1920
   tgcagggtcc ccattggccg cctgccggcc gccctccgcc caaaaggcgg caaggagccg 1980
   agaggctgct tcggagtgtg aggaggacag ccggaccgag ccaacgccgg ggactttgtt 2040
   ccctccgcgg aggggactcg gcaactcgca gcggcagggt ctggggccgg cgcctgggag 2100
   ggatctgcgc cccccactca ctccctagct gtgttcccgc cgcgccccgg ctagtctccg 2160
   gcgctggcgc ctatggtcgg cctccgacag cgctccggag ggaccggggg agctcccagg 2220
   cgcccgggtg agtagccagg cgcggctccc cggtcccccc gacccccggc gccagctttt 2280
   gctttcccag ccagggcgcg gtggggtttg tccgggcagt gcctcgagca actgggaagg 2340
   ccaaggcgga gggaaacttg gcttcgggga gaagtgcgat cgcagccggg aggcttcccc 2400
   agccccgcgg gccgggtgag aacaggtggc gccggcccga ccaggcgctt tgtgtcgggg 2460
   cgcgaggatc tggagcgaac tgctgcgcct cggtgggccg c 2501
<210> 44
   <211> 2501
   <212> DNA
   <213> Homo Sapiens
<400> 44
   gatgtgaaaa gagaaataat tgaaaaagac tggagtacat atactatcta cagtgtctgt 60
   tttaaagaaa caacattcta gcacaccttt ctacccttga ctaagattac tgtaatgaga 120
   gcaccagtac ccctgagtaa ccgaaagggc attttggaaa ctgagctttt ggtgtttata 180
   tgaacattct gtcttccagg acctgccttg atttattcaa gactcatact gctgtatatg 240
   gtgttgtata cattaggggt agttgggtag cagtaactga tatagaaaat tttaaatgta 300
   aaaaacactg gggagtgaac ctttccatta tatatatata tatatatata tatatatata 360
   tatatatata tatatatata tataaattca catcaggatg agtttctgtt taggcaatgt 420
   tggaaaacgc tatttccatt tttttttttt aacaaatatt taacaaacat ttataaggca 480
   cttaaatcca tgctggctct tacaaatgtt gactcatttc tcataaccac cttggggtag 540
   aaacggagag gctaaacaat ctgcaggcga tgcttcacta ctaaatgcag gtggcagcct 600
   tgcctgtgtt ctctgcttgg ctaggaacac aggtcttacc tattgagctg ggctgtgtag 660
   aactctgttg tggagacatc tgcccctggg gcagaagcct ctgctttttc cccctcctcc 720
   catcttactc catgtctcag agagctctga atcccacttg gagaatcaca cttaaaccct 780
   ctaaaaacct aatgatgaat aaaaataagt tctctagaac ttctggagaa aaaagtaata 840
   aagctaccag gttaaatgac tgaaattcct gagagaaaac aacatgtgtg tgtttctcta 900
   gaaagggggc ccaatactga ataccaggaa gtcctatagt aaatggaatg tgactctatg 960
   tgggatccgg cgttcctatt tcatccgaat gcatgtctgc tgcttcagtg ggaagggtgc 1020
   ttgcacacca ggtacccact ccctggtgtc atgtgctatg cagtccaaag acagaaccag 1080
   gaatggtgag cccatgagcc tgctggaccc agcccctccg aggtccggag tgacaaccag 1140
   tgccgtattt ctagatcaaa cctgaacccc tcctacaggg aaaagatttc caggggattt 1200
   tgaaagttcc aacattttac agggaagaag gaagataagc aggatatgaa agaagagttc 1260
   atgttataca gccctggctt ccactgacgc taacactgga ttcagctttt gacactgata 1320
   atctgttgcc accaaatgga aaacgtaaac aagatattct aagtgtggtt agagaatatg 1380
   caacacaagg aacaagcaga acattcttct ctggaatctg acataatgga ctgtactttc 1440
   acagacagca ctgatgttag atgtacgtga aataggctaa actgaaaata agaaaggctg 1500
   aggcagagag gataatatag ctccagccta tctcccagca ccttgttaat ttctctcaac 1560
   ctccagccac aaatccgaga cacaacgctc ttcctccaaa gaggtcgcgc cttctctgtg 1620
   gtggttctca gggatccgcc ccagctcctt ctccgttccc agccccacac actgggatca 1680
   ccaggcaccc aagatcccac ctctcaggtg gtatcttcag cgcaggctgc cactcagccc 1740
   ccctccaggg atctggggca gaaggcgaat atcccagagt ctcagagtcc acaggagtta 1800
   ctctgaaggg cgaggcgcgg gctgcatcag tggaccccca caccccaccc gcaccccaag 1860
   cgctccaccc tgggggcggg gccgtcgcct tccttccgga ctcgggatcg atctggaact 1920
   ccgggaattt ccctggcccg ggggctccgg gctttccagc cccaaccatg cataaaaggg 1980
   gttcgcggat ctcggagagc cacagagccc gggccgcagg cacctcctcg ccagctcttc 2040
   cgctcctctc acagccgcca gacccgcctg ctgagcccca tggcccgcgc tgctctctcc 2100
   gccgccccca gcaatccccg gctcctgcga gtggcgctgc tgctcctgct cctggtagcc 2160
   gctggccggc gcgcagcagg tgggtaccgg cgccctgggg tccccgggcc ggacgcggct 2220
   ggggtaggca cccagcgccg acagcctcgc tcagtcagtg agtctcttct tccctaggag 2280
   cgtccgtggc cactgaactg cgctgccagt gcttgcagac cctgcaggga attcacccca 2340
   agaacatcca aagtgtgaac gtgaagtccc ccggacccca ctgcgcccaa accgaagtca 2400
   tgtaagtccc gccccgcgct gcctctgcca ccgccggggt cccagaccct cctgctgccc 2460
   caaccctgtc cccagcccga cctcctgcct cacgagattc c 2501
<210> 45
   <211> 2501
   <212> DNA
   <213> Homo Sapiens
<400> 45
   ggcgacagag caagactccc tcttaaaaaa aaaaaaaaaa aaagattctg agtcaaagtg 60
   ctcaagttga atgcattttg tcatccacaa gacaaatcgt gttaacccct tgtggtttac 120
   tttatctata aaatagagat aacaatagtt cctgcttcta gggttgttgt gggaattaaa 180
   gacttagaat aatgttcagc ctctaatcag tgctgtcaca actgtctgat acaattgtat 240
   tatatttgtg tactttgtag attgatatta aatcatactt ttaaaaatag gtgcttaatg 300
   ttccactcaa ttaccttaaa acatgtttaa ttatgtctct atcctactct tataacactt 360
   ctataaaaac tttttacata tagcgtccac ttttggttca gtttcttagg aaaataactt 420
   tgagagtcag ctatctgaac caaagaaaca ttaacattac cagactatat tgggattttt 480
   gagactggct tttatcaatt ctttagctac gggctcttgt catcatctct accagtgacc 540
   taagtgtcaa acccaaatgc cttgtatctg tcccattaaa gagatgcagc atctgctcct 600
   ttcttactgt ttccatttcc tctgccatgc ctcctcttac aaccataaat atccaggtct 660
   cttaggtttt aaacggggca tctctcaacc cccacattct tttccttggt tattcccttc 720
   cctccaacag ttcaattcac ctagatcccc acgcctgaaa ttatcctaga tgtcctagag      780
   gcgcctcatc attacaatgg tacattattc tccactcctt tacatgtcac gccagctttc      840
   aaactgaaaa tctgagcgtt catccctggt gcatcacctt taaattccag atctccaaaa      900
   tccagggtca tgtaacctta aaaaattttt accctctctt ctccactgcc cttgttcagg      960
   ccttatctct tccagcagct gttccaaagg cctactctgt tttcctttcg gagtgctaac     1020
   ctccaccgaa gcctccaccc agttgccaat tctgccccat gcctgataat ttgctcgtgc     1080
   gttgacatac ataaaatttc taagacaaaa attttttaat aatggtaaat gaaccttggg     1140
   aactgcatac agatcataca gatccataat aagagaaaag gtcccagatt aacacggaaa     1200
   actttccatt taactaacat ttgcactggt aaacttcatc aagcaagacc ctacttaatc     1260
   ccacattacc ttctactgaa gaggttgtgg tcattctctg gaaatatctg aattcattcc     1320
   tacaagttag agaaacagcg ttactcgaaa cattatccct tgggctcgag ctctaaggca     1380
   cctgacaaac ggagcgctgt gggtaggggt gaggtgtttt ctccagggct gggactttgc     1440
   cctgggcgag ggcgccgcag ggcaaagacc tcaccgggca gcagaatccg ggcagaaatc     1500
   agcaactggg cctcccgcgc agcagaaaag gggaatccag tcggggccca cccttcctgc     1560
   cagcgcagac cgcaagtctg gccccatcct ctcgccggga gtcggcctgg cgcgtcccgc     1620
   ccaggtaccc cgaccgtggg cagcctgcgc ccgtttgggt cccatcgccc cggcccggca     1680
   gatacctgag cggtggccag ggcaggtccc cgttcttgcc gatgcccatg ttctgggaca     1740
   cagcgacgat gcagtttagc gaaccaacca tgacagcagc gggaggacct ccgagcccgc     1800
   tcgttacagc agaacgcgcg gtcaagtttg gcgcgaaatt gtggccgccc cgccccctcg     1860
   tccctatttg tgcaggcgag gccccgcccc cccgccccgg cgcacgcagg gtcgcggcgt     1920
   gctcgcgccc gcagacgcct gggaactgcg gccgcgggtt cgcgctcctc gccgggccct     1980
   gccgccgggc tgccatcctt gccctgccat gtctcgccgg aagcctgcgt cgggcggcct     2040
   cgctgcctcc agctcagccc ctgcgaggca agcggttttg agccgattct tccagtctac     2100
   gggaagcctg aaatccacct cctcctccac aggtgcagcc gaccaggtgg accctggcgc     2160
   tgcagcggcc gcagcgcccc cagcgcccgc cttcccgccc cagctgccgc cgcacgtagt     2220
   aggttctgtc tgggactggg cagggccatc ggggctgggg gggcggggct tgtgggtaag     2280
   gcgggcggag gcgtggaccc tccgcccgat gatagggctg gaggaggaag gggcgggctg     2340
   aagaagggga aggtgggaag agcccagccg gggctacaaa ttgggtgaag cgctgaggtt     2400
   ttagtacttc cgtttgagga gataggcaaa ggttatgcag gtttttaatg gcaggcctga     2460
   gacaggaact caggtctcct gactcccatt ctgatgaggg g                         2501
<210> 46
   <211> 1092
   <212> DNA
   <213> Homo Sapiens
<400> 46
   aagcttcccc ttcatcatcc aagaaggcat tcaggtcttt ctgtgctagg ccccaggtaa      60
   agtgctggac tacccagtaa ttgggttcag tagcaggatg gcctcagatt gaggtcccag     120
   ggccaaagga ccactcctct cctcagcgct ggtccgggaa aggcaagctc cgggcgggag     180
   cgcacgccgc gcccccgaag cctggctccc tcgccacgcc cacttcctgc ccccatcccg     240
   cgcctttcca ggtcttctcc cggtgaaccg gatgctctgt cagtctccta ctctgcgtcc     300
   tcggccgcgg cccgggtccc tcgcaaagcc gctgccatcc cggagggccc agccagcggg     360
   ctcccggagg ctggccgggc aggcgtggtg cgcggtagga gctgggcgcg cacggctacc     420
   gcgcgtggag gagacactgc cctgccgcga tgggggcccg gggcgctcct tcacgccgta     480
   ggcaagcggg gcggcggctg cggtacctgc ccaccgggag ctttcccttc cttctcctgc     540
   tgctgctgct ctgcatccag ctcgggggag gacagaagaa aaaggaggta gaatggatcc     600
   ccttggcctt cccctgtggt cgggggcggg ccagggtggg ccgcgttgcc caggcagccc     660
   tgccgtgttg ctaggcagcc tggtcgccgg cgtgggcgat gccggcgctg gggcgggagc      720
   cgcgagggtg ggaggccctg gggcgtttcc gggacgtgga gttagcaggg ttctgacttg     780
   aaaaacgacg gcaaagcgtg ttcttgactg cttctgagca cctcacacct ttcagaccca     840
   gggcgccttt attcccagct ggaagcccag cttagagcaa tggtgccact aaaaggggtg     900
   tgttggatgt gaaaataccc tttggaagta tttataagcc tgcaggaaat atgttttcct     960
   tattttctta ctctgctccc ttcattaccc atttcaagaa gcaacagaac ctgtgcagag    1020
   tgtgttttaa gttacactgt atgtttattt ttgtttatgt tgaactcggt gtatacttgt    1080
   gagaataagc tt                                                        1092
<210> 47
   <211> 2501
   <212> DNA
   <213> Homo Sapiens
<400> 47
   cgaaatgaaa cctcgcccag gaggccgcgg acctggacac ccggcgccac ctccttcacc      60
   tctgacccag gtttcctccc ggcgctgcga gctcccgggg aagggttaga gccggcagcc     120
   ctccccagcc cggggagggg agagggttat gcgaccccac ctctggctag ggccggggag    180
   gcctttgctt cccgggagcc ctgcccgggc tccttggtcg cagggctgct gggtcccagg     240
   caggaacgag agggtgaggc ccacatgtgg cccggcggcc cagggcggct tgcagcgtcc     300
   tcactgtccc ggctgccagg ggctgcggcg acgcggccag tcagcagcga gttcaggtcg     360
   cgcagatttt attgatgagc tctgactttc agcactttcc ctaagtcaag aagagtctag     420
   cgtacccttc ggctgcttca tttcagcctc cctgcctcag ctcttcagcc ctattccccc    480
   tcgccctgtc ctggggtgtg tacagcagcc caggccttcc ttctccttcc cggctccgtg    540
   gcccgaagcc gccgagagag ctcgggacag cgcaggacca ggcagccgct cgctctcctg    600
   tcaccttaac tgcaggctcc gaggggcgcc tttggagtgt actgaggtgt gtcctaatcg    660
   tgcggcattc aacaaatgga cttctggtgt gtggtcagaa gagaaaagcc atttacttac    720
   tttcctcccc ggttttctgg caacagctga aggggagctg cctccgtgga ctgagcagac    780
   ccaggagagg gagtcgtggt gcggagacac acgcaccaca cacagatgac cggtggcaca    840
   cacgacacac gctgacatac cgacatcgcc agtgggacac acacacacac acacacacac    900
   acacacacac acacagagag agagagagaa tccctcccag cattggtcat ccgccccccc    960
   acccaggctt ccactccccc tcccctctta tctcccctgg cttcccctcc tctcgggcgc   1020
   tgcgaaaagc agccgcactt agtcaacaaa tggcacgtgg gagaagttgg tgagtgtttg   1080
   gtgaggactc ttcagggctt ttcacaagaa ccctctgtac acaaagtaag tggcgtgttt   1140
   actcgggcct ctccagccag agctgtgcct ctgctccgct gcgcaccgcg gcttccgaaa   1200
   ggagaaagga gagaagaaag ggcggggaga gcggggtgga ggatttggac aggccctgga   1260
   ggcttgggct ggggaggcct ctggcctcgt ttagttctcg gcccggcaac ctcctctcgg   1320
   cctaggcttc gccgcggcct ccgcagctgg aatggagctg ccaggaccca gtgacgctcc   1380
   cgcccctttc ctcttcttcc aaggggccag gtgggctggg gtgcggccgc cgctgtgctc   1440
   tgtgtcttgg ggccccggct gggatggggt gggggcgggc gggggcgggg cggcaggcca   1500
   cgctgtcctg gagttggcaa gaaaggacag cacagaaact tgcaccctcc gaggactggg   1560
   agtcccgagt ccagcttagg gggagtgggg gcgcgacccc caacccagaa accttcactt   1620
   gaccgctcaa gttcgcggca gcagggcggg ccgcgccgaa tctcggcgtg cgcggagcgg   1680
   ggagatgcag gcgagcgcca gagcccgggc tcgggggccc tgcgccgggg agaggagccg   1740
   ggacccaccg gcggagccga aaacaagtgt attcatattc aaacaaacgg accaattgca   1800
   ccaggcgggg agagggagca tccaatcggc tggcgcgagg ccccggcgct gctttgcata   1860
   aagcaatatt ttgtgtgaga gcgagcggtg catttgcatg ttgcggagtg attagtgggt   1920
   ttgaaaaggg aaccgtggct cggcctcatt tcccgctctg gttcaggcgc aggaggaagt   1980
   gttttgctgg aggatgatga cagaggtcag gcttcgctaa tgggccagtg aggagcggtg   2040
   gaggcgaggc cgggcgccgg cacacacaca ttaacacact tgagccatca ccaatcagca   2100
   taggtgtgct ggctgcagcc acttccctca cccacactct ttatctctca ctctccagcc   2160
   gctgacagcc cattttattg tcaatctctg tctccttccc aggaatctga gaattgctct   2220
   cacacaccaa cccagcaaca tccgtggaga aaactctcac cagcaactcc tttaaaacac   2280
   cgtcatttca aaccattgtg gtcttcaagc aacaacagca gcacaaaaaa ccccaaccaa   2340
   acaaaactct tgacagaagc tgtgacaacc agaaaggatg cctcataaag gtgagtccgc   2400
   ttctttcttc tcgctttatt tttattgcaa tattcagaca ggtctccccc ttcctccccc   2460
   cttccttcct cccctctcgc cggtcccctc ccccactgct a                        2501
<210> 48
   <211> 2501
   <212> DNA
   <213> Homo Sapiens
<400> 48
   tgatggttgc acaactctga gtacatgaaa aatcaatgaa ctgatacttt gagtgagctg      60
   tatgatactg gaattacacc tcaataaagc atggtaactg ttttaagata ggctggaaag     120
   agaaagcctg aaaacaacaa taatgatatt aataaattag tttacttctc tagtctcata     180
   tacttctgtg cccacacttg ctcctgttct attcataatg gtccccttgc agttgccata     240
   ttatatcctg ccatttgatg cccggtgaac attctatacc tgcttcccag aattctcttt     300
   acctttcctc tatctgccta acttccacat atctaaaatt aatcagagta aactatttac     360
   tagaacaacc aactccaaat cctagtaacc taacatgata aaggtttgtt tctcactcat     420
   atagcccctc cccagatgat cgaggggtcc aggctcctta cctctagtgg ctcccccacc     480
   ttctggagtc ttctgcattc tttatacatg gttgagataa actatgagtc attagcacag     540
   ctagaccttg aggtcctaca agaaaatttg caaatcattc actctgtttt gaacaaggta     600
   tatttaagat gatgttaaaa tacccaatgg tcttgggtca aatacagttt atgactgtgt     660
   atctaaaata tatattgcaa tattcttccc tttttctact gacttcatga atttagcggg     720
   gatccatttt ataagctcaa agataattac ttttcagact aagaatattt agggtaaaaa     780
   gtactgttca acatctctac tgaggatgtt atgatgtagc acactgtata agctggagct     840
   aaaggaaact ttccttaaag tgctatttac taaaaattgg aacacattcc ttaagacaaa     900
   tcgaagtgtg gcacacaaca tccaaacttc catcatagat acagaggtgt taccatctcc     960
   cactcccaaa tttctttgtc acgctgagga tactcaagag gagcaggaca tgttggtcgc    1020
   agcaggagaa acttgaaagc attcactttt atggaactca taagggagag aatttcttat    1080
   tttagtatcg tccttgatac atttattatt ttaaaagata atgtagccaa atgtcttcct    1140
   ctgtgttaaa tctttacaaa actgaaatct taaaatggtg acaaaaattc tacttctgat    1200
   agaatctatt catttttcca attagatagg gcataattct taatttgcaa aacaaaacgt    1260
   aatatgctta tgaggttcca tcccaaagaa cctgctattg agagtagcat tcagaataac    1320
   gggtggaaat gccaactcca gagtttcaga tcctaccggt aattggggta gggaggggct    1380
   ttgggcgggg cctccctaga ggaggaggcg ttgttagaaa gctgtctggc cagtccacag    1440
   ctgtcactaa tcggggtaag ccttgttgta tttgtgcgtg tgggtggcat tctcaatgag    1500
   aactagcttc acttgtcatt tgagtgaaat ctacaacccg aggcggctag tgctcccgca    1560
   ctactgggat ctgagatctt cggagatgac tgtcgcccgc agtacggagc cagcagaagt    1620
   ccgacccttc ctgggaatgg gctgtaccga gaggtccgac tagccccagg gttttagtga    1680
   gggggcagtg gaactcagcg agggactgag agcttcacag catgcacgag tttgatgcca    1740
   gagaaaaagt cgggagataa aggagccgcg tgtcactaaa ttgccgtcgc agccgcagcc 1800
   actcaagtgc cggacttgtg agtactctgc gtctccagtc ctcggacaga agttggagaa 1860
   ctctcttgga gaactccccg agttaggaga cgagatctcc taacaattac tactttttct 1920
   tgcgctcccc acttgccgct cgctgggaca aacgacagcc acagttcccc tgacgacagg 1980
   atggaggcca agggcaggag ctgaccagcg ccgccctccc ccgcccccga cccaggaggt 2040
   ggagatccct ccggtccagc cacattcaac acccactttc tcctccctct gcccctatat 2100
   tcccgaaacc ccctcctcct tcccttttcc ctcctccctg gagacggggg aggagaaaag 2160
   gggagtccag tcgtcatgac tgagctgaag gcaaagggtc cccgggctcc ccacgtggcg 2220
   ggcggcccgc cctcccccga ggtcggatcc ccactgctgt gtcgcccagc cgcaggtccg 2280
   ttcccgggga gccagacctc ggacaccttg cctgaagttt cggccatacc tatctccctg 2340
   gacgggctac tcttccctcg gccctgccag ggacaggacc cctccgacga aaagacgcag 2400
   gaccagcagt cgctgtcgga cgtggagggc gcatattcca gagctgaagc tacaaggggt 2460
   gctggaggca gcagttctag tcccccagaa aaggacagcg g 2501
<210> 49
   <211> 2501
   <212> DNA
   <213> Homo Sapiens
<400> 49
   taccttcata aaaggatctt tgacttggta agtgtgtgcg atgcatactt ttcatgttac 60
   accacaagtg ccacttagca actccactag acagggcagt gtttcagcat ggggtggggt 120
   gccccctgac aggcttttaa aaggcccgga tgccaatgca cattccaaca ctatccacaa 180
   aaaggagact ggagcagtgc tcttccctgc attgggcaag gagactctcc ctccctgcct 240
   aaccacttgc ctgccctgtt ttgtgggaga attacaagta aatgctacag aggcagtgga 300
   gaaaaaaggg tgttttaatt cctctccaga gtttccttta tttgatgtat gttgcatcct 360
   ttaaacaagt tgtgcaaaat ggctgcaggg tagattggct ctccctttta aagctctcca 420
   tccggctggg tttatttgta aatactgcat ctatccttct tagtgtttta ggactggctg 480
   gaaagactct tcttcctgta ggttgggtca gtgtgagaga tctaaaaaat cattttccct 540
   taaaattact gtattttaat aaaaggattg ggcaggggct ggaatgagag aaaactggtc 600
   cttcaaaatg taaaactgtc atacttaaac cagtttacaa aatatgcgtt taattatgtg 660
   gtgggatgtg tgtaggtgta tgatgagaga ggcaaccaac atggctattt ggggtgcaag 720
   gatgtgggaa caggcaagta attttcacat tggactttca tcctagggag ctgggttcta 780
   gtcacagctc tgagctgtgt gaccttgggt aggtctcatc tccccggggt tttgtttcac 840
   cagttgaaca gtatgaggat gagtcacagc taacatttgt tccatgatat ttacccagca 900
   ccatacaagt gttatttctg tcctcccagt taacactgac gtgggtagta ttatatgccc 960
   attttacaga tgaggaaact gaagcctgaa gaagttaaat acttatccca gaacacacag 1020
   ctggtaagtg gcagacctgg aattggaatc tagttcagtt tgattcccca acccatgctc 1080
   ttgaccacta tactgttttt tcaagtccag atctgaaatc tcattttctg tgtggctgtg 1140
   tgtttgggac aggggtaacc aattcctgac tactctatat gctgcataga acctggagag 1200
   gatttttcaa agtaaatgaa tctcgaaagc tggattgcag agcaaacgag tgcagtcaat 1260
   tcagccaggg gcttgcaaga gggagaaaga gaaaaagact gtggaatgga aagtttccca 1320
   acccaagcct ttcccaaggg gtagccattc tctgttctac agtttagggc ttgcatgtgc 1380
   tttttctgga gtggaaaaat acataagtta taaggaattt aacagacaga aaggcgcaca 1440
   gaggaattta aagtgtgggc tggggggcga ggcggtgggc gggaggcgag cgggcgcagg 1500
   cggaacaccg ttttccaagc taagccgccg caaataaaaa ggcgtaaagg gagagaagtt 1560
   ggtgctcaac gtgagccagg agcagcgtcc cggctcctcc cctgctcatt ttaaaagcac 1620
   ttcttgtatt gtttttaagg tgagaaatag gaaagaaaac gccggcttgt gcgctcgctg 1680
   cctgcctctc tggctgtctg cttttgcagg gctgctggga gtttttaagc tctgtgagaa 1740
   tcctgggagt tggtgatgtc agactagttg ggtcatttga aggttagcag cccgggtagg 1800
   gttcaccgaa agttcactcg catatattag gcaattcaat ctttcattct gtgtgacaga 1860
   agtagtagga agtgagctgt tcagaggcag gagggtctat tctttgccaa aggggggacc 1920
   agaattcccc catgcgagct gtttgaggac tgggatgccg agaacgcgag cgatccgagc 1980
   agggtttgtc tgggcaccgt cggggtagga tccggaacgc attcggaagg ctttttgcaa 2040
   gcatttactt ggaaggagaa cttgggatct ttctgggaac cccccgcccc ggctggattg 2100
   gccgagcaag cctggaaaat ggtaaatgat catttggatc aattacaggc ttttagctgg 2160
   cttgtctgtc ataattcatg attcggggct gggaaaaaga ccaacagcct acgtgccaaa 2220
   aaaggggcag agtttgatgg agttgggtgg acttttctat gccatttgcc tccacaccta 2280
   gaggataagc acttttgcag acattcagtg caagggagat catgtttgac tgtatggatg 2340
   ttctgtcagt gagtcctggg caaatcctgg atttctacac tgcgagtccg tcttcctgca 2400
   tgctccagga gaaagctctc aaagcatgct tcagtggatt gacccaaacc gaatggcagc 2460
   atcggcacac tgctcaatgt aggtttattt ttttcccttc t 2501
<210> 50
   <211> 2501
   <212> DNA
   <213> Homo Sapiens
<400> 50
   ggaggataga aatataaatt aaagaatgac acaaataatt ataaagttac agctgttaaa 60
   agaaaagcat atggtgccaa gagaacgtgt aatacaagat ctactcatgg aggtgaggga 120
   aagcttgccc atcaaagaag ttatgattca atccacgaag accaggagtt ggctgggtga 180
   agaaaaaaag gtcagaggaa ggaagtccac actggggaag gctctaagca taaagggtag 240
   gaggattaca gaggcatatt cacgaaattt ggagaaggct ttcagtaagc aaggagaagc 300
   caaatgaaag tttacgggag agttggaggc ttgaagacac gttcaaggat ctggttttta 360
   tcttctcttt atctcaagag cagtgggaag ccattaaatg attttaatca gagggttggt 420
   ataactagtt ttgtattttg aaaagctgaa ttcagctctc gtttgagaaa ctgagtgaaa 480
   gagcccagaa cggccgtggc tgagggtgac tcgtgggaga ctcctacaca agccatggca 540
   gtggcatggg ctggtggcag aagagggaat agggagaaga tttggaactc aatcttcctc 600
   cattgacaaa gtcactccag ctttggcaag gcaattaatt ggtgggaaag aagatgccta 660
   gccctcctga tttcactgca ctttctgcat cttcaacatg agtactggga agtggcaaaa 720
   catccagagg cagcttgggt gctaggtgga gcatgagtta aaattccagg atgaagcaaa 780
   tgaacactta gaatgacagg aaagatttgg gagttgggtt tgggggaggg ctatttacct 840
   ttattccctg gagaccctgg cacaaaccct tgcctctgca atcttcctct caggtaaagg 900
   aattcattaa atgaattgct agaagatcta ctgaccagag ggctgtacag aatcatatct 960
   ttgagagtgg gaagtaggtt gatcacatag tttattatcc aatcaggaca tatctgaaag 1020
   agaaaggggg ttctattaat atttaaacta caaaacatgt acaccaggaa tgtcttgggc 1080
   aaatctggtt gccctagcaa gaaaggaaat ttgaaagttt atactgttct gctcccatgt 1140
   taccccgttt gcacatgaga gggtaagtat tctctttctt cacctgcatt aagggaataa 1200
   aagcacaagc attcaggtga ctcccaaccc acttttaatt ttacagtttc tgctatactc 1260
   tatacattct gaaaattaca tttcccacca ctatcacttc gtgataggtg atcatttaca 1320
   attactcact gactcagtcc cgggaagagg cggtgcaaaa tgggacgctc tatccaggtg 1380
   ctcattagaa atgcagaatc tctgcctgcc tcctagacct actgaattag aatctgcatt 1440
   tttaaataag atttccaggt gatcaatatg tacattaaaa cttgagaaaa acctctagac 1500
   ttcgacctaa agaaaaacat tttacaactt gacagtgtat gcacatacat acatgcatat 1560
   agacacaact gaagcacaaa tttaatgaag tagaatttac cgttactatt ttatttggga 1620
   aagaaatgtg ctcgcgactc aatagattgg agtattcact cctggatctc aacttgcaat 1680
   ttgaaaacgc atctctaaag cacctaggag caatctgaag aaagctgagg ggaggcggca 1740
   gatgttctga tctactaggg aaaacgtgga cgttttctgt tgttactttg tgaactgtgt 1800
   gcacttagtc attcttgagt aaatacttgg agcgaggaac tcctgagtgg tgtgggaggg 1860
   cggtgagggg cagctgaaag tcggccaaag ctctcggagg ggctggtcta ggaaacatga 1920
   ttggcagcta cgagagagct aggggctgga cgtcgaggag agggagaagg ctctcgggcg 1980
   gagagaggtc ctgcccagct gttggcgagg agtttcctgt ttcccccgca gcgctgagtt 2040
   gaagttgagt gagtcactcg cgcgcacgga gcgacgacac ccccgcgcgt gcacccgctc 2100
   gggacaggag ccggactcct gtgcagcttc cctcggccgc cgggggcctc cccgcgcctc 2160
   gccggcctcc aggccccctc ctggctggcg agcgggcgcc acatctggcc cgcacatctg 2220
   cgctgccggc ccggcgcggg gtccggagag ggcgcggcgc ggaggcgcag ccaggggtcc 2280
   gggaaggcgc cgtccgctgc gctgggggct cggtctatga cgagcagcgg ggtctgccat 2340
   gggtcggggg ctgctcaggg gcctgtggcc gctgcacatc gtcctgtgga cgcgtatcgc 2400
   cagcacgatc ccaccgcacg ttcagaagtc gggtgagtgg tccccagccc gggctcggcg 2460
   gggcgccggg ggtcttcctg gggtccccgc ctctccgctg c 2501
<210> 51
   <211> 2500
   <212> DNA
   <213> Homo Sapiens
<400> 51
   ttcccatcaa gccctagggc tcctcgtggc tgctgggagt tgtagtctga acgcttctat 60
   cttggcgaga agcgcctacg ctccccctac cgagtcccgc ggtaattctt aaagcacctg 120
   caccgccccc ccgccgcctg cagagggcgc agcaggtctt gcacctcttc tgcatctcat 180
   tctccaggct tcagacctgt ctccctcatt caaaaaatat ttattatcga gctcttactt 240
   gctacccagc actgatatag gcactcagga atacaacaat gaataagata gtagaaaaat 300
   tctatatcct cataaggctt acgtttccat gtactgaaag caatgaacaa ataaatctta 360
   tcagagtgat aagggttgtg aaggagatta aataagatgg tgtgatataa agtatctggg 420
   agaaaacgtt agggtgtgat attacggaaa gccttcctaa aaaatgacat tttaactgat 480
   gagaagaaag gatccagctg agagcaaacg caaaagcttt cttccttcca cccttcatat 540
   ttgacacaat gcaggattcc tccaaaatga tttccaccaa ttctgccctc acagctctgg 600
   cttgcagaat tttccacccc aaaatgttag tatctacggc accaggtcgg cgagaatcct 660
   gactctgcac cctcctcccc aactccattt cctttgcttc ctccggcagg cggattactt 720
   gcccttactt gtcatggcga ctgtccagct ttgtgccagg agcctcgcag gggttgatgg 780
   gattggggtt ttcccctccc atgtgctcaa gactggcgct aaaagttttg agcttctcaa 840
   aagtctagag ccaccgtcca gggagcaggt agctgctggg ctccggggac actttgcgtt 900
   cgggctggga gcgtgctttc cacgacggtg acacgcttcc ctggattggg taagctcctg 960
   actgaacttg atgagtcctc tctgagtcac gggctctcgg ctccgtgtat tttcagctcg 1020
   ggaaaatcgc tggggctggg ggtggggcag tggggactta gcgagtttgg gggtgagtgg 1080
   gatggaagct tggctagagg gatcatcata ggagttgcat tgttgggaga cctgggtgta 1140
   gatgatgggg atgttaggac catccgaact caaagttgaa cgcctaggca gaggagtgga 1200
   gctttgggga accttgagcc ggcctaaagc gtacttcttt gcacatccac ccggtgctgg 1260
   gcgtagggaa tccctgaaat aaaagatgca caaagcattg aggtctgaga cttttggatc 1320
   tcgaaacatt gagaactcat agctgtatat tttagagccc atggcatcct agtgaaaact 1380
   ggggctccat tccgaaatga tcatttgggg gtgatccggg gagcccaagc tgctaaggtc    1440
   ccacaacttc cggacctttg tccttcctgg agcgatcttt ccaggcagcc cccggctccg    1500
   ctagatggag aaaatccaat tgaaggctgt cagtcgtgga agtgagaagt gctaaaccag    1560
   gggtttgccc gccaggccga ggaggaccgt cgcaatctga gaggcccggc agccctgtta    1620
   ttgtttggct ccacatttac atttctgcct cttgcagcag catttccggt ttctttttgc    1680
   cggagcagct cactattcac ccgatgagag gggaggagag agagagaaaa tgtcctttag    1740
   gccggttcct cttacttggc agagggaggc tgctattctc cgcctgcatt tctttttctg    1800
   gattacttag ttatggcctt tgcaaaggca ggggtatttg ttttgatgca aacctcaatc    1860
   cctccccttc tttgaatggt gtgccccacc ccccgggtcg cctgcaacct aggcggacgc    1920
   taccatggcg tagacaggga gggaaagaag tgtgcagaag gcaagcccgg aggcactttc    1980
   aagaatgagc atatctcatc ttcccggaga aaaaaaaaaa agaatggtac gtctgagaat    2040
   gaaattttga aagagtgcaa tgatgggtcg tttgataatt tgtcgggaaa aacaatctac    2100
   ctgttatcta gctttgggct aggccattcc agttccagac gcaggctgaa cgtcgtgaag    2160
   cggaaggggc gggcccgcag gcgtccgtgt ggtcctccgt gcagccctcg gcccgagccg    2220
   gttcttcctg gtaggaggcg gaactcgaat tcatttctcc cgctgcccca tctcttagct    2280
   cgcggttgtt tcattccgca gtttcttccc atgcacctgc cgcgtaccgg ccactttgtg    2340
   ccgtacttac gtcatctttt tcctaaatcg aggtggcatt tacacacagc gccagtgcac    2400
   acagcaagtg cacaggaaga tgagttttgg cccctaaccg ctccgtgatg cctaccaagt    2460
   cacagaccct tttcatcgtc ccagaaacgt ttcatcacgt                          2500
<210> 52
   <211> 286
   <212> DNA
   <213> Homo Sapiens
<400> 52
   tttgcactag gctggaagtg gccgccagtc ccccgtgcaa ttccattctc tggaaaagtg      60
   gaatcagctg gcattgccca gcgtgatttg tgaggctgag ccccaacagt ccaaagaagc     120
   aaatgggatg ccacctccgc ggggctcgct cctcgcgagg tgctcacccc gtatctgcca     180
   tgcaaaacga gggagcgtta ggaaggaatc cgtcttgtaa agccattggt cctggtcatc     240
   agcctctacc caatgctttc gtgatgctgc tgctgatcta tttggg                    286
<210> 53
   <211> 1400
   <212> DNA
   <213> Homo Sapiens
<220>
   <221> unsure
   <222> (1371)
   <223> unknown base
<400> 53
   ttccagctgt caaaatctcc cttccatcta attaattcct catccaacta tgttccaaaa      60
   cgagaataga aaattagccc caataagccc aggcaactga aaagtaaatg ctatgttgta     120
   ctttgatcca tggtcacaac tcataatctt ggaaaagtgg acagaaaaga caaaagagtg     180
   aactttaaaa ctcgaattta ttttaccagt atctcctatg aagggctagt aaccaaaata     240
   atccacgcat cagggagaga aatgccttaa ggcatacgtt ttggacattt agcgtccctg     300
   caaattctgg ccatcgccgc ttcctttgtc catcagaagg caggaaactt tatattggtg     360
   acccgtggag ctcacattaa ctatttacag ggtaactgct taggaccagt attatgagga     420
   gaatttacct ttcccgcctc tctttccaag aaacaaggag ggggtgaagg tacggagaac     480
   agtatttctt ctgttgaaag caacttagct acaaagataa attacagcta tgtacactga     540
   aggtagctat ttcattccac aaaataagag ttttttaaaa agctatgtat gtatgtgctg      600
   catatagagc agatatacag cctattaagc gtcgtcacta aaacataaaa catgtcagcc     660
   tttcttaacc ttactcgccc cagtctgtcc cgacgtgact tcctcgaccc tctaaagacg     720
   tacagaccag acacggcggc ggcggcggga gaggggattc cctgcgcccc cggacctcag     780
   ggccgctcag attcctggag aggaagccaa gtgtccttct gccctccccc ggtatcccat     840
   ccaaggcgat cagtccagaa ctggctctcg gaagcgctcg ggcaaagact gcgaagaaga     900
   aaagacatct ggcggaaacc tgtgcgcctg ggcggtggga actcggggag gagagggagg     960
   gatcagacag gagagtgggg actaccccct ctgctcccaa attggggcag cttcctgggt   1020
   ttccgatttt ctcatttccg tgggtaaaaa accctgcccc caccgggctt acgcaatttt    1080
   tttaagggga gaggagggaa aaatttgtgg ggggtacgaa aaggcggaaa gaaacagtca    1140
   tttcgtcaca tgggcttggt tttcagtctt ataaaaagga aggttctctc ggttagcgac    1200
   caattgtcat acgacttgca gtgagcgtca ggagcacgtc caggaactcc tcagcagcgc    1260
   ctccttcagc tccacagcca gacgccctca gacagcaaag cctacccccg cgccgcgccc    1320
   tgcccgccgc tgcgatgctc gcccgcgccc tgctgctgtg cgcggtcctg ncgctcagcc    1380
   atacaggtga gtacctggcg                                                1400
<210> 54
   <211> 2501
   <212> DNA
   <213> Homo Sapiens
<400> 54
   gataatcttt tcatacaaga tgcattctgc ttttgtgggc ctcttgcagc cctcaagccc      60
   ccatctgatt tgtacacaat gatccagtgg gccagaggag cccagagcca tgagcggccc     120
   atccctccaa gaactatttc tgactgtcca gtatcatgga gcaagtggaa agaagaaaaa     180
   aaaaacccaa ttacttttcg aagagcaaga tgaatgctgt agaaggagaa ggaaggggag     240
   ggagatggat gggtgccgat tccagaatct tcagatctgc ttggatgaat cattacctat     300
   gatttgcggg acaagaatct gattttattc atcaaccagt agaaactttt ctttctgcct     360
   cccaacatct gaaatccaac aaacatgtgc cttaggaaca taccggtcat cttttagagg     420
   cattttatat acatattgag taactagaaa acactctttc cgtaatacac acacacacac     480
   acacacacac acaccatctt gtcatacaac actcccacgc aagaaaagcg aaactgctgt     540
   ttgatgaatg taaacacttg gctgtttgca gcagtcggga gtcctgccag gtttaagtgc     600
   taagatggga ggtgaacccc aggggtttcc ccctgcccgt gctgagatcc ttatttggtc     660
   aagcttctac ctatgccctg gcctcggagc gagcccgata gcgctggatc acagcagagg     720
   gagcgaggcg gctgacgtcc catcccgaag agatgaatgg aattccagga agctagagtc     780
   atgctggctt gggacagtgg cttggagacc agacttcaat gacagaagca ctaggcagcg     840
   gcactcatgg caatgtgtgc acccacagaa atgtaaccca cacctcgggt tcaggagccg     900
   aaaaatgaaa agaacgttta gggaggaaaa agggaaatac aataataggc agagagtaat     960
   ttattactct atgggtctgc tctgtaaata gctgaagact ctggagccag atggttctgc    1020
   aaattctcca aacaggagtc acgttaagaa gcacgagtgg gcacaaaaac tgtttttcaa    1080
   gacacaattt caatttggct tgtggaaact ggatacgagt aagtttcctt aaaattcgag    1140
   tagaaagcag ctgtcctccc cgggcccctt gatgagaata cgcacaccgc ccccaagcgg    1200
   ccggccgagg gagcgccgcg gcagcgggag aggcgtctct gtgggccccc tggcagccgc    1260
   ggcaggaaag ggcccgaagg cagcgaaggc gaacgcggcg caccaacctg ccggccccgc    1320
   cgacgccgcg ctcacctccc tccggggcgg gcgtggggcc agctcaggac aggcgctcgg    1380
   gggacgcgtg tcctcacccc acggggacgg tggaggagag tcagcgaggg cccgaggggc    1440
   aggtacttta acgaatggct ctcttggtgt cccctgcgcc ccgtcggccc atttttcttt    1500
   ttacaaaacg ggcccagtct ctagtatcca cctctcgcca tcaaccaggc attccgggag    1560
   atcagctcgc ccgaaagccc ctgcgccacc ccgcgggccc tcctaggtgg tctccccagc    1620
   cccgtccctt ttcgggatgc ttgctgatca ccccgagccc gcgtggcgca agagtacgag    1680
   cgccgagccc gtgcgcgcca aggctgcgtg ggcgggcacc gacttttctg agaagttcta    1740
   gtgctcccaa gccccgaccc ccgccccctt cactttctag ctggaaagtt gcgcgccagg    1800
   cagcgggggg cggagagagg agcccagact ggcccccacc tcccgcttcc tgcccggccg    1860
   ccgcccattg gccggaggaa tccccaggaa tgcgagcgcc cctttaaaag cgcgcggctc    1920
   ctccgccttg ccagccgctg cgcccgagct ggcctgcgag ttcagggctc ctgtcgctct   1980
   ccaggagcaa cctctactcc ggacgcacag gcattccccg cgcccctcca gccctcgccg    2040
   ccctcgccac cgctcccggc cgccgcgctc cggtacacac aggtaagtcg cccccggcgg    2100
   ccgccgagga ccaaagctgc ccgggacatc cacctggagc gctgaggctt cagtccctct    2160
   ggtggacccc ggaacctaca ctctccccgc tcgcctaccc cagcccgctc ctctcagccg    2220
   ctggaggact cttcagggca aggctccaga gccatcctct ccagccttga ggttcacaaa    2280
   ccaactcatc aggacacccc aagatttcct tactctctga agtcctcctt aagcctttgt    2340
   atcagcactc cagggaagag tctgtacttc ccctgccctc cctgcaaccc caaactacag    2400
   ttcctgatct tgctcacctt cgacttccca aaagccccca aattgttggt cttgcgcccc    2460
   ccacacttta aaaccagcat ctctttcctc cacctctctc t                        2501
<210> 55
   <211> 7258
   <212> DNA
   <213> Homo Sapiens
<400> 55
   ttcaatagga agcaccaaca gtttatgccc taggactttg ttcccacaat cctgtaacat      60
   catatcacga cacctaaccc aatccttatc aagccctgtc aaaaacggac tttaaaccaa     120
   gctgcaaatt ttcagtaatc tggccttgcc tttccccctc tgatagcacc atcaaacaaa     180
   cccccttact gccgaaagca ataagcccgg ctttgttcca tccactggtt gtgttggtga    240
   tatctgggga ctgccactga acagacgcac agagggagcc cctacaggca ggggtttttc     300
   tgtctgtgct tcttgggaga gtatgtctcg tacatttgtc gcgtgatgaa gacttcacag     360
   ctccatccag cgaccagact cacagctcca tccagctgcg gcaagggggt ctgaggcagt     420
   cttaggcaag ttggggccca gcgggagaag ttgcagaaga actgattaga ggacccagga     480
   ggcttcagag ctgggctgag gtagagagtc tcctgtgcgc cttctctcct ctctgcaatt     540
   cggggactcc ttgcactggg gcaggccccg gcaggtgcat gggaggaagc acggagaatt     600
   tacaagcctc tcgattcctc agtccagacg ctgttgggtc ccctccgctg gagatcgcgc     660
   ttcccccaaa tctttgtgag cgttgcggaa gcacgcgggg tccgggtcgc tgagcgctgc     720
   aagacagggg agggagccgg gcgggagagg gaggggcggc gccggggcgg gccctgatat    780
   agagcaggcg ccgcgggtcg cagcacagtc ggagaccgca gcccggagcc cgggccaggg     840
   tccacctgtc cccgcagcgc cggctcgcgc cctcctgccg cagccaccgg tgagtgccgc     900
   ggtcctgaga tccccgggcc ggatgcgcgg cggccccagc tcccgagcgt ctgcctgccc     960
   cgccctgggc tgcccgggct ccctgggctc cccggcggct gcacggagtc aaggcgcccc    1020
   gtcccgggcg tcccccgcgg gtgccgatcc aggctgcccg gagtccggag cccatagagg 1080
   agagagacag ctggggagcc tggtcaccgc gggcatctcc cctgcgctgc agtcgcccgc 1140
   ctggcctgcc ttcccgttcc tccgcctctt gccctgactt ctccttcctt tgcagagccg 1200
   ccgtctagcg ccccgacctc gccaccatga gagccctgct ggcgcgcctg cttctctgcg 1260
   tcctggtcgt gagcgactcc aaagtgagtg cgctcttgct ttgactgatg ctgcccaagg 1320
   acctctgatc agcaccaggg gagaggaggg gctgctcagg gagctggggt ctccggattc 1380
   catccacagc agggccagac tctccccagg aaatgggaca gggtggcagc ggaggcttga 1440
   gaaccacggg ggttggcact ggctggcaag ggaggaagag ggccaccggg actgccccag 1500
   cctgcgggca tctggtagat gaagcttaat ccatttctcc tggctggaaa ccatggtctt 1560
   ccatttgaga actagatacg aacagggtga ggcgagaggg agagggaaga gtgggttttg 1620
   ggattggggc cagtttaccc tcaccctgga tccctggagc atgggacctt tgatgaagcc 1680
   tcctcccgaa tctcttccag ggcagcaatg aacttcatca agttccatgt gagtatccac 1740
   ccctacaaca gttggctgca cagacaagtt gggaaggctt caggggacac tcccctccct 1800
   gccctctgct gcagcgtgcg ccacccctta ccacttccac tccccctcgc ttaccccacc 1860
   tttgttctct ccagcgaact gtgactgtct aaatggagga acatgtgtgt ccaacaagta 1920
   cttctccaac attcactggt gcaactgccc aaagaaattc ggagggcagc actgtgaaat 1980
   aggtatgggg atctccactg caactgggag agaaatttgg ggacagggag ggatgggtgg 2040
   gaggcaagag caggcaggag ttaggagctg gaggtagggt gggtgacatc ttcatcccta 2100
   tgtgacaagc ataaacacac acacacgctc acgaaacagt ggccacacaa atgtgaggtg 2160
   gggttggaag gagaccctgt ccagtcttct ggcaggtctg aaacgacatc tttaaaatgt 2220
   ccgttggcag ccgggcatgg tggctcacgc ttgtaatccc agcattttga gaggtcaagt 2280
   ttgagtggat catttaggtc aggagttcaa gaccagcctg gacaacatgg tgtaaccctg 2340
   cctctactaa aaatgcaaaa atcagcctgg catggtggtg gatgcctgta gtcccagcta 2400
   cttgggaggc tgaggcagga gaattgcttg aacatgggag gccagatctc agtgagctga 2460
   gatcacacca ctgcactcca actgggcgac agagcaagac tccatctcaa aaaaaaaaaa 2520
   aaataaaagt tagttggaat gttcttctct ttctcatatt ctctcatcct cctgtcccct 2580
   tgtagataag tcaaaaacct gctatgaggg gaatggtcac ttttaccgag gaaaggccag 2640
   cactgacacc atgggccggc cctgcctgcc ctggaactct gccactgtcc ttcagcaaac 2700
   gtaccatgcc cacagatctg atgctcttca gctgggcctg gggaaacata attactgcag 2760
   gtgaggtggg ggcaacaagg accaaaagcc ctccctacag cttcccagaa accttgttac 2820
   catccccttc tcccagaggg ctggccatag cacaagagaa gtgcggcctc tggttgagtc 2880
   ttccctgagg ggaggaggca gggaaggccc tctgggttgg aatgacatcc cctatctttc 2940
   tgtgttgtgc caggaaccca gacaaccgga ggcgaccctg gtgctatgtg caggtgggcc 3000
   taaagccgct tgtccaagag tgcatggtgc atgactgcgc agatggtgag catcactgac 3060
   ctgctgatga caggtgggtg gaaggggaca aacttacatg tccccttatt ccatcacagg 3120
   aggactgagg aggtgggggg tgcccgagag ggatgctttc tcctacctgc ctccctaaga 3180
   catccctctg tttgtcctcc aggaaaaaag ccctcctctc ctccagaaga attaaaattt 3240
   cagtgtggcc aaaagactct gaggccccgc tttaagatta ttgggggaga attcaccacc 3300
   atcgagaacc agccctggtt tgcggccatc tacaggaggc accggggggg ctctgtcacc 3360
   tacgtgtgtg gaggcagcct catgagccct tgctgggtga tcagcgccac acactgcttc 3420
   atgtacggcc ctgggtttct cctcttcgac tcttctgccc caccccaagc acatcccttt 3480
   ctccttccca gcaaagtgtt ccgcctcatt tctccctcat ctgcccctgt ccatgcgccc 3540
   atggccttgg ggacaagtcg tgctttgagg cctctaggga gggaaggaag aagtggcatg 3600
   atttcatggg actaagctgt ttgatgggta tcttcttcca cagtgattac ccaaagaagg 3660
   aggactacat cgtctacctg ggtcgctcaa ggcttaactc caacacgcaa ggggagatga 3720
   agtttgaggt ggaaaacctc atcctacaca aggactacag cgctgacacg cttgctcacc 3780
   acaacgacat tggtgagggg gaacgcccgc gactactgtg gccataatgg cttggggaga 3840
   gtgggaccca gggagagact ggagctgagt tgaagctgcc ggtggggcag gggtggggcg 3900
   agggaccttg aagcctcgat atacatgaca aaggatggca gggaagagtt ccatgaagtc 3960
   tgaggggcct ggtgctcctc tggagagacc ctgaatttcc ccaacaagta gccctcttgc 4020
   gagtggaaac agccctgtgg gtatatggct tgggctggga aggccctgtt tatatgaatt 4080
   agaaaaagac acaccttcct ttgtgggatg cagcctctgt ctgtgctagg atatagaact 4140
   tggagaatgg agccttggga tggattccag cctaactacc tcagggggat cctctagagt 4200
   gcagctggga gtttttgcag aaacgacctg tacagctgta tgcagtggct ctggccatcc 4260
   aagccttttt caacacctgg aacaaagccc ttggggcatg ggcaggggga ggtttccagg 4320
   tgataagcga ccagcagacc tccctggatg actgacctag ggataggcat agctacttcc 4380
   tcggcacttg gaggggacag atggggaccg cctaaccagt agtgatcttt ctcctctgac 4440
   cctctgtcct cccccagcct tgctgaagat ccgttccaag gagggcaggt gtgcgcagcc 4500
   atcccggact atacagacca tctgcctgcc ctcgatgtat aacgatcccc agtttggcac 4560
   aagctgtgag atcactggct ttggaaaaga gaattctagt aagtgacaat tgcgactgac 4620
   ttagaaggtc ctgaggagtg ttttgacctg aaaatgagcc cagtgtgatc aagggaagac 4680
   tgcagagtta gaggtgggag cactgaggcg gtggcagatg ggtccaggga tggatgaaga 4740
   gtgttgttta gggagcgatg ggctgcaaag gtaaatagat ggtaggggct ataggtggag 4800
   gtaaatggct cagatttgca tggagagaga ataatgggcc tctccctggg tgatgatact 4860
   ttatggtgtc ccctctctgg cgagacgtcc cacgtggagg cagataaatc ttgatgcaaa 4920
   cgcctccctg ttttctccac ctagccgact atctctatcc ggagcagctg aaaatgactg 4980
   ttgtgaagct gatttcccac cgggagtgtc agcagcccca ctactacggc tctgaagtca 5040
   ccaccaaaat gctgtgtgct gctgacccac agtggaaaac agattcctgc caggtgagtg 5100
   ttccaagcat ctctctccac ctcttccata tctccccaga gctcctgggc ttgttccagc 5160
   cagcttaagg gtgtctctct ctagccaaag ccctaagtag ccagaatcag gagctcaggt 5220
   ctttgagggt ttaaaccagt ccttatgtgt ttgccagaca ttaccaaaaa aatcccagct 5280
   ctgcgctagt cacttcagac tgggggcacg agatcctaga aagaggaaac agtaaaagac 5340
   aatgtaactc agtgcccagg gtgtgttgtg aactataaat gatcaggtgt tcaggagagg 5400
   gaggtgagtg ccaacctgag ggtcagggag gggaggcttt aaaggaaatg tgacttgata 5460
   ggcatttgaa gaggcagagg gaagaaagga aggtgtttca gttgaaagat acaaaactga 5520
   gaaggaggct ggcatattcc gggtggggag gagaactagg gtctgggagt gtggatggaa 5580
   tagtggcaga tgacagggct tttaaagcca agcaggggat tttccaactt cgatgtggta 5640
   gaaatggggc tgcgtcaggc acagtggctc atgcctgtaa tcccagcatt gggctaggcc 5700
   gtagtcgatg gatcattgag gccagagttg agaccggcct ggaccaacat ggtgaaaccc 5760
   tgtgtctact aaaaaatgca aaaaaaaaaa ttagccaggt gtggtggtgc ctgcctgtaa 5820
   tcccagctaa tcaggaggct gagacatgga atcgcttgag cacaggaggc aagtttgacg 5880
   tgagctgaga tcacgtcatt gcacgccagc ctgggcgaca gagcgagatt ctgtcctccc 5940
   gccgaaaaaa gaaagaaaat gggaagtcgc taaggacttt gactgggaaa ctcttccctc 6000
   tctctggtat ggttgggtga tgggatcaga aatcccctcc tcacttctct agggctcatc 6060
   ttttgtatct ttggcgtcac agggagactc agggggaccc ctcgtctgtt ccctccaagg 6120
   ccgcatgact ttgactggaa ttgtgagctg gggccgtgga tgtgccctga aggacaagcc 6180
   aggcgtctac acgagagtct cacacttctt accctggatc cgcagtcaca ccaaggaaga 6240
   gaatggcctg gccctctgag ggtccccagg gaggaaacgg gcaccacccg ctttcttgct 6300
   ggttgtcatt tttgcagtag agtcatctcc atcagctgta agaagagact gggaagatag 6360
   gctctgcaca gatggatttg cctgtgccac ccaccagggt gaacgacaat agctttaccc 6420
   tcaggcatag gcctgggtgc tggctgccca gacccctctg gccaggatgg aggggtggtc 6480
   ctgactcaac atgttactga ccagcaactt gtctttttct ggactgaagc ctgcaggagt 6540
   taaaaagggc agggcatctc ctgtgcatgg gtgaagggag agccagctcc cccgacggtg 6600
   ggcatttgtg aggcccatgg ttgagaaatg aataatttcc caattaggaa gtgtaacagc 6660
   tgaggtctct tgagggagct tagccaatgt gggagcagcg gtttggggag cagagacact 6720
   aacgacttca gggcagggct ctgatattcc atgaatgtat caggaaatat atatgtgtgt 6780
   gtatgtttgc acacttgtgt gtgggctgtg agtgtaagtg tgagtaagag ctggtgtctg 6840
   attgttaagt ctaaatattt ccttaaactg tgtggactgt gatgccacac agagtggtct 6900
   ttctggagag gttataggtc actcctgggg cctcttgggt cccccacgtg acagtgcctg 6960
   ggaatgtact tattctgcag catgacctgt gaccagcact gtctcagttt cactttcaca 7020
   tagatgtccc tttcttggcc agttatccct tccttttagc ctagttcatc caatcctcac 7080
   tgggtggggt gaggaccact ccttacactg aatatttata tttcactatt tttatttata 7140
   tttttgtaat tttaaataaa agtgatcaat aaaatgtgat ttttctgatg acaaatctcc 7200
   ctggtgcttg tatgggaagg agttggagta cataaaaagg agaaaataac aaaggtgg 7258
<210> 56
   <211> 852
   <212> DNA
   <213> Homo Sapiens
<400> 56
   cagctgcgct ggaggctgag gccgattgct tgagcccagg atttggaggc cagcatgcgc      60
   aacataatga gacccagtct ctaaatgcat gcctctctat atattaaaat tctgatgtga     120
   aaatatttta aaatttaata catttcaaat gtttttaatt gtataataaa caaaatgtaa     180
   ataataaaat aatttaatat taaattcaaa aatgaggtag aaacaaagca cagcgatata     240
   aataataaat tttcctttac atttttgagg cggtcttttg agttttggat ttccttctta     300
   ggtcactgaa atgtgctcct tggagccagc ccgcaaatca cgcatttaga aaaacataac     360
   tatacactcc taaccctaag tattagaagt gaaagtaatg gaatctcgat gtaaacacaa     420
   tatcactttt ttgtagagct attttgagta taataaattt gaactgtgcc aatgctggga     480
   gaaaaaattt aaaagaagaa cggagcgaac agtagcttcc tcgtccgctg actagaaaca     540
   gtaggacgac actctcccga ctggaggaga gcgcttgcgc tcgcactcag ttggcgcccg     600
   ccctcctgct ttttctctag ccgccctttc ctctttcttt cgcgctctag ccacccggga     660
   aggcactgcg gtagctgggc tctgattggc tgctttgaaa gtctacgggc tacccgattg     720
   gtgaatccgg ggccctttag cgcggtgagt ttgaaactgc tcgcacttgg cttcaaagct     780
   ggctcttgga aattgagcgg agagcgacgc ggttgttgta gctcgctgcg gccgccgcgg     840
   aataataagc cg                                                         852
<210> 57
   <211> 2501
   <212> DNA
   <213> Homo Sapiens
<400> 57
   tcttgtcact ccatgcactg tgttccgtat gctaaatagt ttgagaaacc caaatgggcc      60
   atgttcgcct acatttcatt gtcctgtact tcctgtcctg tactagcaaa gcagtcccat     120
   tggtctttct tctcctcatt aacaataaag gtaacacttt tgatgttgtt tcttcagaaa     180
   accttcattc atcaaaactg cctcaaagat catgtttgtt tgattccaga acttcctgta     240
   attacctgtt attgtaacac tcatcactgt attttactta cttgtgtaac taattttcca     300
   tattctgcac tagacaacaa agtcctttaa gtcaggtact atatctattt acatagcatt     360
   cacatctcct acaataaggg acattagcag ataaacaaca catattaaat gaataatgaa     420
   gtttctgaaa tactacagtt gaaaactata ggagctacat tatatagaat aaacatttac     480
   tttgctatag aattcagtgt aacccaggca ttattttatc ctcaagtctt aggttggttg     540
   gagaaagata acaaaaagaa acatgattgt gcagaaacag acaaaccttt ttggaaagca     600
   tttgaaaatg gcattccccc tccacagtgt gttcacagtg tgggcaaatt cactgctctg     660
   tcgtactttc tgaaaatgaa gaactgttac accaaggtga attatttata aattatgtac     720
   ttgcccagaa gcgaacagac ttttactatc ataagaaccc ttccttggtg ctctttatct     780
   acagaatcca agacctttca agaaaggtct tggattcttt tcttcaggac actaggacat     840
   aaagccacct ttttatgatt tgttgaaatt tctcactcca tcccttttgc tagtgatcat     900
   gggtcctcag aggtcagact tggtgtcctt ggataaagag catgaagcaa cagtggctga     960
   accagagttg gaacccagat gctctttcca ctaagcatac aactttccat tagataacac    1020
   ctccctccca ccccaaccaa gcagctccag tgcaccactt tctggagcat aaacatacct    1080
   taactttaca acttgagtgg ccttgaatac tgttcctatc tggaatgtgc tgttctcttt    1140
   catcttcctc tattgaagcc ctcctattcc tcaatgcctt gctccaactg cctttggaag    1200
   attctgctct tatgcctcca ctggaattaa tgtcttagta ccacttgtct attctgctat    1260
   atagtcagtc cttacattgc tttcttcttc tgatagacca aactctttaa ggacaagtac    1320
   ctagtcttat ctatttctag atcccccaca ttactcagaa agttactcca taaatgtttg    1380
   tggaactgat ttctatgtga agcacatgtg ccccttcact ctgttaacat gcattagaaa    1440
   actaaatctt ttgaaaagtt gtagtatgcc ccctaagagc agtaacagtt cctagaaact    1500
   ctctaaaatg cttagaaaaa gatttatttt aaattacctc cccaataaaa tgattggctg    1560
   gcttatcttc accatcatga tagcatctgt aattaactga aaaaaaataa ttatgccatt    1620
   aaaagaaaat catccatgat cttgttctaa cacctgccac tctagtacta tatctgtcac    1680
   atggtactat gataaagtta tctagaaata aaaaagcata caattgataa ttcaccaaat    1740
   tgtggagctt cagtatttta aatgtatatt aaaattaaat tattttaaag atcaaagaaa    1800
   actttcgtca tactccgtat ttgataagga acaaatagga agtgtgatga ctcaggtttg    1860
   ccctgagggg atgggccatc agttgcaaat cgtggaattt cctctgacat aatgaaaaga    1920
   tgagggtgca taagttctct agtagggtga tgatataaaa agccaccgga gcactccata    1980
   aggcacaaac tttcagagac agcagagcac acaagcttct aggacaagag ccaggaagaa    2040
   accaccggaa ggaaccatct cactgtgtgt aaacatgact tccaagctgg ccgtggctct    2100
   cttggcagcc ttcctgattt ctgcagctct gtgtgaaggt aagcacatct ttctgaccta    2160
   cagcgttttc ctatgtctaa atgtgatcct tagatagcaa agctattctt gatgctttgg    2220
   taacaaacat cctttttatt cagaaacaga atataatctt agcagtcaat taatgttaaa    2280
   ttgaagattt agaaaaaact atatataaca cttaggaaag tataaagttt gatcaatata    2340
   gatattctgc ttttataatt tataccatgt agcatgcata tatttaacgt aaataagtaa    2400
   tttatagtat gtcctattga gaaccacggt tacctatatt atgtattaat attgagttga    2460
   gcaaggtaac tcagacaatt ccactccttg tagtatttca t                         2501
<210> 58
   <211> 2501
   <212> DNA
   <213> Homo Sapiens
<400> 58
   attaattctg caaattttaa taaatgcttt attttaagct aaatgctgag atgaaaaaat      60
   gaaaccatat gagttagcaa agtagaaaat ataggcatat taatcagtaa atgcagaatg     120
   ataaatgctc catcaatatg cacttgttgt agtgaggcca ccgaggaggg tgcaatcctc     180
   tcaacctggg aggagcaggt aggacttcag atgtcatcca actcaaagat atagtgaggg     240
   acttgatcaa acatttgcca agaccactat gagttaaatg aatagattag gcatttctcc     300
   aatgttgcaa gcttcgaatc atatccaaac tcagaacaac atagcttggt cataatgatc     360
   ccaaggatcc tattggccat tgtctttgag cctcaaagga acatattaaa actccataat     420
   acccttttga tctattctga agttaagtag tgaatttaca tgatgatgac acaaacactg     480
   taaaggacct ctgggttact tgtttataag ctagtatttc ctgaatcaat ttttctgatc     540
   cctagatatt tggtaggtga agtcatacct atatatcccc acaccctaga acagcatctc     600
   caacttattt ttccctcctt gtcttttagt gggagccaca tcagtatcca agaggagatc     660
   cagaagcctc tccaaccagg tagggacagt tatagattcc agacctcagc tatggccttt     720
   gttacagagt acaaatgtta tatagtacaa gtttattgta cacatcccat tgagtctctg     780
   agctttagaa ttttcttgta gaatttaaca gttttttcat gccgtattta catattattg     840
   ctagtattta gaattttctt ctccaaatgt ataacgttta ttattgcatt ttttgtatcc     900
   actaagtgga aaatcatgca ttagatattg tagaagtaga tacaacaatg aacaagaact     960
   ggtcctgacc atgagaggaa ctgatgatcc aatgggggag atagacctgc acgtgtttaa    1020
   taaaaggaag tggctattcc ggtttctttt tgatgggcaa gcattttgca aggccttggg    1080
   ctatgtgtgt gcaaggctaa gccagttagt taattgggat ttttttaaaa aggcacttca    1140
   ctggggggaa aaggaacata gagttggtta ttgtcccctt gcctataata aaaacctatt    1200
   atttttaatt ttttaactgg gtttgcggtt aaatctcaca gcccaagaga tttgccactt    1260
   cagatggatt ccatacactt gcatttaagt atgcaaaaaa attccaatta tccagcaatt    1320
   taaccaaatt attggtaact tttctaaaac aaaaaaaaat tgtttccctt gttttggcag    1380
   caatttcagt tacagtcctt tactttctac tcaagaaaat agtttcaaaa agttgatgtt    1440
   tgttgctaaa agaactattt ttatgaataa atataaaact aagaagttat ggtgtccctt    1500
   ttttaaaaaa tgactcatca aaagaaataa ctttttcctt tctcttgtaa gagaaaaaaa    1560
   ttaatctctt ttagaattgc aaacatattt ccttgatgga gaaaatcaat tcacatggca    1620
   tagtcgttat ttatccagtt caaaaaccag agtagaattt actactctgt ctccattttt    1680
   tctctcccca cccccttaac ccacattgga ttcagaaagc ttcattctgc aatcagcatt     1740
   gtcctttatc tttccagtaa agatagcctt ttggagtcga agatgaggaa aagcctgtat    1800
   tttatagtct tggaagtgtc ttcttttgcc aggacagaga gaggagcttc agcagtgaga    1860
   gcaactgaag gggttaatag tggaacttgg ctgggtgtct gttaaacttt tttccctggc 1920
   tctgccctgg gtttcccctt gaagggattt ccctccgcct ctgcaacaag accctttata 1980
   aagcacagac tttctatttc actccgcggt atctgcatcg ggcctcactg gcttcaggag 2040
   ctgaataccc tcccaggcac acacaggtgg gacacaaata agggttttgg aaccactatt 2100
   ttctcatcac gacagcaact taaaatgcct gggaagatgg tcgtgatcct tggagcctca 2160
   aatatacttt ggataatgtt tgcagcttgt aagttatttc ccttcatctg tttcaaatgt 2220
   tagcattcaa ttttagccct ggttttggct tcagtcagtt ttgcgatagt agtgaagtaa 2280
   agacactagg attttaaaca gtaggaaaag ttaatttagt ctaactttta atatgcaatt 2340
   gagttttgct atataccatt gtactgtcat agttagagct gaaaattgat gtttttggta 2400
   tctttttttc caaaggcaat tgagtaattt ggattctgtc tctagtcggt ctgtctcttt 2460
   agtttcctat acttgacaat gaggtcaaac ttagcaaata a 2501
<210> 59
   <211> 2501
   <212> DNA
   <213> Homo Sapiens
<400> 59
   ataaaaaaag acatgaaatg aatcggggaa aatatttgct acataactaa gaatgaaggc 60
   ccttaataaa atctgtaaaa ctatacacac ttttaggaat gaatcaacaa ataatttcta 120
   tgaattagaa aaaagtgaca atccaactaa aaaatgaata agggatataa gcaatgtgtt 180
   tcacagaaaa aataaaaatt gacaatgaag ttatgaaaaa atgttcagtc tccttagtaa 240
   ttgcacaaaa caaactaaaa caatgagaca ttacccctaa gattagtaaa tgttaaagaa 300
   aaataataat tggtgagggt gtggggaagt gggcacttac acctatgttt ggaaatataa 360
   attggtgcaa ccttataggg agagcaatct cacaacattt tccaaagact tacatgcaca 420
   accctatggc agagaaattt attcctcttc caggattttt tttccttcaa aaacagtgat 480
   gtggatgaaa aacacatgtt cactactgca cagggtataa cagctgaaaa ctggaaacga 540
   taatactcac attcccttca gtaggggaat ggttaaataa attttacaag ccatctggta 600
   gataccaggc atgagctaaa agttagggtc cagttagaga tggaaagcac accagtaatt 660
   tgaaagggaa aatgtaatat gaagaattat taactagtaa aagaaggcta actgctaaag 720
   gtacaagagc actcaagctg tctgcagtca gcaggccccg gctggtgagc aggaagctgc 780
   ccgctgggag gctgccaaag ttccctgaag gtgagcacca ctggttctac aagctgctgg 840
   cagtcatggc gttaagagca ggaagagaag caccagaacc cggaagagaa atccagtcct 900
   ctgctaggcc ttgcaccgtc cctctggcgc cctctactga caaagccagt aaaattgtgc 960
   cgctagcaaa ggagatcttt ttatgggatg tagcttggtg tcaccaaaga gaacagagtg 1020
   gacttggagc tcagatgcaa cacaatgatt gatactggca cagtatactt accctgcttt 1080
   tgtaaacaaa atggtatatg tgatgtctct ctttgtctct ctgtatataa aacaatattt 1140
   gtttctactt attatgtatt tatgtcttta ctctgcatgc caggagctaa gtattttgca 1200
   tgtattaact cattttgttc tcataataac cttcacatgc aggaatcatt atagctactt 1260
   tatgaatgag ccgaggaagg cactgagacg ttaagtaact tgcccaaggt cacgcagcta 1320
   gtaagtggca gagcaagaat tactatggct ttataagcct aggaaaaagt ctgaaagaat 1380
   caaaatgtta acagcgggga cctcaaggaa gcattgaaga ggccatggga gaagttttca 1440
   ctttgttaaa aaatcagtcc ttcaaataaa taaatacagt gaggcttccc cagaagcaga 1500
   tgtcactatg cttcctgtac agcctgtgga actgtgagcc agttaaacct cttttcttta 1560
   taaattatcc agtcttaggt atttctttat aacagtgcta ggatgagctg atacagtttc 1620
   ctacactgta acctaaggca atgctttgca caaagggatg agccagattg cttagtaatt 1680
   aaaacgcaaa tacaaaccac aagcatatcc attcatgaat tggggggctg ctttgtgtgc 1740
   atagataagg tatatttttt aaaaaaatta tttttccaag aagaaaataa accagttaat 1800
   aaacgacaac tcacagtgcc aggaagtgag aaacaagtgt gtgataaacg gtggagaatg 1860
   ggagcactct ccgcagtggg cgggaggaga cgaggagggc gttccctggg gagtggcagt 1920
   ggttggagca aaggtttgga ggaggtaagt catgtgctct gagtttttgg tttctgtttc 1980
   accttgtgtc tgagctggtc tgaaggctgg ttgttcagac tgagcttcct gcctgcctgt 2040
   accccgccaa cagcttcaga agaaggtgac tggtggctgc ctgaggaata ccagtgggca 2100
   agagaattag catttctgga gcatctgctg tctgtgagat taagcactat gtatattgct 2160
   ttattcactc cccacagcaa ccttaccaag cagttctttt ccacgtgaaa agatggaggc 2220
   tgggtggagc aaaaggaggt atttagagtc ctcagcaagt gagaggcaga gctgggattt 2280
   gaatccagat ctgcctgata ctgaagtcta ggctggttcc acctctccgg actgctttcc 2340
   agggagtaga agacagatat tttaccttag ctggctgctt ctagaagtct gaccctgctg 2400
   gctcaaaacg actttagttc cttgcccaga ggctgcgggc tgcgggtcaa gacatcagta 2460
   gaaggagggc ccagccagag aggctgacat gggcttctac t 2501
<210> 60
   <211> 2501
   <212> DNA
   <213> Homo Sapiens
<400> 60
   cgggcaggaa taatcactgc ctcccatccc cttaaacatg ccaagatgct ttatccctag 60
   gatgaggtga cttactccag gtaactccta ttgcctaacc actgaccaat tactctgccc 120
   tttagtcttt atgtcattaa atctgcatta agaatttcat ggaataggcc cggcatggtg 180
   gctcatgcct gtaatcccag caccttggga gaccgaggtg ggaggatcac ttgaggtcag 240
   cagttcgaga ccagcctgga caacatggcg aaaccccatc tctactaaaa acacaaaata 300
   actagccagg tgtggtggtg ggcacctgta atcccagcta tttgggaagc tgaggcagca 360
   ggagaatcgc ttgaactggg gaggcagagg ttgcagtgag tcgagatcgt gccagtgcac 420
   tccagcctgg gcgacagagc gagactctgt ctcaaaaaaa aaaaaaaaaa aaactcaggg 480
   aatggatagc agcattgatg aatattgcgt ctggagagat cagatcactt gtcacttgtt 540
   tccaggcaca gggcttacca agaggcagat tccagattta aataattctg taacagcaaa 600
   gtccaagcta ttttcactgc tttggagaaa agacccagac ccagagcttg aacctcactt 660
   tgcagcaccc cagttctaat cttttaagtt tttttttttt tttttttttt tttctgctgg 720
   gcacggtggt tcatgcctat aatcccagca ctttgggaag ccgaggggga aggatcgctt 780
   gaggccagga gttcgaaacc agtctgggca acatggcaaa accccatctc tacaaaaaat 840
   acaaaaatta ggccagagtg gtggcgcgca cctgtagttc cagctacgtg agaggcggag 900
   gtgggagaat cgcttgaacc cgggaggcag aggttgcaat gagctcagat cccgccactg 960
   cactccaggt tgggcgacag agcgataccc tgtgtgaaac tttttttttt ttctccaacg 1020
   ggctttccag agaagtgtgt gtatgtgcgt gtgtgtgcgc gagcgtgctt gcttgggctt 1080
   aaactttctg tcgggccaca ctttcccaag tctttgcact ggctgtaggg tgggctttat 1140
   cctcgggacg tcctcctccc caagtccagc ctgcagctgg aagtcttcac tgatctccat 1200
   ctctcctccc tgatctccgt ctctcctccc tgcccgcctc aggactggga ggccgatctc 1260
   tctctctcgc cctcccctcc accagccttt tccagatgta tgtctgccaa agacccccca 1320
   gtgcagagga tgatgaatga agatcctcga gccagcccgg tgggaaagtt tcgtcgccta 1380
   caaaagcgag ggaaagggaa gggaagttgg gggtagggga aaagttagag ctgagaggct 1440
   ggggcgcgac gagtctggac accgggcggg gacccaagct ctctccgctc agccaataac 1500
   tgtgcctccc ttaggaaggc gtgaggaaat gctccaatca atccctgcac tcctcccttg 1560
   gaatttgggc tgtatttttt tatttactgc aaaccccaca atccacccag gggtttcccc 1620
   agtgtttgcc tccagcggtc ccggtgccca tttactagtg ctgctccctc tcttccgcaa 1680
   gactgcgctc cagtcccagc ctccttctcc gcgggtgcct cccaaaccgt tctatcattc 1740
   tcgggttcag ggaggcggaa tcgtgcctgc tctccggttc ctttaagagg cgtcggctcc 1800
   acccctctca gagtcgcggt ctgacgcgag atgacagcaa cgagttcggt atgtctatgc 1860
   aaataagcgc cctcttgtgg gccaatgggg agcggaggtg ccggaaccac ggaccaatgg 1920
   ggcgggggcg ctggggctca ccatataagg agcggcctcg ccataaaagg aaacattgta 1980
   tctctttata tggggggaag ggtcggggga tccctccgcc gccagcgcgt ggtcccggcc 2040
   ccctccaccc gccgtctcgg ccgcggccag cagcccctgc cccccggggg acgctgacgg 2100
   ccgcccggcg cgccgcccta gcagacggac agggggcgct gcgcgcggcc tggggcaacc 2160
   cgggccacag gggcaggaaa gtgagggccc aggtcggccc gggcgtgcag gggccccggg 2220
   ttcgcagcgg cggccgcggc agcgatagcg gcactagcag cagcgggagt gccgggttga 2280
   gccgggaagc cgatggcggc ggctgcggcg gctccgattc ctcgctgact gcccgtccgc 2340
   cctcctgcat cgagcgccat gttaccgacc caagctgggg ccgcggcggc tctgggccgg 2400
   ggctcggccc tggggggcag cctgaaccgg accccgacgg ggcggccggg cggcggcggc 2460
   gggacacgcg gggctaacgg gggccgggtc cccgggaatg g 2501
<210> 61
   <211> 2501
   <212> DNA
   <213> Homo Sapiens
<400> 61
   ggaaccctct gatagagagg gctgactgta tttattgaaa acaaaacaaa acaaaacaag 60
   ggttgtattg gtggacccat gcagctcaaa cccttgttgt tcccaggtca actgtatatc 120
   cagagcttat aggaaaatac ctctcccagt aaccctgctc accatttctc tcttaagcta 180
   ttattatgat tagccacggt ttgctattta aatttaaatt taaataaaaa tgtggccttt 240
   cagttatgct agccacattt aaagtgctca atagccatat gtggctaatg gttactattt 300
   cggacagcac atatttagaa cattcccatc atttcagaaa ttttcattgg gaacactctg 360
   cggaaaaagg gggccatcat aatgtgagtc catcttctgg aaaaatcctg ggaaggggac 420
   aaaggaggtc tgtttggcat tgtgtaatgg taatttggta tttaattttc aaaaatgttt 480
   acccaattcc tattcatcag ccaggtgtgg tggctcttgc ctgtaatccc agcactctgg 540
   gaggccgagg tgggaggact gctgcagccc aggagtttga gaccagcctg ggtaataata 600
   gggagatcct gtttctacaa aacaccaaaa acaaaacaac aactttgatg ttgtggagtc 660
   aggacagtcc tgggttaaaa cctttgctct ccttagctgt gtaaaccgtg ggtctcagct 720
   ttcttatctg ttaacggtag gtacttcttc ctagggctgt tttgaggatt aagtgaaagt 780
   ccaagattgt gtctggcaca cagtagcttc tcagcaaatg ttttcctcct atgtcaggga 840
   atggctcctt tatcccgttt tgggcccatg ggtggccctg aagggtgggt gctcaggtgt 900
   taagttctgt agatggcata tccttgggaa aagcaaggca attaaaaaca gtgagaggtt 960
   gctctggtta agttttctcc tataactttc cccatgggtc aattgggtag aatctgccat 1020
   tttcctaata cttactgatg gtagtggcat tcggaagcac aatagctgaa gccggagctc 1080
   tgagtggaga gaaaggtctg tttctcaggc ccaaaaagag gttacacacc catggctgtc 1140
   cagtttggtg gtgcaggccc tgaaatcaga ccaaactgga tttaaatccc caaacctata 1200
   ctctaagcta tgtgaccttg ggctagatac ttcacctctc tggccttatg aagtaggaat 1260
   aataataata ccgtctaggt tgttaggagt attaaatgag gtaaagcact gaaaacgttt 1320
   agggactgtg ttaaatcatt aaataaataa aaacggggat gaccttatcg gcttgacaca 1380
   ggggattaaa tgagataata tatgaagaca agtacacggc aaatgcttaa ttaatgttgc 1440
   ttatttttat gtctgcaaac tgacttaaag gggaggcctt taagaaagac agtggggcaa 1500
   tttgcgcgtt gatgcattgt aggagaaaat gtgcaggggg cccgttggga ccagagttca    1560
   accaggtaag cggcagaaaa ccacaaatac ctccaggcgt tcctggggca gcgccgcctc    1620
   cccaaaatca cgcaaaactt ggtttgctaa gaattgtcag ctcttctaaa ggaggcgctt    1680
   cacgcatctc agtctgtgaa atgggaccca ggacccaggt agaggtgcgt tctcggcctg    1740
   gggaccgagt attttgtgcg ctccggtaac gcaggaagac agcgccactg acactctaga    1800
   gaccagcggg caccgcctgg aggcgccttc accacttggc ggttccgggt ccgcgcccca    1860
   ccgcgccaca agactcacgc ccgaaccacg tgatcagggc cgtggctccg ccccgctccc    1920
   gcgccgcgcg ccgcttccgg taggggcgga aagcggaagt gtgggagggt ctgcggggcg    1980
   ggctcaggag gtccgcggga ggatggagca gtgagcgggt ctgggcggct gctggcagcg    2040
   ccatggagac ggtacagctg aggaacccgc cgcgccggtg aggggccact ggctaagagg    2100
   acgggcatgg ggtcagggga agaaaaggcg ggaactggtt gaggggatac acctgtgtgg    2160
   gagtccccgg agctaagcga cccagccgat ggggcacctg ctgagtgagg ggggggacgt     2220
   ctggtgggtg agggtccggc tgaggggagc atctgctaag gaggttagac ttgggaccgg    2280
   ttagagggag cactcgctgt ggtgagactg tgctgaggaa cgtggggaca agttagggag    2340
   agtacctgct gaggccgggc cactcggggg aacgctatcc aagcagggac tcacggaggt    2400
   gggggcgaat gctgaagcag ggtgagaatc tgtgagggat ctctttaagg gggtggatcg    2460
   agaactggcc aagaggaagg ccgggtggac tttctaaggg t                         2501
<210> 62
   <211> 2501
   <212> DNA
   <213> Homo Sapiens
<400> 62
   gcatggtggc tcacgcctgt aatcccagca ttttgggagg ccaaggcagg cagatcacga      60
   ggtcaggaga tcgagaccat cctggcgaac acggtgaaac cccgtctcta ctaaaaatac     120
   aaaaaattag ccgggcatgg tggcgggcgc ctatagtccc agctactcgg gaggctgagg     180
   caggagaatg gcgtgagccc aggaggcaga gcttgcggtg agctgagatg atcgggccac     240
   tgtactccag cctgggcaac agagtgaggc tccgtctcaa aaaaaaaaaa aattactaca     300
   tgatactaag taatgcggaa ggtgactcaa agggggaaag gaacacagca gtgtaaagga     360
   aggaggttgt agatggatct agaatttccc cctcatttcc atcaggtgaa agcctgagaa     420
   aactgcaatc tttgtgcagg ctgggtttgc tttgtacaca ctggtcccct agtgttcatc     480
   tccaataatg ctgacaactc tgaaaaccat ctgtagacat tctgcaggct ccatctcagg     540
   aacaatggct attttttcgg gtagttgaag caaaattaag tccaatgata agcaaatata     600
   accattatca aaatcttcca tttatgtttg ttaaagcaac ctaagtatga tctgagaagg     660
   actctgtatt ctatatttga gtccttgtgg atgaactgta acctagctta ataggcagac 720
   aagattgaaa acctaattta ggagtatgtg cctttaacaa tagctgagtc ttggccaatc 780
   ccagtggcca tacttcaacc attcatacac tgctgagtgt tcaaactgtg ttcaaagaag 840
   gcaaaagcca acctgtaacc aatccagttg tttctctgcc ttacctccaa tttctgtatg 900
   tcacttccct ttttttgtct ataaatatgt tctgaccatg aggcatccct ggagtctctg 960
   aatccgctgt gattctggaa gctgccccat tcgcaaatca ttcattactc aattaaactg 1020
   ctttaaattt aattctgctg aagttttctt ttaacaggtt tagaaaaaat aatggcaaaa 1080
   atgaatgaaa atccaataac cctggaagca gaaaaggctg ggggctccaa taagtgtaaa 1140
   tagtcccatc cctatatttt ctccatggca attacaatcc agcacattat atatatattt 1200
   ttttgcttct cgcattttgg cttagggtaa agctttttaa aacaggcact gccaaccagt 1260
   gttatcaaga aggtctggat gccgttttgt gggaacattt taaagaggaa tgtccaaaag 1320
   gaaaaggggg atgggttggg agaagggtat caggcgggta tctcaaaacc attcttaggg 1380
   ctataggttt aatttatttg gttgtggacg tcagagccgt catggtaaga aggaagcaaa 1440
   gccttttgta ataattaaag ccttcagaag cagcgtgccc cattgcccac tagtgcgccg 1500
   tgaagtctgg tgttcaccta cagggtccct ctcagcactg cccaggcctc ccgagtgctc 1560
   cagcacagta gcttggagct tgttggtttg gtgaccaaga tacactccag ggaatatgcc 1620
   atgcagtgga gtctcttccc cggcactgca tagcaaaagg aaagggccgc tgggtgtctg 1680
   tgggtcctgg gcagtcacag aagccaccgc gctggcgggg aggaggggga ccgatgcggt 1740
   ccatgtcccg ggcagcccca ctttctctgc ctgcgaaggg cccttgtccg gcgggaggag 1800
   agaggcgcgc cccacccggg ctcctctaca cctgccgccg cctgggccga ttccgcgggc 1860
   ctcgcccggc gcttcagccg attcccgccc agctccgggc tcatgggcgc ggtcagcagg 1920
   gcgggccagg gcggcggggc gcgacactgg gaggaagtgc gggccgcctg cccgggcgcg 1980
   ttaaggaagt tgcccaaaat gaggaagagc cgcgggcccg gcggctgagg ccaccccggc 2040
   ggcggctgga gagcgaggag gagcgggtgg ccccgcgctg cgcccgccct cgcctcacct 2100
   ggcgcaggta ggtgtggccg cgtcccctac ccggccggga ctttctggta aggagaggag 2160
   gttacgggga acgacgcgct gctttcatgc cctttcttgt tctaccttca tcggccgagg 2220
   taaaagtgct gaaaccatgt gaataaaata caggtgggtt ccgccagctt cgctcctgaa 2280
   cctacccgcg ctcgggatcc agaagctgcg ccgggagaga ggggctcagg cctgggcgga 2340
   ggggacggag gtcagaccgt gcggaaagtg acccgggcac cccagggcgc ccaggccccc 2400
   agggagcgcg gaaagtgcgg tcgcggcccg gccctcggga gacgcgggat tgggatcagg 2460
   cacagcgcga ggaagtcgat cttggagcta gaacattttc c 2501
<210> 63
   <211> 2501
   <212> DNA
   <213> Homo Sapiens
<400> 63
   cccaaaagat acaaaggggt ataaggtgaa aaattattct aacccatccc tcagtgacct      60
   agttcccttc ctctgaggtg accaatttct tgtgtatctt tcctgagata atctatacat     120
   atagcaccat atacaagcaa atgaaatatg ttttatttat ttttttgaga ctgggtctca     180
   ctctatcacc caggctggag tgcagtgaca ccatcttggc tctccgcaac ctctgcctcc     240
   tgggctcagg tgatcctccc accttaacct ccagagtagc tgggactaca cgctcacacc     300
   accacaccca cctaattttt gtttttttgt agagacgggg tttcaccatg ttgcccaggc     360
   tggtctcaaa ctcctgagtt caagtgatct gcccacctcg gcctcccaaa gtgctgagat     420
   tacaggcgtg agcctccacg cccggcccca aaatctgttt taaaagcaga catttcttgg     480
   tgattctaat aaagggggtt ctcagacata tttggaaaaa tatatcccta cttttatgcc     540
   agaccctgtg ctgggtcccc gggctgtgtg acctgacact gcacagtcct gcttagaatg     600
   cttaaagaga gttaataagg taccaccttc tatgccatag gcggggagca aaggggctcc     660
   agtgggccct gcctaggagg cctgaagcta gagctgctga gggcagggct gtgctgcaaa     720
   gaaaatgtct gagagctgca ggcgtttcat cttctgtcat cagctgtggc acctggcaga     780
   cactggatag gcttgtagac aaagacctgg taactcaagg agctgcttgg ccttcctgcc     840
   cagtcccatc ccagaggcac tgtacatctc tggtttcttc agggggccct gtgtggaagt     900
   atcttttgtc ttcctggtgt cagggatatc atcacgtgcc tgttggctag gcgagcccgg     960
   cgcccagtct cctaggatgg ggagagtaat gttcccgagc agaacagggt ggggctttca    1020
   gactactccc tttcctttac agctggcttc attccatcga cctcatcaaa gccttcctgg    1080
   gagcacccta gagaagagtt acgtccaggc cgggccctgg ctgcctggtt cacggcggaa    1140
   tccccagcac cacgcctcgc acgtcgggct caaagcatgt ttagtgaagg agtaggtacc    1200
   tactgctaga tggagccatc tctctagact tggggtttcc ctataacgat ggctatgttt    1260
   ggcatggaag cctctttaga agtcaatagt aggaaataag ggctaacagc acctaattgt    1320
   ggagtaaggt tcaaatccta gctctgccac ttaaccgttc cgaacctgtt ccctcactgc    1380
   agaggcgaaa aggctaacac tatttcacct cggagggtta ccgtggagaa tggaagctgg    1440
   acaagctgta tcagttcagt agtaaaacac acacacacaa gcgccccacc cccaccccac    1500
   cccaccccag gaatgaacac acacacccgc gcgcgcacat acacctcagg aatgaacaca    1560
   cgcgcgtaca cacacacgca gcccccccca ggagtgaaca cacacacaca cgccccgttc    1620
   tgttgttccc aggaacacac acagagacgc acacactcgc ccggttttgt tttttccagg    1680
   ctttttaact ggggtctttc actcggctta gggcaccgct gcctgaaaga cctttctagg    1740
   ccagtcgggg tccggcaccc agttgacgag acagcgcggc gctttcagag ctggggagag    1800
   gcgaaaactc ttccggcccc ccgatccccc ggccagccgc ccccggcagc tccttgccgc    1860
   ctcccggcct gggcccgccc agccgttctc ggcctgccgt caggcgatct cggcggccag    1920
   cccagccgcg atgtgacgcc gcgcgccccg gggtcctcgg cgcctgcgcc ctctcctata    1980
   aagcagacgc cgcgccgcgc tgcgacgctg tagtggcttc gtcttcggtt tttctcttcc    2040
   ttcgctaacg cctcccggct ctcgtcagcc tcccgccggc cgtctcctta acaccgaaca    2100
   ccgtgagtag ccgcccactg aactggaaag ggtcgtggct accggattgc gtgccggctg    2160
   gcctcaccgc tgcggtttgg gcctgcccgc ggcgggcggt gactgggcct ggccttcttt    2220
   cgggcccggt ggatcgcgtt gtcgaccctg ttcttcggga gacactacca ggttccgttc    2280
   acctgccccg cccccgactc agcgaggcct cctctggccg ggcgtcctca cggcgctcca    2340
   taagtgagcc gaaccccggg ctgggccttc tctgcaccgg ccgagcgtca gccggcgcgg    2400
   agctcggctg caaggcccag gctgcggccg ggggcctctc ttggtcttaa gcctgctgtc    2460
   ccggggacca gggcgggggt ggcggcgggg ttgtgaatgg g                        2501
<210> 64
   <211> 2501
   <212> DNA
   <213> Homo Sapiens
<400> 64
   gatctgacag gttaaaggtg tacacttatt ttctctgtaa gaagcgtcat ctggtaagat      60
   gatcaagaat ggtgcaaagc aggatgggga gtttaaaatt gtttccaaat gtgggaatgt     120
   aaatgaatat aaacatgtaa gattttaata taccaaactg atcagattct gtgtaatttc     180
   caagtttctt ttttctttca aaactcctct gaaatctgac tgtccacaaa aacttacttt     240
   atagaatttt atgtgattta tttactcaga tattatactg acctcacatc cagtagtgaa     300
   aacagatttt attgtagaat ctggaaagat agagggccat ataggttgta ttttcagttt     360
   tgtttatact aacacgtgtt tacaacccag tttaatttac accctgtatt gtattattgt     420
   tgtcatatct ctgtatgcat gtaagtataa tatgtgttgg caaaggaaaa ttttgagtaa     480
   gaagaagctc tctgatctat ttgattcaat atgtatttga gtgtctaaca gacactgttt     540
   tagacactgg tgatacaaca ctgaacggag caccaaatac tttacagcgt ctcctggagc     600
   tgttgtcaag acatactttc caaggggaat atttcagaat aggtgataac tagtcaacga     660
   aggaaaagta ccttagtcat ctaggagagt tgtacttaga gtgaactgaa ataaactaag     720
   ctcacgaaag acagagattt tttgtttggc ttttgtctgt tgcattcact actgtatctc     780
   cagggcccaa aatagtgctc ggctcataat aagtattcag caaatatatg ttgttgattg     840
   gagtgtttgt tttgaatttc tgtaatcaaa cacatacctt ggtaaattat ctttacatct     900
   tgctagttga aaattttatc tcagttgctt tgtttttaat gttaccttgc tttttgtttc     960
   tacttgtgcc atacatcagg atgctggaaa agcttattaa tattgacagt catatggtta    1020
   tctgatattg aaaagaatag atttggaaag gaacctaaga ggtcatcttt tgttcagctt    1080
   cctgcctagg aaaactaagt aagatgatta ggtatgtata tttaattagt catttaaaaa    1140
   aaaaccagga caacataatt gagttccctc ttgagaaaat ggagaaaggt acttaaccct 1200
   agctataaag ggactaacct ggaaatttta gaacttctgt gtgggaaagt ggaaaaaaaa 1260
   aaaaagcaca actaagctgc tctttgttga tatcagaaat gggcctgtca ttcattttgg 1320
   cattgaagca tagcctccta tctcggggca ggactgggac atttttttcc tcccacaaga 1380
   gctggacagt tattacaggt tcaaaaagcc ccgaccagtt tttcaagagt ttctcctcct 1440
   cttttccccc tgaaactcgt ggtgcttttg ctctgctttc aagatgcatt aagtctcctg 1500
   ctttgtgact gctttggagc cagcagatac tctgatatgt ataattcaaa ttatgcaggt 1560
   ttcacgagta agtttaatct tattttttaa gttagttaaa aggcaagtga tatttagaaa 1620
   aatgttaact tgtagttatt tcaccctttt tactttaagc atttttattg cttctcggcc 1680
   ttttggctaa gatcaagtgt gtactttaag cattttttaa aataaaaata tccttttaat 1740
   ttaataagaa aacaaggttc tacatagaaa agccccttca tctaagacct gcacttttca 1800
   atttcttttg agatgtcttt gttgtaaaca gtattcatat gtcttttgaa agccagttaa 1860
   ctaaacagtt ttcttgagca tctttttagt tttactgaga agtattttaa attgagcttt 1920
   tctgagctcg attgcttacg tctgacacag tctcaagttt ccactgaatg gtaacaaaga 1980
   ctgtagaatg ttgttggtac tgcagtgaga ggcatgcttc cttagaccag gtaagagaga 2040
   tcagtttgtt tctcactgct gggtgagttt ttacagctct tattttatat tctttaagca 2100
   gcagcaatat taaattgata aatagccagg agcacgctga tttcaagacg tccttgcttg 2160
   ttgcagacag aaaaactaca gggttatgta tgggggttgg ggtggggggg gaggggaaga 2220
   attagtttat tactcagtta cttatataaa ttaattaaaa tgtgaaaata attctggagc 2280
   tcagttttct taattcagga actaaagcag cagttgagga aatcagtaat tttaaaggta 2340
   cttcatggtt attacttgtg aaagcaattc aaaggatagt ttttactttc atttttttcc 2400
   ccagtagtta ataaaataag ctttgccctt aactaaacat tttttccact tacgaaaact 2460
   tttaaattgc caacagcaaa atatacttcc caaggatcct t 2501
<210> 65
   <211> 2501
   <212> DNA
   <213> Homo Sapiens
<400> 65
   cacaagtcaa gaccgctccc tgcttcttag cccgctgggg agccaggcca gcaggcccca 60
   cattcctgag gaagggacag ggttctggcc tggagggtct agcagaagcc accccagggg 120
   agggcccgac aggaaggaag gtaggcctgc cggaggggca tacaggagct tcctctcccg 180
   ccacagtgtc cagggccaac tgctccagcc ctcaggctgg gtcaacagga tgggacagcc 240
   caggcggaag gaaacctgtg gggagggaca ccccgcagac agaagcaggg acatggggtg 300
   gggagaggca ggaagagctg ccgggctgct gagctggcgc ctctccagca gactcaggag 360
   gggcggtgac aggaggccat tccctcctca tccccgcagc cctgggcctc tctggtcctg 420
   gccaacagta ttactatcat tattattgct gttgttcgct agcctgggcc ttagatacat 480
   tagaaaaaaa ccatcggaag atacgcatag cattggcagt ttctaaaaga attaattccc 540
   ttcctgtgtt cattctgtga ttactgggat agaaatgcta tttgcattac cagcctttca 600
   ttcagttaca gagacgtgag tgctcgaagg agagacagtg atttttgcct taaattcagc 660
   ctgtccaaat cggataagat ctccgatttg ctttaagccc cgttatcact gccttcctct 720
   ccaacaacag ctgctgtgat cacgcacaaa cggccaaacg gggcaaatcc cgtgccaaag 780
   cagggccatg ggctttcctg atcagaaggc ctagccccag cccccaggcg cagcacacgg 840
   gcggcttcct ttcagaaacc cagcctgcct cccaccagct ggagtgggtg ggtggggcgg 900
   tagtggtgcc agtttcaggg aacggccggc aaacccacct ccaggcgtgc tccagcggga 960
   gcctggagac cctaggagag ccctccccac aagcggcttc caggcaggac gcttccagag 1020
   gtcttggtcc aggggtgggg gtgaggtggg gtctaccttt gaaacagcta caatttaaac 1080
   ttcagctaca ccgagctcaa actcgattcc gcagccgagt gtcggcgcca gagaaggata 1140
   aaaactcggg tctacggctc cccaccacgc ccctggtccg gtcctctggg cttccaggag 1200
   tcctcacgcc atcctctggg ttgcccagga ggaaggatgg gcggggcggg caggcgctgc 1260
   gggcgctgca gatggggagg gcgagcccgc ggcacggcgt gagcggggga gaggcgcgcg 1320
   agcaggtgtc ggctccgtga cagggtcccc catcccgcgc cccagtgctc cccgaggctt 1380
   agtgaggcaa aacccagcaa atgcttcaga aatgcagctc agtcggtcac cgggttctgc 1440
   ttcctcatca gacgcgcaag aggatggcgc ttccaatgca aatctcttgg ctccggcccc 1500
   ttggctggca gccgccgcgt cccccgcctg cctggcgtcc cgcccactcc gtggcgggct 1560
   gagacgaggc ccggcgcgga ggggacgggg cggagcgggc atccctcccc accccccacg 1620
   tggggctggc cctccgcagt gcctgggcgc gctgcagtcg ccgcgcctcc ccggccgcgg 1680
   caccgcctct ctaggcaggg gcgggggacg aggggcaagg agtgggcgag gggtgggcga 1740
   ggggcggggg gcgtcactca atcaggtggc ctctggagtt ccccggggca gggcagaggg 1800
   aacacgctgc cggggattgt gtacacgctc cactgacacc agcttcacgc tgccgggcag 1860
   tcgccgatca cgcgtggccc cgcgagccca ttggccggcg cctcacacac ctttgccgtt 1920
   gattggccgg cctcaggctc cgcccccacc cccgcccgcg gcgcggggca ggctgagcgg 1980
   ctacctgaat ggggaggggg cagacggcgc tgagcgcggc ggcggcggga gcggcgtcga 2040
   gtgtctccgt gcgcccgtct gtggccaagc agccagcagc ctagcagcca gtcagcttgc 2100
   cgccggcggc caagcagcca accatgctca acttcggtgc ctctctccag cagactgcgg 2160
   taagtcattt ggggatgccc ctgtgcttcc tcgcctggtc ttgtctgggg ggccaaaggg 2220
   ggcgcgaacc ccgagccccg gacatcagcc atgcctgaga attggggctg cagcggagtc 2280
   gtggggaagg aaagggcttc ctgcctgcag actatgggca ttagtgaggg cgtgtgtgtt 2340
   ggggaggggg tcgaaccagg gggctgggat cttcagacag ggacaggggt cttgctctag 2400
   atgtactgag gggaagggac aactccgcat ggagacccga gagggctggt gaggaggagg 2460
   atgacgagcg ggggaggagt ggggaggggg ccgttgccct g 2501
<210> 66
   <211> 2501
   <212> DNA
   <213> Homo Sapiens
<400> 66
   ggggctgtag aaatggcggc cccatctccc aacaacttgg gcattgtgaa tatcacctcc 60
   ttaaagggga tctcctttgg tcatcccgtc tagagcagcc accataactt ctgagcgttt 120
   attgctagct gatatatatc agaaaaatac aaattccaca aaagcaggga ctggtctgct 180
   tctctccctg cagggcccag gttctggcac atagttggtg cagaaagtgt gcagcctcag 240
   gtcctatcca agcccccagg gcatcacact cgggacttgt tctgcatatt tttacttttg 300
   cctcccactg gtactagttc ttccgtggaa cagcctgagt cccttcagat acttaatgtt 360
   ttttctcaag tgctgccatg aagccagatc tccaccgtct tggggcattc ctttttaggg 420
   atgggaagta tatgtcgctc cttttatgtg atttacattc tatcttggat aatttggcca 480
   tcaccgtagt tcattcagat ctgtttggat cctgcccatc tcagcttcag tccatttcat 540
   tcttttaaat ctgatcgaca gttacctcca acagcttcat cacaaatcac tcacaaaaat 600
   ggccttaatc ctgaagttta tttacggaga gcacacttgc taggtgtgtg gcagatatac 660
   aggaagcaca agatgaggca gcagatctag aggcaaatga cttccttctc cctgcctagt 720
   ggtgactgcc agcatcacgc cctcccggga gaggtgagaa acccctccac gcaagcactg 780
   gaaccttcac agtcaagagt ggcaacagct ccggttactg gacttgggcc tgttgaattc 840
   taatactctg tgactccaca tctgggctga atttttgctg agtatgatgg aatttacatg 900
   cttcctccct agcccctact tgtctgtata gttggaatat ttggttgcct cctctggagg 960
   gatctagtac gtttagagtc tagacgctgg aactgtcaaa gttcagagga aagagctcca 1020
   gctgcaaagc aagagaaatg ggctggaatt ctagcttcac cccttaatga atgcttctga 1080
   tttttttttt tttttttttt ttgagacgta gtctcactct atcgcccagg ctggattgca 1140
   gtggccacga tctcagctca ctgcaacctc cgcctcccag actcaagcga ttctcgtgcc 1200
   tgagcctcct gagtagctgg gattacaggc gtgcgctacc acgcccggct aatttttgta 1260
   tttttagtag agacagtttt tggccatgtt ggtcaggctg gtcttgaact catgacctca 1320
   agtgatctac cttcctcggc ctccgaaagt gctgggatta caggcccgag ccaccgcgcc 1380
   cagccgcttc tgatcattaa aaaaaaattt tttttttggc ggggggaacg aagtgtccct 1440
   ctgttgctca ggctggagtg cagtgcagtg atctcggctc actgcaatct ctgcctccca 1500
   ggttcaagcg attttcctgc ctcagcctcc tgagtagctg ggaatacggg tgccccccac 1560
   cacacccagc taatttttgc atttttagta gcgatggggt ttcgccatgt tggccaaggc 1620
   tggtctcgaa cttctggcct caggtgatct gccttccttg gcctcccaaa gtgctgggat   1680
   tacaggcgtg agccaccgtg cctggccaaa aaatttatgt tttaaaaaga ctagtcaagt   1740
   gcagtagtga gaagggggga aagagtagag caaggagtta tatctgttgc ttctgaccat   1800
   tttgaacaag ttacctaatt ctctgaggac aagctcggag aatgggagag acagttatct   1860
   atttgcaggg ttgttgggag gaataagtga catcatgagt gtgtgccagg tgtctgatta   1920
   cagaaggtgt tcaattaatc tgcaatcatt aattaaccct tcagtcgctg gtattatttg   1980
   ccatccatcc tccgagtgtt gccaagttat gggtgcgttc tgccagcgtc ctagcagtgg   2040
   taaggcttct ggctgccagc ggcgaacctc tcccttcgag tatttctcct cttgctgaga   2100
   tgaaatgcga ccgggtctct ttaagggcca ggcgccggga tccaggcggc gcccaacggc   2160
   tggactagca gtcgtccgcg ccgactcgca caagaaggaa ccccgggcct ctggatccgc   2220
   tcgcccggct atgctgctgt ggccgctgcg gggctgggcc gcccgggcgc tgcgctgctt   2280
   tgggccggga agtcgcggga gcccggcctc aggccccggg ccgcggaggg tgcagcgccg   2340
   ggcctggcct cccggtaacg cgcgtcttgg tcccgcctcc caggagcccc tatgcgccca   2400
   cctactcccg gcccctcggc ttccggaacc cgcccgagcc cgaagcgcct cttccgaggc   2460
   gcgggatttc ctccccggct gcggctggga cgggggcggc c                       2501
<210> 67
   <211> 2501
   <212> DNA
   <213> Homo Sapiens
<400> 67
   atggtctcga tttcctgacc tcatgatccg cccacctcgg cctcccaaag tgctgggatt     60
   acaggcgtga gccactgtgc ccggcctcta tcagcatttt ctttcttttt ctttttcttt    120
   tttttttttt gagacagagt ttagctcttg ttgcccaggc tgaagggcaa tggtgtgatc    180
   tcggctcact gcaacttctg cctcccaagt tcaagcgatt ctcctgcctc agcctcctga    240
   atagctggga ttacaggtgc ccaccaccat gcccagctaa tttttgcatt tttagtagag    300
   acagggtttc accatgttgg ccagtctggt cttgaactcc tgacctcagg tgatccgccc    360
   gcctccacct cccaaagtgc tgggattaca ggtgtgaaag agaccattcc cgatctcttt    420
   cagcattttc atactgaatg tccacagctg ccctgtgagg aggcttttta cccatatttt    480
   ctgactcaga gagaagcagc cacatgtccc ttggccatgg cagttaagac caactccatg    540
   gagctgggtg tcttagctca catctgtaat cccagcactt tggaaagcca aggcaggatg    600
   attgcttgag gccagaagtt caagaccagc ctgggcaaca tagccagacc ccatctctac    660
   aaaaatttaa aaattagcca caaaatttaa aaattaacaa caaaagggcc gggtgcggtg    720
   gctcacgcct gtaatcccag cgctttggga gggtggatca cgaggtcagg agttcgagac    780
   cagcctggcc aagatggtga aatcccatct ctactaaaaa tacaaaaatt agccgggcgt     840
   ggtggcgggc gcctgttgtc ccagctaccc aggaggctga ggcaggagaa tcgcttgaat     900
   ccgggagtct gaggttgcag tgagccgaga tcgcagcatt gcactccagc ctgggcgaca     960
   agagcgaaac tccatcttaa aaaaaaaaaa aaaaaaaagt ggaagatgag gaagttgatc    1020
   agacatcaag gatgagcgga tgacttaata ggcttctttg ctaagacttg gctgggcagg    1080
   tgaaagacaa agtcgaggag tggttatggt gtggcacaga agaagggtca gaggacggtc    1140
   tttgttacct cttcatgcct gagtttcttc ctctgtgaaa tggggataat aagagccgcc    1200
   atacagggaa ttgctgctag gatcaaatga gataatgtat gtgaaacgct ctggctgtag    1260
   gcttctcagc aaatgggcac gacttgcgga gtggggattt gaattcacgt ctggcgggat    1320
   gtccaagctg ctaccctgac cgctagggag cttcagagga cagggctgca ggtgatcagg    1380
   aagaggactg gggcaggtgg gcgaggaatg cctcccagga gtgaaggagg gggaattcta    1440
   gtcagcagga tggagtcggc caggtagaaa cgagggaaag gagacaggac cggatggaac    1500
   ggggaagcca aagggcaggg cgtcggaggg ttgaatggtg gccggtgcag ctttgaacac    1560
   cgaggtgagg acatgcagct gtgtcctagg gtcaggaccg tacacgcctg acccaattcc    1620
   acagcacgga ggggaactcc aggatccggc cgcgttgccc acacacttcg ctctccctcc    1680
   cgcctctcgc aagcccctcc cccgtctccg tccaccgagt gccagccaat agcagaagcg    1740
   acagcgcatc tgggtgccga ctcagccaat cgcggctgag tgacgaatga gccccaggac    1800
   caatgagagt gccgccacca tggcaaaaaa aaaaaaatcc aatggtgacg agcagggaga    1860
   acagagcagc tgccaatggg cgtgtgcgtt tcaggcggcc aatgggagga ggcgtctcgg    1920
   cgggggacaa gcagtagcta cccgcgggag cggggagggg tccgggttcg agcttgtgtt    1980
   cccccggaag ggtgagtctg gacgcgggcg cggaaggagc gcggccggag gtcctcagga    2040
   agaagccgcg gggactggct gcgcttgaca ggctgcactt ggatgggagc acctggtgcc    2100
   tcgggactgc tccgatgccc ggtgggtgca catcccagtt cccgccgttg ccggccgggt    2160
   ttagaggttt tggggggagg acatgggggc gtgcagcctt cccagttgca aacttcactc    2220
   cgaccctgtc ttcaaagctg ggtctgggtc cagtggggac gagaaaggag gaaggaggaa    2280
   gtaggctccg cgaaagcccc atccccggga tctcatctat aacatgaata ggtattaatg    2340
   gcaaaggcta attaagcgct tactgtatac caggcacttt ctctgcctcc tcgcgttaaa    2400
   tcctcccagc agccttttga ggtagacact gttacatgcc cattttccag atgaggaaac    2460
   cagcaacatg ggtggaagtg acagcccctc cacttccata c                        2501
<210> 68
   <211> 2455
   <212> DNA
   <213> Homo Sapiens
<400> 68
   ggagtgcaag aacacagaac taaaacagag cttgaaactt aaagaaaggg agagacttgg 60
   gggaggagtg gggtggagtg acgtgatgtg ctgctggaaa ccagcagttg gtggtttcct 120
   cttgtgcttc ctcttctgtg ggttttctcc tgcttgtggg agggcctttt tctctcctcc 180
   cgacagaaag gctatctttg gtgttcgttc ccttgaactg taacatcctg taagggtatg 240
   attccatgcc tctgtgtggg tgtgaattcc ctcatggtga ccctcaaaat ctgcacacag 300
   gaccccttcc cattgagggg aggggatcaa aacaactcta cttctcaggg tcctctcctg 360
   ttccaactgg tctgtgtcca agagaagcct taggtaaatg gggccagctt gaagatcaaa 420
   caggtttggc agcctctccc ggcctctctt ttctctccta cagctttata gctacagctg 480
   ccttgatatc aatattgact ttggctggct ggcatgacta cccacagggt atcgtgcctt 540
   aatttaccag gtgacaggca acgctgccct ctcctggaac catccagcag agccagggct 600
   gtacccccaa atcctgcaac agaggtttcc ctccatctca cctccctgtc cctgcatttc 660
   tcctatctca gtagctcctc tttccctctc tgggcttctc tttccactcc ctccccttcc 720
   tgggcttggt aaactagtcc ctaatctctt cacaccccag attggaaggt gggtccctcc 780
   ctgacactcc ccagagctgt caccaacctc ctccaagttt ctatagctcc attgctcaac 840
   agatttgcca ggggtaacca ttaacccagc ccttaactct gttcccccac ctttcttgct 900
   ggaggggatt ttccaattac tggttagcac agctaggtca tctcaccccc accatctttc 960
   ctaacttctt gggttggggg gctggggagg aatctcccca tctcagggta ctaggaacaa 1020
   agctggggag gatggtgcat ttaaagggat tatatatata tatatatata ttttttttct 1080
   ttctccctca taaccccacc cccgcaacac acacacacac acacacacac acacacacac 1140
   acacacacac agacgcacaa ataagcttta tggagcagtg acttcattat gttcaccgct 1200
   ttgagtccaa cccctggccc aaaataggca ctaaatagtt gccgaatgca tgaatgatag 1260
   atacctctct gtcttcaggg gtgtgtagaa gtgcgaaggg gtatgggcat gtcccagtag 1320
   gggtgtgagt gttctgatca gaactacttc tctctgccag aatttgatgt aattcgaatg 1380
   cttccacctc tgcttgaagg gtttaaataa taaattaggc cctgtcgtgc cattatgggg 1440
   gtggtcatac cctgtaccca ggaaacaggc acggtagggc tgagacagaa gtcctgcttg 1500
   tttccgctta tttatttgaa acaccgctca tttaggtctt actttgtttg ccaggcactg 1560
   ttctaagctc tgtataaata ttaactcaga gggtacaaat attaacttaa gagttgttgc 1620
   aggaaaaaaa ataagcgcct ctggctcttt aagtttggcc tccccctcaa aacccccgca    1680
   acggtcccaa accccttcca gggactggga ctacggaccc tggtccgacc ttctcgcggg    1740
   cttcccactg cgccaatcaa atcccagaaa cagtgagtgc tagaggcccg gctgctaagc 1800
   aacggcagag ggcgggaagt ttgaacgttc tggacccgcc ccgaaggcaa ataggccaat 1860
   cagcgtccag actcttcagc tacggcagtc cgcttctcct cctcgccctg tcggatctct 1920
   aggctggatc cgggcctctc caatcaacag cggctaggag ggcggggcgc gtgcgcgcgc 1980
   acctcgctca cgcgccggcg cgctcctttt gcaggctcgt ggcggtcggt cagcggggcg 2040
   ttctcccacc tgtagcgact caggttactg aaaaggcggg aaaacgctgc gatggcggca 2100
   gctgggggag gaggaagata agcgcgtgag gctggggtcc tggcgcgtgg ttggcagagg    2160
   cagagacata agacgtgcac gactcgcccc acagggccct cagacccctt ccttccaaag    2220
   ggtaacctcc gcgtgacagg aatgagggtg gggcgcgtgg agtttcccac aatctgtact    2280
   ttagttaaat acccgagaat tcacctcctg tgtccacagc tctccacgcc cctcagccct    2340
   gccccgcagc cctgtagcag aagtacttag tgctttgcat tctgcgcgcc accctacccc    2400
   ggcctcctct gtgaatcgtt gcttccgaac cgccctcact ttttgcatcc gcaga         2455
<210> 69
   <211> 2625
   <212> DNA
   <213> Homo Sapiens
<400> 69
   ttttaaacga gaagtgatgt ttccggagca ttaaaactga agtgatttca aaaccatgtt      60
   gcactcacac gaacaggtgt gcacttaatg gactaaacta gttcagctga catgtcttct     120
   tcattaggaa cagtgtggag actgaaaaac taatttagcc tagagcagct atttaattgt     180
   aaagtctcct ttctcaaata ttgatttact atgtgaggaa atatttactt tgtatagaag     240
   tgtgtggaat tggacgaggg ggttgaccta cacatgtggt ttggtataca catatcctca     300
   ttacagaggg tgtaatgaag atataggtgg ttcagcacca taggaaaggg aaaaaagaaa     360
   aaaaaaagac ggtagaggtg gcctcccaag catccactcc cactcctctt gttaatgatt     420
   cacaatttgt tgttattgtt gtcatttact gttctccaca cctttccaca aggcctgtgt     480
   gctttgaaaa aatatgtctc tactccggat agaagtgggg cacacagggc caggcgcggt     540
   ggctcacgcc tgtaatccca gcactttggg aggccgaggc aggcagatca caaggtcagg     600
   agttcgagat cagcctggcc aatatggtga aaccccatct ctactaaaaa tacaaaaatt     660
   agcctggcgt ggtggcacgt gcctgtagtc ccagatactt gggaggctga ggcagaagaa     720
   tcacttgaac ccgggaggca gaggttgcag tgagccgaga tggtaccact gcactccagc     780
   ctgggcgaga gtgcaatgag actccgtctc caaaaaaaaa aaaaaaaaga aaaaaagaaa     840
   agtaagtggg gcacacgatt caggcctaag ctaaccagac caacctcatt cctgatggtt     900
   gttaatgttt cagatacggg cccgcagccc tacgtagaga agaggccaag gtagaaaaca     960
   tgaatctgag gtaaaaagaa atgaggtact tgtttgcctc atcaagcctc tcaattaaac    1020
   taaccttgaa gcctgtctta cctttggact tctagtgatg tcacccggta aagcccattt    1080
   gtttcaggac gtaagagttg ggttttctgt gacttggaac caaaaccatt ccaatttaca    1140
   aaatgagcaa ctttaatatt acccatgaga aatacttcat tggtatatgc tctttcctag    1200
   cgtttttgaa aactaaacta ggtgggtgaa aagtatatct ttgcatgaaa ctttttcatt    1260
   ccagaaaaca ttttgtcatc ttgataataa tggccaatgc tactatatcc aaatttttgt    1320
   cttttttttt ttttgagaca gagtctcgct ctgccgctca ggtgtgatgg cgcgatctcg 1380
   gctcactgca acctctgcct ccctggttca agcgattctc ctgcctcagc ctccctgagt 1440
   agctgggatt acaggcatgc gccaccacac ctggctaatt tttgtatttt tactgtagac 1500
   ggggtttcac cattttggcc aggctggtct cgaactcccg acttccagtg atcctcctgc 1560
   ctacctcaaa aagcaacttg ataaatccac aggctcggta tattttaaaa attcttttaa 1620
   atacagtata cttttctctt tttttccaga attaaccatg aatcgcacac acagccagag 1680
   gcttttaacc cgagaacgga caaaggggcc tgcttgtgca atacaattat ttttaatggt 1740
   taaacaaatt aatacataag accagcttta cctaatataa taataacgaa ccaaagttta 1800
   caacagacaa gaaaagcacc agctgtcccc gccaccccgg agcgatctcc aaggggacgc 1860
   gggagagcgc cgcgggggac gcggaagtct gacgtcacag gaactggggg cggggcgggg 1920
   aggcccgcac accctattgc gcatgctccc gcctccccgg ccgcggcctg gcgcagtgcg 1980
   cacgcgcgcg ggtgggcggg tttgactggc cgtagagtct gcgcagttgg tgaatggcgt 2040
   tggtggcggg aaagttgagt ctctcctgcg ccgagccttc ggggcgatgt gtagtgcctt 2100
   ccatagggct gagtctggga ccgaggtgag agccgccggg ttgggagtga gggagatggg 2160
   aacaaggccg ccggtgggcg aggggagccg agggaacccg ggggattggg aggcttgggg 2220
   cggcgcggcc tggccgggct gggaccggcc tctcggccta gacgcccgcg atgctggcac 2280
   cctctgccac ctctcacctg ggccccaggg gtccgcccct gggcagcctg gagtcctccg 2340
   aggtgggagg accgggcgga ggtggaggaa gtctttcttt ggaagacttg ctgcctgccc 2400
   agatcgatat aacatacgag gtctctcctc ccaagagtta tggtctaaaa acccctcaca 2460
   aattaactac cgttggaaat gtcaagctat gcaagaaaag ctagaaaagg ggaggggtcg 2520
   cccgttggag catttggagc ttttctggaa caggtggtgt ttgcggaggt tgcctcacct 2580
   ccctgtagcc cacgtgtctc tgcttagggc agctggccct cgcca 2625
<210> 70
   <211> 2540
   <212> DNA
   <213> Homo Sapiens
<400> 70
   tagtcccagc tactcgggag gctgaggcag gagaattgct tgaacccagg aagcagaggt 60
   tgcagtgagc tgagattatg ccactgcact ccagcctggg caacagaggg agactccatc 120
   tcaaaaaaaa aaaaaatcat taaaatacag taattcaggt ttattaagtc attaccattg 180
   ggttacctca caaataaact aagtttagat gcgaactcaa agatactgag acactaatcc 240
   atttcttaag ctgctaagtt agccttcttg aaacctcact tcgtagctct gcaaacaatg 300
   tacttttgac atcccaagct cacaggaata aaaaaccacc tgccagttgt ttccgttttc 360
   cacctatgtc taatttatgt acttatattt ataagaaaca aatcactaag tcttatttca 420
   tccttagtta tgttgtgttt ctatcgataa cagcatgaag atttcgggga cctggacatt 480
   aaaataagtt tgagtactgg ctttacaatc tactaggtgt gatccgaggc aagtcagtct 540
   cttcatgttt cacttctttc acttgtaaac atctattcag aagttgctgt gaacttgata 600
   tttccatgct tataaactga ttttttgaaa agagcctggt acataggacg tgataataaa 660
   tgaaagcatt tgctactttt ggaaaaacaa gcatgacaag atagtttata tactgttgat 720
   cttaagcaca gtatatgcat cttattttta gctagtctga cagtgagata ataaaaagag 780
   ttatctttga cttgcactac gagtagaaga attcaacttc agtttctaga aagatgtata 840
   agaattaaga gtggcagtct tcctagtctc aactgccatc ttcccaccag gtggtaaatt 900
   cgtccagaga agaaaatgaa ttattgctat atgggattct gcagcaactt ctgtgaacat 960
   aggctcataa tttttcacca tggagactca agctttttgg agtcatagtt gtttttgggt 1020
   ctatttgcag gcatgcatcc tttgtccaga aatatacata acatttggca catggacctg 1080
   gaggtaaaag aggaggaagg cctgaggcta gacaccactc caataagtac attaagctcc 1140
   tagaagggca atccaccttt gcagagaact cttaactatt aaaacctata gcttgtaaag 1200
   cagcattttc aaagttaaga gaagaaggtg gaagggtctt gagaggctac tgactaaaca 1260
   gatgaaaatg aaggtatgga gtttggtgcc aaaagaaact ccccccaaaa atcaaacaat 1320
   aacaccagag taaagcccct agggcgagat aaggagttgc aacaaaacaa gcggaaactc 1380
   gagaagcgct aatgcttcaa agggtcaatg accacacata atctacgtag ccaacgtgtt 1440
   aaaacacacc aacgcatttt tttttcctaa acaaagtagg aaagcggact ttgcatgagg 1500
   ggcgggctgc cgacccagca gtcttcctcg gacagtccgt cctgattctc tctggttggc 1560
   cgtggaggga ccacatggct ccaaggcctc tcagctccgg gcccacacac cccgggctgc 1620
   cgcacaaact ccagccctag tctagatcca caaccccttc tcgaagatca accgcgacct 1680
   gggagcccca cttcttacca tagcgaggcc ggcgatgccg cagccacatc acccttccgg 1740
   ggctcaggcg gaagaggctg catgtcccgt ctgcccttct cgccctctcc agccgtccgg 1800
   ttgggcttgt cacggcaccg cctaccaaga cgggcggtta agacactagg ataggctcct 1860
   ctccaccgga aaaggcggga tttagatcac gtcccgcagg ccggcggaag tagctgatac 1920
   tctcattggt tgcaaaacct tgatctgtga aagcgggcgt tttggaagat accggaagta 1980
   gagtcacgga gaggtaggat ccggaagtgg ggctgcctct ttaaataaca aaaatctgag 2040
   gttctgttct ttttatcttt ttgctttctt tttaaaaaag ttccctgcta cttaccccta 2100
   gaactccaca atgcgagaat ccccctcaat ttgtgagctc ccgcgacttc ctcttgtggg 2160
   cttttgggga tgctagggtt ctcggcatta tcctcagggt gcgacctgtt cacccccttt 2220
   tcagtttctc cgtttgcatc tgagggattc ttgggaatgc gaagcacttt tgaaatgctc 2280
   tgtgttggtt gtgggattgg gaggacggtt gaatccagag ggtagtgttg agtaggctgt 2340
   ttgagcattt ccccagcact ggcctgtcct ttcaatcccc agatattggt aaactgtggg 2400
   ttccaaccag gcatcgaggc tgaaacgtac taggcaattt gaggtcagga aagaactttc 2460
   tgtggtaacc aatgggaagg aactgccgtt tgcggactgc agcgattgat taggtacttt 2520
   aaagagatca actggcaaga 2540
<210> 71
   <211> 2610
   <212> DNA
   <213> Homo Sapiens
<400> 71
   ctacaggctc gtgtcaccac actgggcaat acaaaaaata caaaaaaaaa attttgtatt      60
   ttttgtagag acgaggtctt gccatattgc ccaggctgga attcttacct ttgttactgt     120
   atttaacgta tctttttcct ccggccatct tcatggtttt ctctctgatt tccacagttt     180
   gaatacactg catgtgtcag gcaggggctc atatttatca agttttgtgt gtgctctgag     240
   ctcaggtctt tcattatttt gggaaaatta ttggtaattt tctcttcaaa cattttttat    300
   gatttgttct ttcttcttct tttgggagtc ctattacatg catatgatat catttgatat     360
   tttcccacag ttcttggatg ctttttttaa aaaaaaactt tttttcttct ttattttcca    420
   acgtgggtaa ttcctatttt tctcagctgt gttgatccta ctgctgcccc atcagaaaaa     480
   ttacctgtta tcagcgttct tcctttctta taatttgatg agtttcctcc tcatgcatat     540
   tgttcacctt tcgtacaaga gacctccaca tattaatcac agttaattta aatttccagc     600
   ctgtttcaat ttctcgatca cctctgagtc tagtcctgtt aattgcttag tgttattttt     660
   tgtttttgaa acagggtctt gctctgttgc ccaggctgga gtgcagcggc gcgatctcag     720
   gctgttccct gagttcacac catccccctc aaccagcaga ttgcaaagtg tccgagtcgg     780
   gccgtgcagg agtctttgtg ggggtttcat ggactccgaa ttctcatttc tgctccatcc     840
   ccatctcatg aatccaaggc cccactctgt gcctcggctc ttcgtttgtg gtgctgaacg    900
   tcatctacgt catctacgcc atctacgtaa tcaacacaat aaagacgcct gccgggaacg     960
   cggcccttcg gctgaatccc ttcggtggtt ccaaggccac tgccagagga tgcggacggg    1020
   tctccagggc ctctacttac ccaggacttt gaggcacatt agcttcgcct aggcactcgc    1080
   ttttacgaat tcttatgttt ggttttgttt tgagacagag tctcgctctg ccgcccaggc    1140
   tggttaaaag atagggtctc agccgggtgc ggtggctcac gcctgtaatc ccagcacttt    1200
   gggaggccga ggcgggcgga tcacctgagg tccggagttc gagactagcc tgggccaaca    1260
   tggcgaaacg ctgtctctac taaaaataac aaaaatcatc caggcgtggt ggcgcgcacc    1320
   tgcaatccca gctactcggg aggctgaggc aggagaatca cctgaaccca ggaggcagac    1380
   gttgcagtga gccgagatcg cgccactgca ctccagcctg ggcgacagag ggagactccg    1440
   tctcaaaaaa aggaaaaaaa aaaaaaagaa aagaaacaaa agtgatgggg tctcgctctg    1500
   ttgcccaggc tagtctggaa ttcctgggct caagcgaccc tccagcctcg gcctcccaaa    1560
   gcgctgggaa tacaggcgcg gctaccgcgc ggtctccggc tgccgaaaca ccgccctgcg    1620
   cgcggaccgt tcggccgccg ggaggaacag cggctgcccg gagctcagag gcgcgcgcgg 1680
   ctttgcgctc cccgcggcgc tctgagcctg cctcggcttg gttggccagg tggtctcttc 1740
   aggaccaacc ccagtcattc ccggcaggaa ccacgcttga ggggcggcag tctgcccgcg 1800
   cgagacgccc ccgcggacta caccgcggcg gcaaagccaa acgcaaaaac tacctcaccg 1860
   cgcgcaggcg cctcccccag gaccaacatg gccacgacgc aaggcctcga cctgaggggc 1920
   gtggcctggc cgccgccagc caacgggtgt gcgcgcctgg ccgcagccaa taggaaggca 1980
   gcgcgggctc gggcgcaggg agccgccgcc ggggctgtag gcgccaaggc catgtccgac 2040
   tcgtgggtcc cgaactccgc ctcgggccag gacccagggg gccgccggag ggcctgggcc 2100
   gagctgctgg gtaggtgggc gcggcaggcc gcgggagtgg gcggcgtccg gcccgggacg 2160
   gtttcgccgg ttccccgatc ccttcccgcc agagcctccg ccggtcggat ccccggacgc 2220
   cgcgcccggg gggctgtgcg gggtgggcgc ccggctgggg cggcgcggct gcctcggacc 2280
   cggcccctcc tgcgcctggg cggacgccca ccagaccgcc gcccgcgggg cgctcccttc 2340
   tttcccgaac gccgcccccg ccggccgccc tgtcaggcgg gcctggggtg cgcggcctgg 2400
   ggctcccctc agcgcagagg ccgcccctcg ccagccgtcc ccgggctccc ctgcctcggg 2460
   ccctcctggg ccgtcttccc cggcgtccgc ggtggggccg tctccgttag tttcccgaga 2520
   cctgcgccct ggggaggagc cccggcccct cttcgggagg gtgtcgctgg tgggtttctc 2580
   cgcggcgtcc acctgcgcgt cgggccgggg 2610
<210> 72
   <211> 3076
   <212> DNA
   <213> Homo Sapiens
<400> 72
   gctgggatta caggcataac atggcccggc cctggccatg tttttaactg tgtttctcta 60
   atagctaata atgccgagca tctttttatg tgtttcttag ccattagtag atcttttttg 120
   gtaaaatgtc tttttttttt tttttggtcc atcttaaaat tgttttttgt tttgttttga 180
   gacagggtct cactttgttg cccacgctgg agtgcagtgg ctcaatcatg gctcactgca 240
   gcttcgacat ccctgagctc aggtgatcct cccacctaag tttcccgagt agatgggact 300
   acaggtgtgt gccaccatgc ccagctaatt tttgtatttt ttttgtagag gtggggtttt 360
   gctatgttgc ccaggcaggt cttaaacttc tgaggctcaa atgatcctcc cacctcagcc 420
   tcccaaagtg ctgggataac aggcatgaac caccacaccc agctaagatt gtttttaaaa 480
   atctttttct tgagttttgg gagtttttat gtgttaggga taccagtccc ttatgaggta 540
   tataattagc aagtagtttc tcccactctg tgactgtgac ctttcttttt ttgaggcagg 600
   gtctcactct gttactcagg ctggagggca gtggtgtgat catggctcac tgcaacctgg 660
   aactcctagg ctcaagggct cctcccacct cagcctccca agtagctggg tctacaggtg 720
   tgttattgtg ccagggttaa tgttttaaat tttttgtaga gataatgtct ctacaaaaga 780
   caccatcttt gttgcctagg ctggtcttga actcctggct tcagggaatc ctccagcctc 840
   agcctcccaa agtgctggga ttacagcatg agccacatcc agcctatgat ttttcttctt 900
   ttcttttctt ttcttttttt ttttttttga gatggagtct cgctgttgcg caggctggag 960
   tgcagtgggg cgatctcggc tcactgcagg ctctggcccg cggggttcac gcctttctcc 1020
   tgcctcagcc tcccgagtag ctgggactac aggcgcccgc cacatcgccc ggctaatttt 1080
   ttgtattttt agtagagacg gggtttcacc gtgttagcca tgatggtctc gatctcctga 1140
   cctcgtgatc cgcccgcctc ggtctcccaa agtgctggga tcgcaggcgt gagccacggc 1200
   gcccggcccc agcgtatgac ttcttaatga tgtctttgta gtacaagagt ttttaatttt 1260
   aataaagtta actttttttt aaattgtaca agcttttagt gctgtgtcta acaacttgtt 1320
   gccaaaccca aggtcataaa gctgttctct tacgttttct tttttttttt tttttgagac 1380
   ggagtctcac tctgtcaccc aggctggagt gcaatggcac gatgtcggct cactgcaacc 1440
   tccgccaccc gggttcaagc gattcttccg cctcagcctc cggggtagct gggattacag 1500
   gcgcacgaca ccacgccctg ctaatttttg tatttttgta gagaaggttt caccatgtta 1560
   gttaggctgc tttacgtttt cttttagaag ttttatattt ttggctctta tatttagttt 1620
   gtgatccatt gagttgattt tatgtacgta tgtatggtcg cgttcttttc tttcctgtct 1680
   tttttttttt tttttttttg catatggata ttcaattctc ctagctccat ttaatttgaa 1740
   atgattgggc aggtactttt gagcagtgca agtacagagc ggcactgcca gcagactaca 1800
   cgcggtagaa agccgacctt ggtgagcgtg ttggtgctcg acagtgagca gagaaaggat 1860
   ggacgattac ggagcgccct cgtctccagt taccgctttc tggaaacacc atccgccggg 1920
   gcggagctgt tccgccccgg tgcggtacta cgactcccag catgcacctc gcagtcggcc 1980
   ctcggtggaa gcgggaaccc aggaggacct gggggtgtgg cagcgaggaa gggccgagcc 2040
   acggactgtg gggccgaaac tcgctcccgc ccaccctttc tcgaggctgt ggcctccgcg 2100
   agagccgagc gggccgcacc gccggccgtg cgactgcccc agtcagacac gaccccggct 2160
   tctagcccgc ctaagcctgt ttggggttgc tgactcgttt cctccccgag tttcccgcgg 2220
   gaactaactc ttcaagagga ccaaccgcag cccagagctt cgcagacccg gccaaccaga 2280
   ggcgaggttg agagcccggc gggccgcggg gagagagcgt cccatctgtc ctggaaagcc 2340
   tgggcgggtg gattgggacc ccgagagaag caggggagct cggcggggtg cagaagtgcc 2400
   caggcccctc cccgctgggg ttgggagctt gggcaggcca gcttcaccct tcctaagtcc 2460
   gcttctggtc tccgggccca gcctcggcca ccatgtcccg ccagaccacc tctgtgggct 2520
   ccagctgcct ggacctgtgg agggaaaaga atgaccggct cgttcgacag gccaaggtaa 2580
   cacggttgct ggcaccctcg gtttgcagcc tcaagatccc tgaaagcggg tttgcagtgg 2640
   atttacccca acagatgggg agggactgag cttgaccaaa gagccagaaa tgactggaga 2700
   atgcatccct tgccactgct gcaaggggag aaaaaaggat tgatcctcag tgacaacccc 2760
   tccctcatgt ggcaggtggc tcagaactcc ggtctgactc tgaggcgaca gcagttggct 2820
   caggatgcac tggaagggct cagagggctc ctccatagtc tgcaaggtag gcgggtcctc 2880
   cccaggatgg tcagttcccc tcttccatag ccagagaaac atccgctcct gcgtttttgg 2940
   gatcgatata attactcggg gcagggagtc ctgtttaagg cacagaggag actggagtgg 3000
   aatcatcttt gtacaggcaa atccctctct tccttacaca ctcacagagt ggcatttgaa 3060
   aaatggtttc caagat 3076
<210> 73
   <211> 2567
   <212> DNA
   <213> Homo Sapiens
<400> 73
   cacaccatct cttgctccgt gagtatcttt gtctctctag ctcctcttct tctctcagta 60
   catgtccctc cttgactccc gcctctctgc aaggtgtatt tggctgcctc agttggcctc 120
   tccccctctg catctctggg tggggtgttc tctgcccgtc tcccacccac acccaccccc 180
   ggtgctcccc ttccccccag caggacagcg gctcaggttc acgcacccca cggcgggccg 240
   gctgggcgca cgcacgtcct tgcacacaag ccgcacgtag ctgtacttga gcacgtcgat 300
   gagcgtgtag agcgggggcg cactggccca gcggcagcgc gccaggtgca tggagctctt 360
   gacgaagaag agcgccagcc gctgctggca ccacgcgtcg aagaagcggc tgaactcggc 420
   ccacgagaag aaggcccgct cccgcagctc ctgctcctcc tgccccgcag ccgtgccggg 480
   tgggggctcc ggccgctcca tcctgggggc ctgcgtggag gaggggagaa caggtggata 540
   tcagacccat tcccacccgg ggtatctcat ctactccatt cttggcctgc cccgtcggtt 600
   gctggtgcct ctatcgaggt gggtagcccg gggtcggacg tgcctgtttt tctccaaata 660
   tataaatatc aacctccatc ctatctttgg cctcctccca ccgccttatc cctggttcac 720
   ttggagcctg tcatcttgat tcctaattcc aactcgtctc ctcctccgca gatgtgaccc 780
   ttaggtacag ttggaatctc tcctcccaaa atacgaccct taagctcaga tgttccttaa 840
   ggacatctcc tcaaatgtgt tctcaaattc cagctaaaac ctcctcccct tccagctgtg 900
   tctctcaccc aagagtaact tctaactctc gtattcatct ggaactcctc cttccatgtg 960
   ccaacagttg gctgtaaccc ctccaaagac gctccatctc cagatgtgct cccacatcca 1020
   ggccacggac ccctcacccg gtcacatgct tcatgcacct gtggctccgc actccccaga 1080
   tgtgcctctg gcgtgcagct gttgcccctt cccccgatta tgaccctatg gctcgccaca 1140
   tgcagctgta gctggggctt ccctgagaca ctctcatctc cagatgtact ccccacatgc 1200
   agttatccac gcttcgccta caggtgtgtg ccccacttgt ggctagttct cctcggaagt 1260
   gtcaccagta ttcacctgtg gtcccctcct cctcagatgc ggcccccagt ccagctgtgg 1320
   gcccctcctc ccagttacat ccaccatccc ccgcaatatg catcttcgtt ctagacatgg 1380
   cccctcgtcc tcggatgggc tccttcaccc cagatgctcc ccccacgtcc agctgcgcgt 1440
   ctcccctcga gcagccccat ccagcccgct cccgacgctc ctactccccc cctccccgcc 1500
   cgctgcggca ccttccagcc ccgccgtccc acctagctgt gcctctcccc tccccaagat 1560
   gtgcaccctt cccgcccctc cccactcacc tacccgcccc ggagcggcgt ccacctccca 1620
   caatgccccg cgcccaggcc tggcccggcc cttgctcccg ggatgccccg cgcggtctcc 1680
   cgcctctctt cccgccgtgc ctcgcggggg cgcttccacc gattcctcct ctttccctgc 1740
   cagtcactcc tcagaccctc agccacaccc gctcatccag ggcgagggaa agcgcgggca 1800
   ttttcccagt gtgctctgcg ggagggctcg ccccacttca ccccttttcc cgccctcctc 1860
   ccattcggga gactacgact cccagtgtcc tccgcgcgac ggcggcggtg cggacggtgc 1920
   ccaggtcccg cccctaggct ctgccccgcc cccgcccgca gacgtctgcg cgcgaatgcc 1980
   gtggcgcgaa cttgggactg cagaggcgcg cctggcggat ctgagtgtgt tgcccgggca 2040
   gcggcgcgcg ggaccaacgc aaggcaagtg gggccgtccg caagcagatg ggaggcggag 2100
   ggcggcgggt gcgccgaatg cttggggcct atgcttcgcc atgtcggggt gtctgcagag 2160
   gagtgggcgt ggggacgctg aggctgccga gagcgcggtg gagacggaag agcgcgggct 2220
   gcgggccgcc ggagagtgca gagaggtgtc tcccagaggg aggggggcca ggtagagggt 2280
   agacgagaga cagagacagt tggacaggtc ctctgagaag aggccttgag gtgcgagttc 2340
   acctggaagg gggagaggcc aaatggaact gaggggcggg gcgggggggg ggaaaactgt 2400
   gtgggcgggg ccagctggaa atcggaaggc cccccgaggg ggcggggcta tctgggaggg 2460
   ggaggggctg aagggagcta aggggcgggg ccggggaaaa gattgcgtgt gggcggggcc 2520
   acctggaagg gggaggtgcc aagggtgggg ctggctggga accggaa 2567
<210> 74
   <211> 2278
   <212> DNA
   <213> Homo Sapiens
<400> 74
   tcacagaagt caaagctcag gaaaagcccc tcgagggttt ttgtgcggca gaggtgggtt 60
   gtggggtggg attgtgcctg ccacagtgga ggggccctgc agacccagat aaaccttcaa 120
   gtggccagaa gcgggggatg gctctgctgg gtgctggggc tgccatgggc cgtgggagcc 180
   agcagtgtgc ccagctccct cagggcccgt cccctaggcc cttccgtcca ctgggccaag 240
   caccgtccct gcccctccct aggggcatgg atctgacttg agaggttgtg agagcttaca 300
   ggcgctgggc cgtcggggag gcctcagaag cgtaggacgg ctgcgcactg ccgggccgtg 360
   ttcagccctg gtctggcctc ggcctctaga ggaggctgcc tgcgctccag caggcccaac 420
   ccagaacgtg ggcgagctcc cttcagcatc cctgggcgga aagagggatg ggggctctgc 480
   tgcagaggca gaatccgcgc cgctccctcc ttccttcccc cgaccagcct gtgacaaccc 540
   cggccagggg cgggggcctc cgcacaagcc tggcgtccac ttcctggata aggactcccc 600
   ggcccactcc ggaccagggc tggggcggcc tcccaggcgc tcactccgct ggcaccccac 660
   cggaaaacac gtctgcggcc cgccccctcc cccaaagcac gaccactccg cccgggcccc 720
   tcgaggatcc actcaggttc acgacgggcc cgtcctctcg gtggtctgac caccggctgg 780
   tggagtgggc tctggggccg ccaggcgacc agggcgcagg cgggggcgga cagctcattg 840
   ggaggggcgc cggggcacag tgcggggctc gccccacccc caggtgcccc ttccccgctc 900
   tcgcctcgca ggcaccgcat cgggcccggg aatcggtccg gacctggcgg tgggcgctgg 960
   gaagaggatc cacctccacg tggcccgccc cgccccgggg gcgcagccag ttcccggcgc 1020
   tcactgcccc ccttctcccg gcttccgtcc ccttctgcgc aggcgccgct ccgccccggt 1080
   cctaggggtg cttccgtggt cggcggctgc tgggctccgc gccggggtcc gagtcccacg 1140
   aagccccggc ccgagccgcc ggatgcccgc gcgcagcggg gcccaggtga gcgcgcgcct 1200
   cggccgcccc gcggaacaga cgcgcccacc cccaggcgca gcagcgagcg cggccgcggg 1260
   agcgggagtg ccggggacgg gcgtagcgcc caccgccccg agggttcggg gcagagccag 1320
   agcataggcc aagggccaag ctcgggccga gagcagtggc cgcagcgccc gggggctgaa 1380
   cccacggcgc gctggcagcg cgggccgagc tgcggagacg gtcacgtcag cgtccgttcc 1440
   aggccgactg gcagtctccg ttctacatta acgtcagcac tcccgttaaa aataatgcat 1500
   ctctcccatg ccaggaggac ttaggtgctg ctaaagacca gccctccggg tgctgccagg 1560
   ccggcgctca cccgccacct tcatcttccc ttctcctttg ccccaggaca gccgaggatg 1620
   tgtggttagg ttccccctac ccatggggag gccagaggtg ggaggctggc ggcctgctcg 1680
   gtctcagcag accctcctag tccctcagga gaccttgcct ttgccccact tgctcgttat 1740
   ccagcctggg ccatgaagca gaggacagtt agggaccctg agcacgcggt ggtcaccccg 1800
   gtgctcaccc ctccctgtgt gtccgacctt ggccctgcta agatcctgtg ttttgaattc 1860
   tggcaagggt tggatgaaag ggcagggctc cagaaaccag ctcagacgtt tgcttgggac 1920
   ctgcatgatg agtgggaatc ggagggcacc agccctgctg tcccaggctc aggcccccat 1980
   ctgctcccca ggtcatgcag cctgggcccc catgccgtgc agctcgcaca tatgtggggc 2040
   agagcagcca ccctgccccc agcagcagcc gtccatcgtc agacgtgatc atttcctgag 2100
   gcctcgagtg tgtcagggtg tttgtgcctc ataacaaccc acaggatggt cacccccgct 2160
   ttgcagatga agaaaccaaa gcaggtggtc agatccagtc cttgcacttc ctgagcctga 2220
   ccttaccaca cagctgtctc ctattcggat gcttatttat tttttttccc attacagt 2278
<210> 75
   <211> 2401
   <212> DNA
   <213> Homo Sapiens
<400> 75
   tcatgcctgt aatcctaaca ctttgggaag ccaaggtggg aggactgctt gaggccagga 60
   gttcaatact agcctgggca acacagcaag atctcatctc taccaagaaa aacaaaggat 120
   agaggagtca actgaaaaag atcccagtga ctaaagctcg aacaatttta gcaataaaat 180
   aaatacgcat gatataaata catggctgaa taaataaact ggggagaata gaaaaatatc 240
   ctgtgcagaa gaattccaag taacttatat agatatttta cctttacctt caaggaagta 300
   gaacataact tttcattcct tcccaggatg ggctaggcat gatgacttcc ttccaaagag 360
   tacagaacgg aaacagggca gggggattaa cagtggagaa acctgaccaa cgctactgca 420
   gctaggtgat caaggccaaa acatcgacag tgataaagca tgctgagagc acctttgatt 480
   tgatgtagtg aaaatcgtgc tttacctctg taatcttcct gccaaaaacc cataatccca 540
   gccccaatta tgagagaaac attaggcaaa tatcaattga gaaatattct acaaaatacc 600
   tgactggtac tcctgaaaac tgtcaaggtc accaaaaaca ataaaagctc aagaaactgt 660
   cacagcccag aggaacctaa gatgtgacta ctaaatggca tgtagtaccc taaatgggat 720
   cctggaacac aaaaagagta tcaggtaaaa actaagagaa tcagaataaa gaaaggactt 780
   ttgttaataa tagtgtatca atattggttc atcaattttg acaagtgtac catactaata 840
   atgcaaggtg ttaataagaa acattcagca tgagattttt aggaatttcc tatattatct 900
   tcacaatttc ctgttaatct aaatctctcc taatgacaag tttatttaaa aagtaaaaca 960
   aaacttgaag gagggaggaa acaagaaggg aggaaacatt ggagacagaa ccagcttggc 1020
   aagttgacag ataaggtctg agaagtaggc aggggaaaga tcattcattt caggcaatat 1080
   ttttccattt tacctgtata agaaccatat gagccctatt tttctttctt tcttttttct 1140
   ttctttcttt tctttttttt ttttttttgt agagatgaag atttcactat gttgaacagg 1200
   ctggtctcaa actcctggcc tcaagcaatc ctcccacctc agcctcccaa agcatgagcc 1260
   accatggtgg gcctgtatga aggaactttt taaaaaatgc tacaagccgg gtgcagtggc 1320
   tcattacctg taatcccagc attctgggag gccaaggtaa gaggatcact tgggcccaga 1380
   agttcaagac catcctgaac aacatagcaa gaccctgttc tctgcttaaa aaaaacaaaa 1440
   acaagctggg cgtggtggat cacgcctgta atcccagcac tttgggaggc tgaggtgggc 1500
   agatcatgag gtcaggagtt cgagaccaga ctgaccaaca tggtgaaacc ccatctctac 1560
   taaaaataca aaaattagct gggcacggtg gtgtgcgcct gtgatcccag ctactcagga 1620
   ggctgaggca ggagaatcgc ttgaacccgg gagacggagg ttgcagtgag ctgagaaagc 1680
   agtgagctga gatagcacca ctgtgctcta gcctgggaga cggagtgaga ctctgtttca 1740
   aaaaaatcag cctgcccagt cagagcgcct cagcgccgtg ctcgggacat cccgccctgc 1800
   ggccagcccc cgcgtgacgt caccgcattc cggctccgct cctcccgccg cggcgcccgc 1860
   accgcagtga cagccagccg ggcccggtgc cggagaggaa gtgcggtccg cgccaagccc 1920
   gtccccgccg acgccggctc cccgcggctc gggtgacagc gtcgcggccg ccggacgcag 1980
   cgcggggcag gcgcgggcag agccgagcgc agcggaggct ccggcggagg cgcggggaaa 2040
   atggctgatg actttggctt cttctcgtcg tcggagagcg gtgccccgga ggcggcggag 2100
   gaggacccgg cggccgcctt cctggcccag caggagagcg agattgcagg catagagaac 2160
   gacgagggct tcggggcacc tgccggcagc catgcggccc ccgcgcagcc gggccccacg 2220
   agtgggggtg agtcagcgcg gggcctggag aggggctcag ggcgcgcacc cgggggaccc 2280
   cggccggggc ccaggggcac agggaagaga gcctgctcta ggccacccgg ggcaggagct 2340
   gggagacgtg gggaagaatc ttcttggaga tctccatgta ggacttccga gctggggatg 2400
   a 2401
<210> 76
   <211> 2501
   <212> DNA
   <213> Homo Sapiens
<400> 76
   ccagcctggg ccgcagagtg agaccctgtc tcaaaaaaag aacctactag tctacatacc 60
   acacttcctc atccccatct gagactatat atattttttc taacatgagg caatgccaaa 120
   aagaggggct ggtgagtgaa agtaagaaca gaaagacatg gaggcaagtc ttatagaata 180
   atagccaaca cttaaactta cacttaacag cgtgataggt attgttccaa acacattaaa 240
   ttcatttaat ggtccttaca tgtctatgta tttggtgatt attatcctta ttattcacat 300
   tgctgagtgt attattctgt tctcatgatg ctgatagaga catacccgag actggataac 360
   ttattaaaaa aaaaaaggtt taatggactc acagttccac gtggatgggg agtcctcaca 420
   atcatggtag aaagcaaaag acacgtctta catggcagca gggaagagag agaaatgaga 480
   accaaacaaa aggggtttcc ccttataaaa ccatcagctc tcatgcgact tattcactac 540
   catgagaaca gtatggggga aaccaccccc atgattcaat gatctaccag gtgcctccca 600
   caacctgtgg gaattatggg agctacaatt ccagatgaga tttgggtggg gacacagcca 660
   aaccacatca ctgaggaaac tgagttatag ggagattagt aacgcccaac acagctggta 720
   ggtggtggag ccaggcagtc tgactctagg gtctggactc tgaactgcat catgctgcca 780
   agaagttcct cattttttcc tctctctaag tttcccttat tcccctacag tcattccttc 840
   aacagcattt ccttcaccat cttttctact tctactatat aattaatttt ttcttcttgg 900
   tcccaaattc caacgtgcaa atgcagcctt atatacccta attcatcttt acctttagac 960
   tttcttccaa tgtttctact tcattccatt ttaaatttat ccatgagatg cctatttaca 1020
   agctgtaacc atcatgaagt gaatgaagaa taatacctac tactgtacaa tagaattcca 1080
   agagtataaa taggagttat ggctttctga cttgaaacta aatacttgat acttgatttt 1140
   gctgtctgag atcaatctga aaagtaataa taatcactaa catttgttga gcatcaattg 1200
   tgggccaagt gtcatttcaa tcactctgta catattaact catttcatcc tacaacaacc 1260
   cggtgaggca agttctgtta ttctgtttta cagttgagga aacagaggca tagagagctt 1320
   aagtagtttg cccagtagat agccagaaga ggagccagga tgggtctcgg gcagtttaac 1380
   agcacagctg aagtcttaac cactatgcca acagcttttt ggtcctacac atcccatggg 1440
   aagaggaaaa taaaaaggta tctatttgta taccttttta tttctgatat aagaagcaga 1500
   attcctttca catgacctat gtctatttaa tacgtcattt tgaaacttac caataaaatt 1560
   tcccaagcgc cagaaaactg ttagtggctt tttccatttc tctctatttt tttttgtgct 1620
   actaattttg cttctttccc tcagaaggct gccggaatag taaacattca ctgacatgtc 1680
   ataattactg gaaaatgggc actggaaaat cacattgtaa ttaattcaaa gcatgttttc 1740
   caaatgtact actttaaatt ggagcttata tcataatcca aggaaacctt tgtgtgtgta 1800
   ctgttcccac attgctcagc ctgggatatc caggagtaat tcaccttgcg cctgcctcca 1860
   gaccatcttc catggaaggg ggtgacccct tgcctcttgg caaccactat ttctaagctg 1920
   ccaacattac tcttgcatta tcaacattct aacttcatgg gaagggctgt ggtgagtttc 1980
   tggaatgtga ataggaagtt gtttttctaa acagcctgac actgagggga ggcagtgaga 2040
   ctgtaagcag tctgggttgg gcagaaggca gaaaaccagc agagtcacag aggagatggt 2100
   gagtttattt ttttctgcat gggaagtggt tgaagtgagt tggagtggta tggagtaaag 2160
   tcaggcaggt aaaggttcag aaagtgagga acagcgatag ccatggagtt ttatgttgaa 2220
   ttgcctatta gattttgtga gtacttttaa acttgctgtc cactttgacc ctcccaacac 2280
   ccttgtgagt tgaggttgct atttctattt tacaaataaa gccatcgtgg tttacagagg 2340
   ctgtgtttta tctaagcttc actgttaggc tacatgatgt tgggatctgg ggcctgtcct 2400
   ctggctccgc agctgctgtt cctcctacta gaatttatag gggctctctg agaatagatc    2460
   atggtaaacc tgtcacccca ttttccaaga ctgtacttct c                        2501
<210> 77
   <211> 2501
   <212> DNA
   <213> Homo Sapiens
<400> 77
   cctgggtcct ctcttccagc tcccaaaatg tactctattt ttatctgttt cacgaacgct 60
   ggtccagata gtcttccatc ccccactgac tgttagaagt gactctcagc ttttgtccat 120
   ctcgaagttt ctgtgctcag tgtgcctctc agactaaagg cttcctttgg gaagccccga 180
   ctctcgcttc tcaggacaga gatccagggg ttgggggagg aaaaggttga ccagaagcca 240
   tagcggagca gggagagaga gtgtgaaaga cagacccgcg gccaggctcc cagttctcca 300
   gctcgtagag ggcccaagtg gccgctataa tctgaaagag cagatatcgt aatcccatag 360
   tacttcctat tggctgcagg acacagttct gtcctgacac tgaaatttgg gtgtgtcagg 420
   gttctgggaa ttcacaacgc tcacaacttg tgaagcagct gtggggtggg ggatggggag 480
   ggtttcagca gaggaagtga ggtcagtcaa taattgatgc ctgtctgagc ttttagccat 540
   tatctccccc agcctctatt cctgtcaaaa ggtggggcgg ggcaggagga ggggtccctg 600
   gctcatcttg tagaatcccc atattagagt aagacacctt agaggtctac tcctgcttct 660
   aatacccacg tctttccaag tgtctctgag gccaccccct ccccagcctt ttcatttatt 720
   catttaatta acgaacgcct tcattgaggg cctcctctga gtcaggctca gccagccagc 780
   atctttgcta tgagctgaga taagcatcat ttccgtctat tctcacaacc accctatgag 840
   gctggcacgg tttactatgc ctatttagca gatgggggac tgaagcatgg agaggtgtca 900
   ctagcctacg gtaacacaac cagcctgcat tcctagtagg tagtttgact tcagagtctc 960
   tgtggataac caggaggcta ggactaagac cagagtcctg caggtactta gatggttgga 1020
   gcaaagcagg gcagtgaggt cagtgctccc agcctgtgca ggagcatcag gaagagtctg 1080
   tgtccccctc ccctgccggt atgaagccat tctgcttccc tccccagctg ccttgtgtca 1140
   gcagagttcc agggaggctc cattccccac ctctatctaa agctccattt gctggggtgg 1200
   gggccctgcc tggaagggga aggtccaagg ctgctcccag cgtgtccctc catcctgact 1260
   gtccctggcg gggcgggggt gtctttgtca cccagctgca caacggccag gaagggctca 1320
   aaccatcctc agggctaacc caaggccgtc ctctgggcct gtatacccct gtgctgagtg 1380
   cggatcggga gaggctgctg aagacaggag gggacaaatg ggggacgaag gggcccgagg 1440
   gaggggactg aaggatttgg gccaagtcgg gagttcccga gggcggagtc aaaacgcatc 1500
   tggattttgc tagccccaaa ctctgccctc attgctgcaa gcctcctaga ccgaggaccc 1560
   ccgggctgag ggtggggtaa ggataggtag tgtccctccc cgtcccaccc ccgcctgtcc 1620
   cttcctcggt ggccccttcc cggcgccccg attccaggcg gcccctccgc tgctgccagc 1680
   cgatccccct ctacccccac ccactactcc ggccgccaga cgttgcctac agtctcggct 1740
   ctgtctccca cggctgtggg tccggacccc acgggacccc tatgggaccc ccacaggacc 1800
   cccacggcct gagtccaagg cccgccccct cggggaggcg gatgtgggag gcccggcccg 1860
   ggtgcgggcc agcgacccgg gagctgcggg cggctgggag gggaggccgc cctgaggggc 1920
   tgggagcggc gcgggggtgg gtcccggtcc tgcagcccca gcgaggggcg agcggcggcc 1980
   agtcggcgag ctgggcaata aggaaacggt ttattaggag ggagtggtgg agctgggcca 2040
   ggcaggaaga cgctggaata agaaacattt ttgctccagc ccccatccca gtcccgggag 2100
   gctgccgcgc cagctgcgcc gagcgagccc ctccccggct ccagcccggt ccggggccgc 2160
   gcccggaccc cagcccgccg tccagcgctg gcggtgcaac tgcggccgcg cggtggaggg 2220
   gaggtggccc cggtccgccg aaggctagcg ccccgccacc cgcagagcgg gcccagaggt 2280
   gagtcgaggt ccgcggacgg gaccgggtgg cgggcggcct gacccccgct tcagtgggcc 2340
   cttccttcgg gcggacccca gagtcaccgc agagtggtcg cgggaggctc agtcccagct 2400
   cattagaaag gcaagctgct cctggctgac cacgcacagc tcccatgacc ctacctgaga 2460
   cttggagggg aatggacgag actggactgg aaatcagaaa c 2501
<210> 78
   <211> 2501
   <212> DNA
   <213> Homo Sapiens
<400> 78
   tggctaattt tttgtatttt tagtagagac ggggtttctc catgttgagg ctagtctcga      60
   actcctgacc tcaggtgatc tgcccgcctc agcctcccaa agtgctggga ttacaggcgt     120
   gagccaccac gcctggccgc taactacatg tgttctatga ggtgaggtcc ttcccagacc     180
   ctggaatcag gggttgcaat tagggtccaa ataatgaggt tggactacag ataacccatc     240
   tcctttctta cctttgacta gatccaagga ctaaactcca agaacccgag catctgtccc     300
   caaaactgaa aggattggac tagtcacccc ttgtttccct acagccacat cccaggcacc     360
   tggcccttgc tttgtccaga aattcagcta taactccaca catctgatgg ccctttctgg     420
   caagcaggca tttccatcag gaccctcagc tgccagacac atttactgga ggtcacttat     480
   taaacctggg ctcaatttcc acacagggag gctactgaag catcacactg ggtctcccag     540
   ccccttctca tagaggaaag atctctctgt cctgcagggt tggcagtcag cgccaagtaa     600
   agggaattta gctcttggcc caagatccct gcccaggaaa ggtacttgcg cctgctggaa     660
   actttgggct gaagtatact cctttccaaa aactcaggtc tgatatttac acaaagtctg     720
   aaattaatgc agagaaaact tccaagtgct tggactggag cagaaggctg agaacaggaa     780
   ggggctggtc cctggtacta gttttggttt tttggtggtt tttttttttc ttgttttttc      840
   tcacagaaca gggcaaagct gagtgtccct ggatgagtga agcaggagga ttaatcatgc     900
   ccagtgcttc tccactttaa actggttttc ctgggaattt gcaattgaga gtggggaggg     960
   gtaagaatcg tgggaaaagg ctgatggtgt tcagccaaat tcatccttca cgtgcccacc    1020
   cttctacagg cacatgcttt ggggccatcc acggctgcag ccaccccatc cttaggaagc    1080
   accactggcc ttcctttccg gtacctggac tcagcatcac tcccagcctc ttggagatgc    1140
   agccttcatt cagcacacag ctcagctctg agttctgttt ttgtccctag atgtctctgg    1200
   ggtcacctac tactccctgc ttggtggccc aggcccatcc ttctccactc ttgcacctct    1260
   tttagcagaa aaggagtgag aatggatatt tccatgggcc gtgtgtgcac tcccggctac    1320
   ccctgacagc tctactcaga gctaccctcc ctcctggggc ttcttatgtg ttctaaggct    1380
   gaggcaggaa gactgtgaga tcaggtgaca ctcaacagtt atgatcggtc ttaagattaa    1440
   cagtcctggc cgggcgcagt ggctcacgcc tgtaatccca acactttggg aggccgaggc    1500
   aggcagacca cgagatcagg agatcaagac catcctggct aacacagtga aaccccgtct    1560
   ctactaaaaa tacaaaaaat tagccaggcg tggtggcggg cacctgtagt cccagctact    1620
   caggaggctg aggcaggaga atggcgtgaa cccaggaggc ggagcttgca gtaagccaag    1680
   attgcgccac tgcactcccg ggtgacagag cgagactccg tctcaaaaaa aaaaacaaca    1740
   acaacaacaa aaagattaac actccttcta cttccaaacc taatacaaag ggacattgcc 1800
   tagtgattaa gagaattcat tcattcaaca aatacttgtt gagcacctac tatgtgccaa 1860
   gcactgttct aggcaccgga aatacagcag tgagaaaaac caaaaaaact ccctgccctc 1920
   atggggtgta tattcaagta gctgaaacag acagtgaaca aacaaaaaag gacaataatt 1980
   tcaaataata atgatgctat cggccaggtg tggtggctca tgcctataat cccagcattt 2040
   tgggaagcca agtcaagcgg attacctgag gtcaggagtt caagaacagc ctggccagca 2100
   tggtgaaacc ccatctctac taaaaataca aaaattagcc agacatggtg gcacacacct 2160
   gtaatcccag ctacttggga ggctgacgca ggagaattgc ttgagcccgg gaggtggagg 2220
   ttgcagtgag ccaagatctg acaggccttc agcaccactg cactctagac tggctgacag 2280
   agcgagactc tgtcaaaaaa aaaaaagcta taaatagact ttaacagggt aacatgatag 2340
   ggagggaggg ataggggagc agggtggtca aggaagggac atttaaacag gctagaatga 2400
   caatggccag cgagggaaag atccagaagt gtgtgctgga agaagaaaga gcaagcacaa   2460
   aacccttagg acaaaatcag ctcgtgtggt caaggcacag c                       2501
<210> 79
   <211> 2501
   <212> DNA
   <213> Homo Sapiens
<400> 79
   tgtttctgac ccctggctgc agcctaatgg gccgactgct ggacagcggt cctgagtcct 60
   gtttgaattg gtgctgcccc gacatcctct gacctcagct aatgatcctg cctgccgagg 120
   gcagacaggt ctctgcaacc ctatgggtgg taggggtggt gatgagagga gaggtagtct 180
   cacttgcaca gattttggtg tatggttctg tcttttgcac tctttcaaca gaggtctgtc 240
   cagtccctct tgcaagtgtg gggagggggt ggtgcaggac tatgaggtaa ctgtgagaag 300
   aggggctcca gcagaaccag ggtccaatgg ccttgaagag atggctgggg acagctggac 360
   tcattacgtc tactcctaaa tggaggaaac gacccctcag ctacacagca cctgagccag 420
   aatgtcacca tggtgctgct ccacaggatg acagctacct ggtttgtgag ggcccctatt 480
   ctagggacag ctacttcatt ctgccctccc agagcagcaa gcaacaaccc tatgccagga 540
   ggccaattgg cacgtcaagt gccagctcca atcgattgat agtagctgcc tggctctgaa 600
   aggcagctgg gatcgattca ccatgctgcc agcacacaga tggacccagc ggtggtccca 660
   gcagtgagtt cttgccttgg gccatttcat tttctttgtc ctggccaagg aatgattgga 720
   tgaacacact ggactcccaa tatgggtgga taagacaaga gtgtctggtc acacccctcc 780
   accactcata agcatggttg tgggcagttt ggttccccag gcggccttgg agaatgcaat 840
   gagccgagga actggtcatc tccaggtgca tccagggcag gaaaggatga cagcatgcgt 900
   gagccagggt cactggctaa gaagtcatct caggacctcc ccctagaaaa gcccactggg 960
   cagcatccct gctggttccc ccctacacca caaggttacg cagagctggc ggagggtcat 1020
   ggtcccactc atgtcaggtg ctcttaatct ggcaaggaaa tgtaacctac gtgaatctca 1080
   acaggcagtg aagcaccgtt tcttcctgac tccaggtagg gtgaagaaaa tgggacagta 1140
   gtacggggtg cgggcataaa cgcacaactc tgcctcccca gacgcagagc tgtggggctg 1200
   tgagaatgcc aggaggaggt aagaaagggc ggccccatgg ggggcctgca gggtgggaca 1260
   agcccaagag gtctctacat ccaggcctgg tgggggaggt gagcccctgg tttaccgagg 1320
   gggtcccttc ctgccctcgg aaatactgca gctcctacct ccatcgtctc cccgctgcgg 1380
   ggacccaggg gcgtgaggat gagagagccc ccaggcccca gggtcagacg actgtgttca 1440
   agcaagtgag aacctctctg aggctgtttc ccaactgtaa aatggggata gcagcagaac 1500
   tctctctcgc ggcttgcgtg aagaatacaa ttcgatgtcg acaggaggga gcggcgcgca 1560
   gcgcgcagcg agtagcaggc gctgaagaag gatacctgtg aactgggagt ggtggcggag 1620
   gctacgcggc cagagtccgg ggaaggggcg ccggctctgc cagtccctgc tcggggctgg 1680
   atggtcgggg gatgttctcg taagtcggct gggagggagc ggtcccgcgt accctgccac 1740
   cgccgccgca gaggttcggg caggtgcggg gccgcggccc ctccgcgagg gggccggtca 1800
   tccgccggga ctgacatccc ggaggcccaa tggcaagccg tcatctccgc gcatccgccc 1860
   aatcggcgcc ggttgccgtg ccgcgccggg tctctcgacc aatgggaaaa tttgctgtca 1920
   gatggggcgg ggcggagatt cgcgtcgccg gcccggtccg ctttgcgcac gggccgcgtg 1980
   agggcgggag ggctggcccg gggtctcggg ttgcgcgctg ggcctggagg gagggggcgg 2040
   cccccgcacc ggtccgagtt gcggccgcgt ggactgcgac ccgcgccgcg ccgcaccgcg 2100
   ccgcgccctg ggaacgccgc tccccgcgcg ccaacggacc cggggaagcc cttctggggt 2160
   ccgaggccgc gctgcggggc cgcccacgct gcgctccagg taagcctgag ccagtgggcg 2220
   gggtgtggga cccggggctg gggcctcggg tcggagccgg gactgggggc ggggctgcag 2280
   atatgggacg cattccgggc agcggtccgg acagggtcct atccctggag tcgagatccg 2340
   ggcgagggtc tgggccggac gtcggagcca atctccgccc cacccgcgtc ttgtccgcgc 2400
   gctctgcggc gtccgagacc ccgggccggc gggggcgggt ctctttgtgc gtggccttgg 2460
   ggccctaccc tacccgtccg ggcgtcttgc actgagcact c 2501
<210> 80
   <211> 2501
   <212> DNA
   <213> Homo Sapiens
<400> 80
<210> 81
   <211> 22
   <212> DNA
   <213> Artificial Sequence
<220>
   <223> primer
<400> 81
   aatcctccaa attctaaaaa ca 22
<210> 82
   <211> 20
   <212> DNA
   <213> Artificial Sequence
<220>
   <223> primer
<400> 82
   aggaaaggga gtgagaaaat 20
<210> 83
   <211> 22
   <212> DNA
   <213> Artificial Sequence
<220>
<223> primer
<400> 83
   ggataggagt tgggattaag at 22
<210> 84
   <211> 22
   <212> DNA
   <213> Artificial Sequence
<220>
   <223> primer
<400> 84
   aaatcttttt caacaccaaa at 22
<210> 85
   <211>
   <212> DNA
   <213> Artificial Sequence
<220>
   <223> primer
<400> 85
   aaccctttct tcaaattaca aa 22
<210> 86
   <211> 21
   <212> DNA
   <213> Artificial Sequence
<220>
<223> primer
<400> 86
   tgattgggtt ttagggaaat a 21
<210> 87
   <211> 22
   <212> DNA
   <213> Artificial Sequence
<220>
   <223> primer
<400> 87
   ttgaaaataa gaaaggttga gg 22
<210> 88
   <211> 19
   <212> DNA
   <213> Artificial Sequence
<220>
   <223> primer
<400> 88
   cttctacccc aaatcccta 19
<210> 89
   <211> 18.
   <212> DNA
   <213> Artificial Sequence
<220>
<223> primer
<400> 89
   tgtttgggat tgggtagg 18
<210> 90
   <211> 23
   <212> DNA
   <213> Artificial Sequence
<220>
   <223> primer
<400> 90
   cataaccttt acctatctcc tca 23
<210> 91
   <211> 22
   <212> DNA
   <213> Artificial Sequence
<220>
   <223> primer
<400> 91
   ttttagattg aggttttagg gt 22
<210> 92
   <211> 22
   <212> DNA
   <213> Artificial Sequence
<220>
   <223> primer
<400> 92
   atccattcta cctccttttt ct 22
<210> 93
   <211> 18
   <212> DNA
   <213> Artificial Sequence
<220>
   <223> primer
<400> 93
   ggaggggaga gggttatg 18
<210> 94
   <211> 22
   <212> DNA
   <213> Artificial Sequence
<220>
   <223> primer
<400> 94
   tactatacac accccaaaac aa 22
<210> 95
   <211> 19
   <212> DNA
   <213> Artificial Sequence
<220>
   <223> primer
<400> 95
   ttttgggaat gggttgtat 19
<210> 96
   <211> 21
   <212> DNA
   <213> Artificial Sequence
<220>
   <223> primer
<400> 96
   ctacccttaa cctccatcct a 21
<210> 97
   <211> 22
   <212> DNA
   <213> Artificial Sequence
<220>
   <223> primer
<400> 97
   ttgttgggag tttttaagtt tt 22
<210> 98
   <211> 22
   <212> DNA
   <213> Artificial Sequence
<220>
   <223> primer
<400> 98
   caaattctcc ttccaaataa at 22
<210> 99
   <211> 22
   <212> DNA
   <213> Artificial Sequence
<220>
   <223> primer
<400> 99
   gtaatttgaa gaaagttgag gg 22
<210> 100
   <211> 22
   <212> DNA
   <213> Artificial Sequence
<220>
   <223> primer
<400> 100
   ccaacaacta aacaaaacct ct 22
<210> 101
   <211> 20
   <212> DNA
   <213> Artificial Sequence
<220>
   <223> primer
<400> 101
   ggagttgtat tgttgggaga 20
<210> 102
   <211> 21
   <212> DNA
   <213> Artificial Sequence
<220>
   <223> primer
<400> 102
   taaaacccca attttcacta a 21
<210> 103
   <211> 22
   <212> DNA
   <213> Artificial Sequence
<220>
   <223> primer
<400> 103
   tttgtattag gttggaagtg gt 22
<210> 104
   <211> 22
   <212> DNA
   <213> Artificial Sequence
<220>
   <223> primer
<400> 104
   cccaaataaa tcaacaacaa ca 22
<210> 105
   <211> 22
   <212> DNA
   <213> Artificial Sequence
<220>
   <223> primer
<400> 105
   gatttttgga gaggaagtta ag 22
<210> 106
   <211> 22
   <212> DNA
   <213> Artificial Sequence
<220>
   <223> primer
<400> 106
   aaaactaaaa accaaaccca ta 22
<210> 107
   <211> 20
   <212> DNA
   <213> Artificial Sequence
<220>
   <223> primer
<400> 107
   tggggttagt ttaggatagg 20
<210> 108
   <211> 25
   <212> DNA
   <213> Artificial Sequence
<220>
   <223> primer
<400> 108
   cttaaaaaca ctaaaacttc tcaaa 25
<210> 109
   <211> 21
   <212> DNA
   <213> Artificial Sequence
<220>
   <223> primer
<400>.109
   tttttgtatt ggggtaggtt t 21
<210> 110
   <211> 24
   <212> DNA
   <213> Artificial Sequence
<220>
   <223> primer
<400> 110
   cccaactatc tctctcctct ataa 24
<210> 111
   <211> 25
   <212> DNA
   <213> Artificial Sequence
<220>
   <223> primer
<400> 111
   attagaagtg aaagtaatgg aattt 25
<210> 112
   <211> 19
   <212> DNA
   <213> Artificial Sequence
<220>
   <223> primer
<400> 112
   tcaatttcca aaaaccaac 19
<210> 113
   <211> 22
   <212> DNA
   <213> Artificial Sequence
<220>
   <223> primer
<400> 113
   gggatgggtt attagttgta aa 22
<210> 114
   <211> 22
   <212> DNA
   <213> Artificial Sequence
<220>
   <223> primer
<400> 114
   ccttcacaca aaactacaaa aa 22
<210> 115
   <211> 22
   <212> DNA
   <213> Artificial Sequence
<220>
   <223> primer
<400> 115
   taattgaagg ggttaatagt gg 22
<210> 116
   <211> 22
   <212> DNA
   <213> Artificial Sequence
<220>
   <223> primer
<400> 116
   aaaaccaaaa ccaaaactaa aa 22
<210> 117
   <211> 22
   <212> DNA
   <213> Artificial Sequence
<220>
   <223> primer
<400> 117
   agtggatttg gagtttagat gt 22
<210> 118
   <211> 22
   <212> DNA
   <213> Artificial Sequence
<220>
   <223> primer
<400> 118
   aacaaaataa aaacttctcc ca 22
<210> 119
   <211> 22
   <212> DNA
   <213> Artificial Sequence
<220>
   <223> primer
<400> 119
   taggggaaaa gttagagttg ag 22
<210> 120
   <211> 18
   <212> DNA
   <213> Artificial Sequence
<220>
   <223> primer
<400> 120
   cccattaacc cacaaaaa 18
<210> 121
   <211> 22
   <212> DNA
   <213> Artificial Sequence
<220>
   <223> primer
<400> 121
   attttagttt gtgaaatggg at 22
<210> 122
   <211> 21
   <212> DNA
   <213> Artificial Sequence
<220>
   <223> primer
<400> 122
   tcttaaccaa taacccctca c 21
<210> 123
   <211> 22
   <212> DNA
   <213> Artificial Sequence
<220>
   <223> primer
<400> 123
   gtgggttttg ggtagttata ga 22
<210> 124
   <211> 20
   <212> DNA
   <213> Artificial Sequence
<220>
   <223> primer
<400> 124
   taacctcctc tccttaccaa 20
<210> 125
   <211> 22
   <212> DNA
   <213> Artificial Sequence
<220>
   <223> primer
<400> 125
   taggatgggg agagtaatgt tt 22
<210> 126
   <211> 22
   <212> DNA
   <213> Artificial Sequence
<220>
   <223> primer
<400> 126
   acaacttatc caacttccat tc 22
<210> 127
   <211>
   <212> DNA
   <213> Artificial Sequence
<220>
   <223> primer
<400> 127
   tcccacaaaa actaaacaat ta 22
<210> 128
   <211> 21
   <212> DNA
   <213> Artificial Sequence
<220>
   <223> primer
<400> 128
   aggttttaga tgaaggggtt t 21
<210> 129
   <211> 23
   <212> DNA
   <213> Artificial Sequence
<220>
   <223> primer
<400> 129
   tttggagggt ttagtagaag tta 23
<210> 130
   <211> 22
   <212> DNA
   <213> Artificial Sequence
<220>
   <223> primer
<400> 130
   cccaataatc acaaaataaa ca 22
<210> 131
   <211> 22
   <212> DNA
   <213> Artificial Sequence
<220>
   <223> primer
<400> 131
   atacaacctc aaatcctatc ca 22
<210> 132
   <211> 22
   <212> DNA
   <213> Artificial Sequence
<220>
   <223> primer
<400> 132
   agggagaagg aagttatttg tt 22
<210> 133
   <211> 22
   <212> DNA
   <213> Artificial Sequence
<220>
   <223> primer
<400> 133
   ggaagatgag gaagttgatt ag 22
<210> 134
   <211> 22
   <212> DNA
   <213> Artificial Sequence
<220>
   <223> primer
<400> 134
   cctacaaccc tatcctctaa aa 22
<210> 135
   <211> 22
   <212> DNA
   <213> Artificial Sequence
<220>
   <223> primer
<400> 135
   ttagtagggg tgtgagtgtt tt 22
<210> 136
   <211> 23
   <212> DNA
   <213> Artificial Sequence.
<220>
   <223> primer
<400> 136
   caaacaaaac ttctatctca ace 23
<210> 137
   <211> 21
   <212> DNA
   <213> Artificial Sequence
<220>
   <223> primer
<400> 137
   ttatagggtt gagtttggga t 21
<210> 138
   <211> 22
   <212> DNA
   <213> Artificial Sequence
<220>
   <223> primer
<400> 138
   taaacaaaca acaaatcttc ca 22
<210> 139
   <211> 22'
   <212> DNA
   <213> Artificial Sequence
<220>
   <223> primer
<400> 139
   tgaaaatgaa ggtatggagt tt 22
<210> 140
   <211> 22
   <212> DNA
   <213> Artificial Sequence
<220>
   <223> primer
<400> 140
   ttaaaaccat ataatccctc ca 22
<210> 141
   <211> 22
   <212> DNA
   <213> Artificial Sequence
<220>
   <223> primer
<400> 141
   tatgtttggt tttgttttga ga 22
<210> 142
   <211> 22
   <212> DNA
   <213> Artificial Sequence
<220>
   <223> primer
<400> 142
   aaccccatca cttttatttc tit 22
<210> 143
   <211> 22
   <212> DNA
   <213> Artificial Sequence
<220>
   <223> primer
<400> 143
   gggtgtagaa gtgtttaggt tt 22
<210> 144
   <211> 22
   <212> DNA
   <213> Artificial Sequence
<220>
   <223> primer
<400> 144
   tttctcccct tacaacaata ac 22
<210> 145
   <211> 22
   <212> DNA
   <213> Artificial Sequence
<220>
   <223> primer
<400> 145
   tccccttcca actatatctc tc 22
<210> 146
   <211> 22
   <212> DNA
   <213> Artificial Sequence
<220>
   <223> primer
<400> 146
   tgagagtgtt ttagggaagt tt 22
<210> 147
   <211> 22
   <212> DNA
   <213> Artificial Sequence
<220>
   <223> primer
<400> 147
   aaaaccaaaa cataaaccaa aa 22
<210> 148
   <211> 22
   <212> DNA
   <213> Artificial Sequence
<220>
   <223> primer
<400> 148
   gattaggagg gtttgttgag at 22
<210> 149
   <211> 21
   <212> DNA
   <213> Artificial Sequence
<220>
   <223> primer
<400> 149
   aatggttgat gattttggtt t 21
<210> 150
   <211> 22
   <212> DNA
   <213> Artificial Sequence
<220>
   <223> primer
<400> 150
   actctcttcc ctatacccct aa 22
<210> 151
   <211>
   <212> DNA
   <213> Artificial Sequence
<220>
   <223> primer
<400> 151
   tgttagtaga gttttaggga ggtt 24
<210> 152
   <211> 22
   <212> DNA
   <213> Artificial Sequence
<220>
   <223> primer
<400> 152
   acactaccta tccttacccc ac 22
<210> 153
   <211> 22
   <212> DNA
   <213> Artificial Sequence
<220>
   <223> primer
<400> 153
   tttttgtttt tatggggtgt at 22
<210> 154
   <211> 22
   <212> DNA
   <213> Artificial Sequence
<220>
   <223> primer
<400> 154
   ttaaatatcc cttccttaac ca 22
<210> 155
   <211> 23
   <212> DNA
   <213> Artificial Sequence
<220>
   <223> primer
<400> 155
   agttagaaga ggagttagga tgg 23
<210> 156
   <211> 22
   <212> DNA
   <213> Artificial Sequence
<220>
   <223> primer
<400> 156
   taattttcca atacccattt tc 22
<210> 157
   <211> 22.
   <212> DNA
   <213> Artificial Sequence
<220>
   <223> primer
<400> 157
   tgggtagtat ttttgttggt tt 22
<210> 158
   <211> 22
   <212> DNA
   <213> Artificial Sequence
<220>
   <223> primer
<400> 158
   cctaaaaact ctctcatcct ca 22
<210> 159
   <211> 23
   <212> DNA
   <213> Artificial Sequence
<220>
   <223> primer
<400> 159
   agtggtttag gagtatttgg tta 23
<210> 160
   <211> 22
   <212> DNA
   <213> Artificial Sequence
<220>
   <223> primer
<400> 160
   aactccctcc atctacaata tc 22